(19) Europäisches Patentamt
European Patent Office
Office européen des brevets

(11) Publication number: **0 408 859 B1**

# (12) EUROPEAN PATENT SPECIFICATION

(45) Date of publication of patent specification: **09.08.95**

(51) Int. Cl.[6]: **C07K 16/28**, C12N 15/12, C07K 14/705, A61K 39/395, A61K 38/02

(21) Application number: **90109597.6**

(22) Date of filing: **21.05.90**

Divisional application 93116072.5 filed on 21/05/90.

## (54) Monoclonal antibodies to activated endothelial cells.

(30) Priority: **23.05.89 US 355701**
**30.03.90 US 502590**

(43) Date of publication of application:
**23.01.91 Bulletin 91/04**

(45) Publication of the grant of the patent:
**09.08.95 Bulletin 95/32**

(84) Designated Contracting States:
**CH DE DK ES FR GB IT LI NL SE**

(56) References cited:
**WO-A-90/05539**
**WO-A-90/05786**
**WO-A-90/13300**

**PROC. NATL. ACAD. SCI. USA, vol. 84, December 1987, pages 9238-9242; M.P. BEVILACOUA et al.: "Identification of an inducible endothelial-leukocyte adhesion molecule"**

(73) Proprietor: **OTSUKA PHARMACEUTICAL CO., LTD.**
**9, Kandatsukasa-cho 2-chome**
**Chiyoda-ku**
**Tokyo 101 (JP)**

(72) Inventor: **Newman, Walter, Otsuka Pharmaceutical Co., Ltd.**
**9900 Medical Center Drive**
**Rockville,**
**Maryland 20850 (US)**
Inventor: **Wilson, Diane O., Otsuka Pharmaceutical Co., Ltd.**
**9900 Medical Center Drive**
**Rockville,**
**Maryland 20850 (US)**
Inventor: **Gopal, T. Vencat, Otsuka Pharmaceutical Co., Ltd.**
**9900 Medical Center Drive**
**Rockville,**
**Maryland 20850 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 408 859 B1

**THE JOURNAL OF IMMUNOLOGY, vol. 137, no. 6, 15th September 1986, pages 1893-1896, The American Association of Immunologists, US; J.S. PROBER et al.: "Overlapping patterns of activation of human endothelial cells by interleukin 1, tumor necrosis factor, and immune interferon"**

**THE JOURNAL OF IMMUNOLOGY, vol. 136, no. 5, 1st March 1986, pages 1680-1687, The American Society of Immunologists, US; J.S. POBER et al.: "Two distinct monokines, interleukin 1 and tumor necrosis factor, each independently induce biosynthesis and transient expression of the same antigen on the surface of cultured human vascular endothelial cells"**

**THE JOURNAL OF IMMUNOLOGY, vol. 144, no. 7, 1st April 1990, pages 2558-2565, The American Association of Immunlogists, US; S.M. WELLICOME et al.: "A monoclonal antibody that detects a novel antigen on endothelial cells that is induced by tumor necrosis factor, IL-1, or lipopolysaccharide"**

**CELL, vol. 59, 22nd December 1989, pages 1203-1211, Cell Press; L. OSBORN et al.: "Direct expression cloning of vascular cell adhesion molecule 1, a cytokine-induced endothelial protein that binds to lymphocytes"**

**EUR. J. IMMUNOL., vol. 19, 1989, pages 715-720, VCH Verlagsgesellschaft, Weinheim, DE; J.F.M. LEEUWENBERG et al.: "Induction of an activation antigen on human endothelial cells in vitro"**

**The Journal of Biological Chemistry, vol. 259, no. 15, 10th August 1984, pages 9799-9804, The American Society of Biological Chemists, Inc., US; R.P. McEVER et al.: "A monoclonal antibody to a membrane glycoprotein binds only to activated platelets"**

**EXP. CELL. BIOL., vol. 55, 1987, pages 117-126, S. Karger AG, Basel, CH; S. GOERDT et al.: "Characterization and expression kinetics of an endothelial cell activation antigen present in vivo only in acute inflammatory tissues"**

**Proc. Natl. Acad. Sci. USA 88, 7859, 1991**

(74) Representative: **Hansen, Bernd, Dr. Dipl.-Chem. et al**
**Hoffmann, Eitle & Partner**
**Patent- und Rechtsanwälte,**
**Postfach 81 04 20**
**D-81904 München (DE)**

## Description

## FIELD OF THE INVENTION

The present invention relates to monoclonal antibodies and their applications to inflammatory processes. More particularly, the present invention relates to new monoclonal antibodies that are directed to activated endothelial cells in vitro and in vivo. The present invention also relates to new endothelial cell surface antigens which are induced as a consequence of IL-1 treatment. The complete cDNA sequence for one of the antigens is disclosed. The new monoclonal antibodies as well as the new antigens are useful as medicaments for treating acute and/or chronic inflammatory responses associated with endothelial cells and the present invention further relates to such medicaments as well as to methods for treating such inflammatory responses. Additionally, the present invention relates to methods of detecting inflammatory responses associated with endothelial cells such as early graft rejection, subclinical infection, and vasculitis.

## BACKGROUND OF THE INVENTION

Endothelial cells (EC) are major participants in chronic and acute inflammation. They regulate the passage of cells and fluids between the bloodstream and the extravascular space. In addition, they respond to inflammatory stimuli by secreting factors which have a variety of effects on hematopoiesis (Ouesenberry and Gimbrone, Blood, 56: 1060-1067, 1980), chemotaxis (Streiter et al, Science, 243: 1467-1469, 1989), and coagulation (Bevilacqua et al, J. Exp. Med., 160: 618-623, 1984). Interleukin-1 (IL-1) is secreted by a wide range of cell types as an almost universal response of cells to injury (Neta and Oppenheim, Ann. Int. Med. 109: 1-31, 1988). Many cell types secrete this factor under appropriate stimulation, and many cell types respond, and in a variety of ways. IL-1 is found at sites of inflammation, and when injected as a purified protein, results in erythema and an influx of granulocytes from the bloodstream and secondarily, tissue destruction (Beck et al, J. Immunol., 136: 3025-3031, 1986; Pettipher et al, Proc. Natl. Acad. Sci., 83: 8749-8753, 1986).

Several laboratories have shown that endothelial cells have a rapid response to IL-1 which is consistent with their accessory nature in the inflammatory reaction. Two of the best described responses are an increase in procoagulant activity (Bevilacqua et al, 1984, supra) and a concomitant increase in adhesive capacity for leucocytes (Bevilacqua et al, J. Clin. Invest., 76: 2003-2011, 1985).

As the lining of blood vessels, endothelial cells are uniquely positioned to regulate the traffic in inflammatory cells and their reactive by-products. In addition, endothelial cells can themselves secrete into the bloodstream newly synthesized proteins which may be an early indicator of the inflammatory process. Despite this key role, very few therapeutic approaches or diagnostic indicators have been developed that directly address the role of this cell type. The biology of endothelial cells is starting to be understood now in much better detail due to the recent success in culturing these cells in the laboratory.

As mentioned above, a key mediator of the inflammatory response is IL-1. IL-1 is a 17 Kd polypeptide secreted by macrophages and many other cell types which is capable of eliciting a wide array of responses ranging from induction of fever, to proliferation of inflammatory cells and the recruitment of mature leucocytes from precursors in the bone marrow (reviewed by Dinarello, FASEB J., 2: 106-115, 1988).

Endothelial cells both respond to and secrete IL-1 at a very early stage of the inflammatory process. While a byproduct of bacterial infection with gram negative organisms is the production of endotoxin and the consequent secondary production of IL-1, the issue of whether mechanical injury can trigger IL-1 release is a critical but as yet unanswered question for students of sports-induced inflammation.

It is believed, based upon work by several laboratories in the past decade, that the binding of leucocytes to the vascular wall, a process that can be mimicked in vitro with IL-1 treatment of endothelial cells, is the first step in the diapedesis of leucocytes into the tissue space. However, several aspects of this process, especially as they may be related to the utility of current anti-inflammatory agents for intervention, remain unclear.

IL-1 induces a rapid alteration in the membrane properties of cultured human endothelial cells. This is evident from the ability of treated cells to bind leucocytes, their acquisition of procoagulant activity and the expression of new cell surface antigens including some for which no functional properly has yet been assigned. In those cases studied, the development of the new properties described is sensitive to the action of actinomycin D and cycloheximide, suggesting a requirement for the synthesis of new message and new proteins.

Binding of leucocytes and tumor cells of hematopoietic origin to basal and activated endothelial cells as well as to the "high" endothelial cells of capillaries has been studied by several laboratories. Evidence has been presented that granulocytes, monocytes, T, and B-cells all can bind to endothelial cells after stimulation with physiologic concentrations of IL-1 (Bevilacqua et al, J. Clin. Invest., 76: 2003-2011, 1985; Cavender et al, J. Immunol., 136: 203-207, 1986; Pohlman et al, J. Immunol., 136: 4548-4553, 1986). T-cell binding can also be induced by pretreatment of endothelial cells with IFN-gamma (Yu et al, Clin. exp. Immunol., 62: 554-560, 1985; Masuyama and Kano, J. Clin. Invest., 77: 1596-1605, 1986) as well as IL-1 (Cavender et al, J. Immunol., 136: 203-207, 1986; Bender et al, J. Clin. Invest., 79: 1679-1688, 1987), lps (Yu et al, J. Immunol., 136: 569-573, 1986), and TNF (Cavender et al, J. Immunol, 139: 1855-1860, 1987). The pro-adhesive effects of these cytokines for quiescent endothelium is widely viewed as a model of early events in the extravasatory component of inflammation. In the case of high endothelial venules (HEV), the constituent endothelial cells are presumably already activated in some manner which facilitates their role in the recirculation of T and B-cells (reviewed in: Yednock et al, Adv. in Immunol., 44: 313-378, 1989). The binding of monocytes (Pawlowski et al, J. Exp. Med., 168: 1865-1882, 1988; Wallis et al, J. Immunol., 135: 2323-2330, 1985) and granulocytes (Lo et al, J. Exp. Med., 169: 1779-1793, 1989) to cultured endothelial cells which are otherwise not stimulated has been taken as a model for the margination of these cell types within the circulatory system.

A fundamental assumption of research into lymphocyte recirculation and the early stages of inflammation is the existence of selective mechanisms for the binding and extravasation of leucocyte subsets. The existence of such mechanisms is inferred from the differential recirculation of lymphocytes through mucosal versus peripheral lymphoid organs (reviewed in: Yednock et al, Adv. in Immunol., 44: 313-378, 1989), the kinetic difference in the extravasation of neutrophils, monocytes and lymphocytes in inflammation (Issekutz et al, Am. J. Pathol., 103: 47, 1981), and the selective binding and accumulation of monocytes in atherogenesis (Taylor and Lewis, Am. J. Pathol., 125: 152, 1986). T-cells are late but persistent elements of inflammatory lesions, especially those which continue in chronic form. The role of T-cells in the pathogenesis of autoimmune diseases (Wofsy and Seaman, J. Exp. Med., 161: 378-391, 1985; Ranges et al, J. Exp. Med., 162: 1105-1110, 1985; Taurog et al, Cell. Immunol., 75: 271-282, 1983; Maron et al, J. Immunol., 131: 2316-2322, 1983; Rossini et al, Ann. Rev. Immunol., 3: 289-320, 1985) has led to interest in their emigration to sites of eventual tissue damage with special emphasis on their interaction with activated or pro-adhesive endothelial elements.

Direct and indirect evidence strongly suggests that the adhesion of leucocytes to basal or activated endothelial cells is a complex process. While four apparently distinct structures have been described on activated endothelial cells (Hagemeier et al, Int. J. Cancer, 38: 481-488, 1986; Goerdt et al, Expl. Cell. Biol., 55: 117-126, 1987; Duijvestijin et al, Am. J. Pathol., 130: 147-155, 1988; Leeuwenberg et al, Eur. J. Immunol., 19: 715-720, 1989), at least two additional well characterized molecules have been directly implicated in adhesion events. The ELAM-1 protein appears to mediate primarily the adhesion of granulocytes to cytokine activated endothelium (Pober et al, J. Immunol., 136: 1680-1687, 1986; Bevilacqua et al, Proc. Natl. Acad. Sci., 84: 9238-9242, 1987; Bevilacqua et al, Science, 243: 1160-1165, 1989); and the ICAM-1 protein is partially responsible for the adhesion of some mononuclear cells (Dustin and Springer, J. Cell Biol., 107: 321 - 331, 1988) and granulocytes (Smith et al, J. Clin. Invest., 83: 2008-2017, 1989; Smith et al, J. Clin. Invest., 82: 1746-1756, 1988) to activated endothelial cells. A significant point of agreement in these and other (Haskard et al, J. Immunol., 137: 2901-2906, 1986) studies is on the existence of ICAM-1 and ELAM-1 independent pathway(s) for the adherence of lymphocytes to activated endothelial cells.

Nonetheless, the role of these and other molecules in the binding of mononuclear cells remains to be clarified.

## SUMMARY OF THE INVENTION

Accordingly, one object of the present invention is to develop new monoclonal antibodies that are specific to antigens on activated human endothelial cells, both in vitro and in vivo.

A second object of the present invention is to develop new monoclonal antibodies that block binding of white blood cells, thereby making the monoclonal antibodies useful as medicaments and in methods for intervening in inflammatory responses associated with activated endothelial cells.

A third object of the present invention is to provide new antigens and/or new epitopes of antigens exhibited on activated endothelial cells which are useful as medicaments and in methods for intervening in inflammatory responses associated with activated endothelial cells.

A fourth object of the present invention is to provide a detection method capable of detecting inflammatory responses associated with activated endothelial cells.

These and other objects have been achieved by providing a monoclonal antibody or binding site-containing fragment thereof that binds specifically to IL-1 activated endothelial cells, wherein said monoclonal antibody is selected from the group consisting of

(A) a monoclonal antibody having the following identifying characteristics:

(1) does not bind significantly to normal resting endothelial cells;

(2) does not bind significantly to normal resting or IL-1 activated epidermal keratinocytes or resting or IL-1 activated fibroblasts;

(3) does not bind significantly to a mixture of resting or IL-1 activated granulocytes or mononuclear cells comprising T-cells, B-cells and monocytes;

(4) does not or partially inhibit(s) binding of T-cells to IL-1 activated endothelial cells in vitro;

(5) does not inhibit binding of granulocytes to IL-1 activated endothelial cells in vitro; and

(6) specifically binds to 1E7/2G7 sialoglycoprotein antigen found on the surface of IL-1 activated endothelial cells and defined by the cDNA sequence shown in Figures 34A to 34D;

(B) a monoclonal antibody having the following identifying characteristics:

(1) does not bind significantly to normal resting endothelial cells;

(2) does not bind significantly to normal resting or IL-1 activated epidermal keratinocytes or resting or IL-1 activated fibroblasts;

(3) does not bind significantly to a mixture of resting or IL-1 activated granulocytes or mononuclear cells comprising T-cells, B-cells and monocytes: and

(4) specifically binds to CMP-170 antigen present in the cytoplasm of resting and IL-1 activated endothelial cells and on the surface of IL-1 activated endothelial cells; and

(C) a monoclonal antibody having the following identifying characteristics:

(1) does not bind significantly to normal resting endothelial cells;

(2) does not bind significantly to normal resting or IL-1 activated epidermal keratinocytes or resting or IL-1 activated fibroblasts;

(3) does not bind significantly to a mixture of resting or IL-1 activated granulocytes or mononuclear cells comprising T-cells, B-cells and monocytes;

(4) partially inhibits binding of T-cells to activated endothelial cells in vitro;

(5) completely or partially inhibits binding of granulocytes to IL-1 activated endothelial cells in vitro;

(6) specifically binds to the N-terminal 20% of ELAM-1 sialoglycoprotein antigen present on the surface of IL-1 activated endothelial cells; and

(7) does not bind to 1E7/2G7 sialoglycoprotein antigen present on the surface of the IL-1 activated endothelial cells.

In a further embodiment, the present invention provides hybridoma cell lines that produce the above-described monoclonal antibodies.

In preferred embodiments, the monoclonal antibodies are monoclonal antibody 1E7, produced by hybridoma cell line 1E7 having ATCC Deposit No. HB 10136, monoclonal antibody 2G7 produced by hybridoma cell line 2G7 having ATCC Deposit No. HB 10137, monoclonal antibody 3A2 produced by hybridoma cell line 3A2 having ATCC Deposit No. HB 10138, monoclonal antibody 7A9 produced by hybridoma cell line 7A9 having ATCC Deposit No. HB 10135, and monoclonal antibody 3B7 produced by hybridoma cell line 3B7 having ATCC Deposit No. HB 10391, or binding site-containing fragments of these monoclonal antibodies.

In an even further embodiment, the present invention provides a medicament for treating inflammatory responses associated with activated endothelial cells, the medicament comprising:

(I) a pharmaceutically effective amount of a monoclonal antibody or binding site-containing fragment thereof, or pharmaceutically acceptable salts of the monoclonal antibody or the fragment, wherein the monoclonal antibody is at least one of the above-described monoclonal antibody (A) which partially inhibits binding of T-cells to activated endothelial cells in vitro or monoclonal antibody (C), and

(II) a pharmaceutically acceptable carrier, diluent or excipient.

In a preferred embodiment, the monoclonal antibodies are monoclonal antibody 2G7 produced by hybridoma cell line 2G7 having ATCC Deposit No. HB 10137, monoclonal antibody 7A9 produced by hybridoma cell line 7A9 having ATCC Deposit No.HB 10135 and monoclonal antibody 3B7 produced by hybridoma cell line 3B7 having ATCC Deposit No. HB 10391, or binding site-containing fragments of these monoclonal antibodies.

In still another embodiment, the present invention provides a method for treating inflammatory responses associated with activated endothelial cells, the method comprising administering to a subject in need of treatment a pharmaceutically effective amount of the monoclonal antibodies or binding site-containing fragments thereof or pharmaceutically acceptable salts of the monoclonal antibody or the fragment described for the medicament.

In a preferred embodiment the monoclonal antibodies are monoclonal antibody 2G7 produced by hybridoma cell line 2G7 having ATCC Deposit No. HB 10137, monoclonal antibody 7A9 produced by hybridoma cell line 7A9 having ATCC Deposit No. HB 10135 and monoclonal antibody 3B7 produced by hybridoma cell line 3B7 having ATCC Deposit No. 10391, or binding site-containing fragments of these monoclonal antibodies.

The present invention further provides a substantially pure antigen or antigenic fragment thereof found on IL-1 activated endothelial cells, wherein said antigen or antigenic fragment thereof is selected from the group consisting of:

(A) a 1E7/2G7 antigen having the following identifying characteristics:

(1) is a sialoglycoprotein;

(2) molecular mass, as determined by SDS-polyacrylamide gel electrophoresis under reducing conditions;

(a) major band at about 114 kDa,

(b) minor band at about 95 kDa;

(3) molecular mass, as determined by SDS-polyacrylamide gel electrophoresis under non-reducing conditions:

(a) major band at about 100 kDa,

(b) minor band at about 93 kDa;

(4) molecular mass, as determined by SDS-polyacrylamide gel electrophoresis under reducing conditions, reduced by about 1 to 2 kDa after removal of O-linked sugars;

(5) isoelectric point about 4.8 to 4.9, as determined by 2-D gel analysis;

(6) not expressed on constitutive or thrombin stimulated peripheral blood mononuclear cells, granulocytes, platelets, fibroblasts or keratinocytes;

(7) expression on IL-1 activated endothelial cells stimulated by E. coli lipopolysaccharide (lps) and tumor necrosis factor alpha (TNF) but not by gamma interferon (IFN$\gamma$);

(8) exhibits chronic kinetics as determined in vitro by ability of monoclonal antibody 1E7 and/or 2G7 to bind to human endothelial cells pretreated for increasingly longer periods of time with IL-1, said monoclonal antibody 1E7 being produced by hybridoma cell line 1E7 having ATCC Deposit No. HB 10136 and said monoclonal antibody 2G7 being produced by hybridoma cell line 2G7 having ATCC Deposit No. HB 10137;

(9) exhibits chronic and acute kinetics as determined in vivo by expression of the antigen in blood vessels of tissues undergoing either chronic or acute inflammation;

(10) does not bind to antibodies that bind specifically to ELAM-1 or ICAM-1;

(11) binds to the monoclonal antibody 1E7 and/or the monoclonal antibody 2G7; and

(12) expressed by COS cells transfected with the cDNA sequence shown in Figures 34A to 34D;

(B) a CMP-170 antigen having the following identifying characteristics:

(1) molecular mass, as determined by SDS-polyacrylamide gel electrophoresis under reducing and non-reducing conditions:about 170 kDa;

(2) not expressed on resting or IL-1 stimulated peripheral blood mononuclear cells, granulocytes, fibroblasts or keratinocytes;

(3) present in cytoplasm of resting endothelial cells, fibroblasts and leucocytes;

(4) expression at cell surface of activated endothelial cells inhibited by cycloheximide and actinomycin D;

(5) does not localize to Weibel-Palade bodies in endothelial cells;

(6) associates at cell surface with ELAM-1 in IL-1 stimulated endothelial cells;

(7) exhibits acute kinetics as determined in vitro by ability of monoclonal antibody 3A2 to bind to human endothelial cells pretreated for increasingly longer periods of time with IL-1, the monoclonal antibody 3A2 being produced by hybridoma cell line 3A2 having ATCC Deposit No. HB 10138;

(8) binds to the monoclonal antibody 3A2; and

(C) an antigen which consists of an antigenic fragment of ELAM-1 antigen having the following identifying characteristics:

(1) comprises about the N-terminal 31% of the ELAM-1 antigen;

(2) binds to monoclonal antibody 7A9 produced by hybridoma cell line 7A9 having ATCC Deposit No. HB 10135;

(3) binds to monoclonal antibody 3B7 produced by hybridoma cell line 3B7 paving ATCC Deposit No. HB 10391;

(4) not expressed on constitutive or thrombin stimulated peripheral blood mononuclear cells, granulocytes, platelets, fibroblasts or keratinocytes; and

(5) expression on IL-1 activated endothelial cells stimulated by E. coli lipopolysaccharide (lps) and tumor necrosis factor alpha (TNF) but not by gamma interferon (IFN$\gamma$).

The present invention also provides a medicament for treating inflammatory responses associated with activated endothelial cells, the medicament comprising:

(I) a pharmaceutically effective amount of a substantially pure antigen or antigenic fragment thereof, or pharmaceutically acceptable salts of the antigen or fragment wherein the antigen is at least one antigen selected from the group consisting of at least one of the above-described 1E7/2G7 antigen, the CMP-170 antigen and the ELAM-1 antigen fragment; and

(II) a pharmaceutically acceptable carrier, diluent or excipient.

The present invention further provides a method for treating inflammatory responses associated with activated endothelial cells, the method comprising administering to a subject in need of treatment a pharmaceutically effective amount of the substantially pure antigen or antigenic fragment thereof or pharmaceutically acceptable salts of the antigen or fragment described above for the medicament.

The present invention additionally provides a method for detecting inflammatory responses associated with IL-1 activated endothelial cells, the method comprising:

(I) contacting a biological fluid with any of the above-described monoclonal antibodies or binding site-containing fragments thereof, and

(II) assaying for specific binding of the monoclonal antibodies or binding site-containing fragments thereof to antigen in the biological fluid.

In a preferred embodiment, the monoclonal antibodies are monoclonal antibody 1E7, produced by hybridoma cell line 1E7 having ATCC Deposit No. HB 10136, monoclonal antibody 2G7, produced by hybridoma cell line 2G7 having ATCC Deposit No. HB 10137 and monoclonal antibody 3A2, produced by hybridoma cell line 3A2 having ATCC Deposit No. HB 10138, monoclonal antibody 7A9, produced by hybridoma cell line 7A9 having ATCC Deposit No. HB 10135 and monoclonal antibody 3B7, produced by hybridoma cell line 3B7 having ATCC Deposit No. 10391.

Finally, the present invention provides a cDNA sequence, or fragment thereof, and a vector carrying the cDNA sequence, or fragment thereof, that codes for antigen 1E7/2G7.

The cDNA sequence comprises the cDNA sequence substantially as shown in Figures 34A to 34D.

The vector comprises a vector carrying the cDNA sequence substantially as shown in Figures 34A to 34D.

## BRIEF DESCRIPTION OF THE FIGURES

Figure 1 is a schematic representation of the mechanism by which the novel monoclonal antibodies (MAb) of the present invention that block binding of white blood cells to IL-1 activated endothelial cells and by which the novel antigens (Peptide) according to the present invention act to block inflammation.

Figure 2 shows a flow cytometric analysis of uninduced (...) and IL-1 induced (—) human umbilical vein endothelial cells (HUVEC). On the horizontal axis, every 25 channels represents an approximate doubling of fluorescence intensity a.u. = arbitrary units. Cell number in arbitrary units is indicated on the vertical axis. In Figure 2A, 1 represents the intensity of fluorescence on endothelial cells stained with the indirect fluorescent goat anti-mouse reagent only. 2 represents the intensity of the W6/32 anti-HLA reagent which is a standard for comparison with following figures. In Figure 2B the solid line represents staining with the 1E7 antibody. Figures 2C, 2D, 2E and 2F represent staining with antibodies 2G7, 7A9, 3B7 and RR1 (anti-ICAM-1), respectively. The dotted line in Figures 2B to 2F represents staining with the indicated monoclonal antibody of uninduced HUVEC.

Figure 3 is a histogram showing the reactivity of monoclonal antibodies 1E7, 2G7 and 3B7 with 1E7/2G7 cDNA transfected COS cells. Results are expressed as CPM x $10^{-3}$ of $^{125}$I-streptavidin following biotinylated goat anti-mouse antibody.

Figure 4 is a graph showing inhibition of the binding of human mononuclear cells to IL-1 activated endothelial cells pretreated with different doses of the F(ab')$_2$ fragment of monoclonal antibody 2G7 or with 10 $\mu$g/ml of the monoclonal antibody 1E7 of the present invention.

Figure 5 is a histogram showing the effect of monoclonal antibodies on granulocyte adhesion to HUVEC. Results are expressed as cells bound per well ± s.e.m. (n = 3).

Figure 6 is a histogram showing the effect of monoclonal antibodies on T-cell adhesion to HUVEC. Results are expressed as cells bound per well ± s.e.m. (n = 3).

Figure 7 is an SDS-polyacrylamide gel electrophoresis (SDS-PAGE) pattern (10% acrylamide) of the antigens defined by the monoclonal antibodies 1E7 and 2G7 of the present invention under non-reducing conditions. Molecular weight standards are also shown.

Figure 8 is an SDS-polyacrylamide gel electrophoresis pattern (8% acrylamide) of the antigens defined by the monoclonal antibodies 3A2 and 7A9 of the present invention under reducing (Figure 8B) and non-reducing (Figure 8A) conditions. Molecular weight standards are also shown.

Figure 9 is a graph showing binding of the monoclonal antibodies according to the present invention to normal resting and IL-1 activated endothelial cells (EC).

Figure 10 is a graph showing binding of the monoclonal antibodies according to the present invention to normal human white blood cells, either granulocytes or mononuclear cells (T-cells, B-cells, and monocytes) in the absence of IL-1.

Figure 11 is a graph showing binding of the monoclonal antibodies according to the present invention to endothelial cells exposed to IL-1 for varying time periods from 2 hours to 96 hours.

Figure 12 is a 2-D gel pattern of TRITON X-114 detergent pellets (membrane proteins) from $^{35}S$-cysteine labeled quiescent (Figure 12A) and IL-1 (Figure 12B) activated endothelial cells. The three arrows on the top left of the uninduced cells (-IL-1) 2-D gel (Figure 12A) point to regions where induced proteins appear in the induced gel (Figure 12B) on the right (+IL-1). The apparent molecular weights and isoelectric points are indicated, based upon reference to known proteins in a database.

Figure 13 is a 2-D gel pattern of 1E7 and 2G7 immunoprecipitates alone and in mixtures of total membrane proteins from uninduced (-IL-1) and induced (+IL-1) HUVEC. In each of the eight squares is shown a portion of a 2-D gel which corresponds to the upper left quadrant of the total gel analyzed in Figure 12. Figures 13A and 13B are the immunoprecipitates alone with either the 1E7 (top) or 2G7 (bottom) antibody. No additional spots were seen in other portions of these gels. Figures 13C and 13D show the total membrane proteins from uninduced (-IL-1, top) or induced (IL-1, bottom) HUVEC with no added immunoprecipitate. Figures 13E and 13F show the effect of addition of the 1E7 (top) or 2G7 (bottom) immunoprecipitate to the membrane proteins of uninduced HUVEC. Figures 13G and 13H show the effect of addition of the 1E7 (top) or 2G7 (bottom) immunoprecipitates to the membrane proteins from IL-1 induced HUVEC. Plus (+) indicates the acidic and negative (-) the basic end of each gel.

Figure 14 is a 2-D gel pattern of immunoprecipitates alone and in combination. The monoclonal antibodies used for the immunoprecipitation were as follows: Figure 14A, RR1 (anti-ICAM-1); Figure 14B, 2G7; Figure 14C, 7A9; Figure 14D, RR1 + 2G7; Figure 14E, RR1 + 7A9; Figure 14F, 2G7 + 7A9. Only the upper left hand quadrant of each of the six 2-D gels is shown. No additional spots were present in other regions.

Figure 15 is a diagram of the pCDN-ELAM-1 expression plasmid. ELAM-1 cDNA was cloned into the plasmid (pCDN-1; T.V. Gopal, unpublished work) containing SV40 early promoter (area shown by diagonal bars) and SV40 "t" splice and polyA addition sequences (dotted area) derived from pSV2 NEO (Southern and Berg, J. Mol. Applied Genetics. 1: 327-341, 1982). The rests of the plasmid contained pBR322 (—) sequences. Important restriction sites are identified in the figure. MCS indicates multiple cloning site.

Figure 16 is a histogram of a monoclonal antibody binding assay of ELAM-1 transfected and mock transfected COS cells. Results are expressed as the mean (± s.e.m. n = 3) of the total cpm/well.

Figure 17 is a 10% SDS-polyacrylamide reducing gel pattern of confluent HUVEC treated with IL-1 without or with tunicamycin B2 and immunoprecipitated with either the 2G7 or the 7A9 monoclonal antibody.

Figure 18 is a 2-D gel pattern of HUVEC that were activated with IL-1 and metabolically labeled with $^{35}S$-cysteine. Cells were left untreated or treated for 30 minutes with neuraminidase prior to lysis. TRITON X-100 lysates were immunoprecipitated with either the 2G7 (Figure 18A) or 7A9 (Figure 18B) antibody. A portion of each of the four 2-D gels is shown in the figure.

Figure 19 is a 10% SDS-polyacrylamide reducing gel pattern of TRITON X-100 lysates of IL-1-activated, $^{35}S$-cysteine labeled HUVEC immunoprecipitated with either the 7A9 or 2G7 antibody. The immunoprecipitates were treated with either buffer alone, neuraminidase (N'ase) or neuraminidase followed by endo-$\alpha$-N-acetylgalactosaminidase (O-gly'case).

Figure 20 is a 10% SDS-polyacrylamide reducing gel pattern of HUVEC incubated with IL-1 for the indicated periods of time. Immunoprecipitates of TRITON X-100 lysates were formed with either the 2G7 or the 7A9 antibody.

Figure 21 shows histograms of competition assays performed with fluorescein conjugated monoclonal antibodies 1E7, 2G7, 3B7 or 7A9. Figure 21A : 1E7; Figure 21B : 2G7; Figure 21C : 3B7; Figure 21D : 7A9. On the vertical axis is the percent positive cells. Data is derived from flow cytometry.

Figure 22 is a graph showing the results of exposure of HUVEC to IL-1 for the indicated periods of time. Antibody 2G7 (●) or 7A9 (▲) was used in a sandwich binding assay at a saturating concentration. Results are expressed as the mean cpm ± s.e.m. (n=3).

Figure 23 shows flow cytometric analysis of quiescent (...) or IL-1 stimulated (-) endothelial cells stained with indirect reagent only (Figure 23A), the W6/32 anti-HLA antigen (Figure 23B), the anti-ICAM-1 antibody RR/1 (Figure 23C) and 3A2 antibody (Figure 23D).

Figure 24 shows an SDS-polyacrylamide reducing gel (8% acrylamide) pattern from metabolically labeled endothelial cells that were untreated (-) or IL-1 treated (+), lysed with 1% TRITON X-100 and immunoprecipitated with the indicated antibody. In the Figure, MAb 7 and MAb 3 represent monoclonal antibody 7A9 and 3B7, respectively.

Figure 25 shows histograms of endothelial cells treated with IL-1 and actinomycin D or cycloheximide and then tested in a plate binding assay for expression of the 2G7, 7A9 (anti-ELAM-1) or 3A2 antigens. Figure 25A : IL-1 ± cycloheximide; Figure 25B : IL-1 ± actinomycin D (act D).

Figure 26 shows a light microscopic evaluation of 3A2 antigen. Figure 26A : endothelial cells cultured in growth medium only and stained with antibody 7A9; Figure 26B : endothelial cells activated with IL-1 and stained with antibody 7A9; Figure 26C : endothelial cells cultured in growth medium only and stained with antibody 3A2; Figure 26D : endothelial cells activated with IL-1 and stained with antibody 3A2. The larger patches in Figures 26B, 26C and 26D are due to red color from the staining which indicates the presence of corresponding antigen. The small dark spots in Figure 26A are nuclei stained blue but are not surrounded by any red cytomplasmic staining.

Figure 27 shows SDS-polyacrylamide gel patterns for untreated (-) or IL-1 treated (+) endothelial cells immunoprecipitated with the 3A2 antibody (MAb 3) or with control goat anti-mouse IgM reagent only (MAb-). A portion of these immunoprecipitates was run on 8% SDS-polyacrylamide gels under non-reducing (Figure 27A) or reducing (Figure 27B) conditions. Figure 27C, first and second lanes : immunoprecipitates with monoclonal antibodies 7A9 or 2G7 (MAb 7 and MAb 2, respectively) from IL-1 activated cells (as controls); Figure 27C, third and fourth lanes : immunoprecipitates from the 3A2 immunoprecipitate with 7A9 or 2G7 antibody. Hence the second band co-precipitated with monoclonal antibody 3A2 (170 kDa) in Figure 27A and Figure 27B with the 7A9 and not the 2G7 antigen.

Figure 28 is an SDS-polyacrylamide reducing gel (10% acrylamide) pattern for 7A9 or 3A2 immunoprecipitates of COS cells subjected to mock transfection (-) or transfected with the ELAM-1 cDNA in the CDN plasmid (+). Monoclonal antibody 7A9 precipitates the ELAM-1 protein, monoclonal antibody 3A2 precipitates a COS cell endogenous 170 kDa antigen and co-precipitates ELAM-1.

Figure 29 shows SDS-polyacrylamide reducing gel (10% acrylamide) patterns for immunoprecipitates of endothelial cells pulsed with $^{35}$S-cysteine for either 45 or 120 minutes and either lysed after 45 minutes (Figure 29A, first two lanes) or chased for an additional 1 or 2 hours prior to lysis (Figure 29A remaining lanes). For Figure 29B, the endothelial cells had been exposed to IL-1 for 4 hours and then surface-labeled with $^{125}$I. The gels show the kinetics of 3A2 : ELAM-1 association. In the Figure MAb 3 and MAb 7 represent monoclonal antibodies 3A2 and 7A9, respectively.

Figure 30 is a graph showing the kinetics of cell surface expression of CMP-170 and 7A9 (ELAM-1) antigens. The open circles represent the 7A9 antigen and the open triangles represent the 3A2 antigen.

Figure 31 shows diagrams of various truncated forms of the antigen ELAM-1.

Figure 32 shows immunoprecipitation patterns of the intact antigen ELAM-1 and of various truncated forms of the antigen ELAM-1 with the monoclonal antibodies 3A2, 3B7 and 7A9. Antigenic fragments were obtained from the supernatants only of COS cells transfected with the indicated ELAM-1 construct and, 48 hours later, labeled with $^{35}$S-cysteine for 14 hours. The structures of the truncated forms are shown in Figure 31.

Figure 33 is a diagram of the VCAM-1 antigen and the 1E7/2G7 antigen.

Figures 34A to 34D show a cDNA sequence for bases 24 to 2243 (coding region) of the molecule coding for the 1E7/2G7 antigen. The corresponding amino acid sequence is also shown using standard one letter symbols for the amino acids.

## DETAILED DESCRIPTION OF THE INVENTION

The entire specification of co-pending U.S. Application No. 07/355,701, filed May 23, 1989, is expressly incorporated herein by reference.

The present invention provides novel hybridomas that produce novel murine monoclonal antibodies having the identifying characteristics described above, and in the Examples herein. The present invention also provides novel antigens to which the monoclonal antibodies specifically bind.

The phrase "does not bind significantly" as applied to the novel monoclonal antibodies of the present invention means that the binding is not statistically significant as compared to controls wherein no binding occurs.

The phrase "does not inhibit binding" as applied to the novel monoclonal antibodies of the present invention means that the inhibition is not statistically significant as compared to controls where no inhibition occurs.

The phrase "partially inhibits binding" as applied to the novel monoclonal antibodies of the present invention means that the inhibition is statistically significant as compared to controls wherein no Inhibition occurs, but that the inhibition is less then complete inhibition.

The phrase "completely inhibits binding" as applied to the novel monoclonal antibodies of the present invention means that the inhibition is complete within experimental error. In general, the experimental error with respect to inhibition of binding of cells such as T-cells, granulocytes, and/or monocytes is about ± 10%.

The phrase "inflammatory responses associated with activated endothelial cells" as applied to the novel monoclonal antibodies and to the antigens according to the present invention, means inflammatory responses characterized by the participation of endothelial cells, and the blood vessels wherein they reside, in the stable binding and/or extravasation of leucocytes from the blood stream into the tissues. Inflammation might consist in binding only, if this leads to leucocyte-mediated damage to the local vasculature. Damage may also result from actual passage through the blood vessel resulting in the damage to surrounding tissues from their interaction with any of the different leucocyte subtypes.

The term "sialoglycoprotein" as used herein means a protein defined by ability to incorporate $^{35}S$-cysteine whose mobility on polyacrylamide gels is increased as a result of the inhibition of glycosylation by tunicamycin and the removal of sialic acid by pretreatment of the immunoprecipitate or the activated endothelial cells.

The phrase "substantially pure" as applied to the antigens defined by the monoclonal antibodies of the present invention means that the antigens are not in their natural state. That is, they are not associated with the cells which exhibit the antigens or with any bodily fluid in which the antigens are found.

The term "chronic kinetics" with respect to in vitro assays as applied to the antigens defined by the novel monoclonal antibodies of the present invention means that the antigens continue to be expressed on human endothelial cells in the presence of IL-1 for a period of about 72 to 96 hours and that the expression is at levels above or about equal to the level initially reached upon addition of IL-1. Two examples of such behavior are shown in Figure 11 for the antigens defined by monoclonal antibodies 1E7 and 2G7 and in Figure 22 for the antigen defined by monoclonal antibody 7A9.

The term "acute kinetics" with respect to in vitro assays as applied to the antigens defined by the novel monoclonal antibodies of the present invention means that the antigens decrease and might disappear altogether from the surface of human endothelial cells in the presence of IL-1 within a period of about 24 hours from the initial addition of IL-1. One example of such behavior is shown in Figure 11 for the antigen defined by monoclonal antibody 3A2.

The term "chronic kinetics" with respect to in vivo assays as applied to the antigens as defined by the novel monoclonal antibodies of the present inventions means that the antigen is expressed in blood vessels of tissues undergoing chronic inflammation. This is a conventional determination made by a trained pathologist knowledgeable of the patient's history and is based upon the number and nature of infiltrating leucocytes, and other standard criteria.

The term "acute kinetics" with respect to in vivo assays as applied to the antigen as defined by the novel monoclonal antibodies of the present invention means that the antigen is expressed in blood vessels of tissue undergoing acute inflammation. This is a conventional determination made by a trained pathologist knowledgeable of the patient's history and is based upon the number and nature of infiltrating leucocytes, and other standard criteria.

## Hybridomas and Monoclonal Antibodies

The hybridomas according to the present invention can be produced reproducibly by routine experimentation according to established methods as is described in detail in Example 1 herein.

Generally, the immunogen for preparing immunized cells is IL-1 activated human endothelial cells.

Human endothelial cells can be obtained from umbilical cords according to known methods (Jaffe, et al., J. Clin. Invest., 52: 2745-2757, 1973). Briefly, cells are stripped from the blood vessel walls by treatment with collagenase and cultured in gelatin coated tissue culture flasks in M199 medium containing 20% low endotoxin fetal calf serum, 90 µg/ml preservative-free porcine heparin, 20 µg/ml endothelial cell growth supplement (ECGS), glutamine and antibiotics. ECGS is a crude growth factor preparation obtained from bovine hypothalamus, the key ingredient being fibroblast growth factor. ECGS is commercially available.

The endothelial cells are activated by addition of IL-1 beta at 1 ng/ml for 4 hours. IL-1 beta is readily available to those skilled in the art, both commercially and otherwise.

The particular host being immunized for eventual production of hybridomas should be a mouse. Balb/c mice are preferred.

The immunization schedule and amount of activated endothelial cells used for immunizing the mouse can readily be determined by the skilled artisan. By way of example, one suitable immunization schedule for Balb/c mice is to inject 3 x $10^6$ IL-1 activated human endothelial cells on 4 occasions, 10 days apart, the last injection being 3 to 4 days prior to fusion.

The sensitized cells, e.g., immunized spleen cells, are removed and splenocytes are fused with the SP2/0 myeloma cell line or other suitable myeloma cell line by well established techniques (Köhler, G. and Milstein, C., Nature, 256: 495-497, 1975 and Young, W.W., et al, J. Exp. Med., 150: 1008-1019, 1979). Fusions are preferably carried out with polyethylene glycol.

The particular myeloma cells employed in the present invention for fusion with the sensitized spleen cells of the immunized host are not critical thereto and can be any well known myeloma cell useful for preparing hybridomas of mouse origin. Examples of such myeloma cells include HAT sensitive mice myeloma cells such as NS/1, SPI and SP2/O cells.

The fused cells are cultured under conditions readily determined by the skilled artisan.

After an appropriate culture period, hybridomas, producing monoclonal antibodies that react with IL-1 activated endothelial cells but not with normal resting endothelial cells are cloned and subcloned by limiting dilution.

In general, screening is performed on confluent monolayers of resting or IL-1 activated endothelial cells. Culture supernatants are added to the monolayers of cells and incubated for a suitable period of time, e.g., 60 minutes at 4°C, to allow the monoclonal antibodies in the culture supenatants to react with the monolayers of cells. Antibody bound to an antigen-coated well is usually detected by secondary antibody, e.g., biotinylated anti-mouse IgM and IgG goat or rabbit antibodies, followed by a suitable radioactive probe, e.g., $^{125}$I-streptavidin.

Monoclonal antibodies produced by hybridomas thus isolated according to the present invention can be produced in quantity by growing large batches of hybridoma cell cultures and purifying the antibody from the supernatant or by injecting mice with the hybridoma line to stimulate the production of ascites fluid. Both methods are well known in the art.

According to the above-described method, five hybridomas that produce preferred monoclonal antibodies have been produced. The preferred monoclonal antibodies were designated 1E7, 2G7, 3A2, 7A9 and 3B7. The hybridomas producing these monoclonal antibodies, designated hybridoma 1E7, 2G7, 3A2, 7A9 and 3B7, have been deposited with the American Type Culture Collection, Rockville, Maryland, and have ATCC Deposit Nos. HB 10136, HB 10137, HB 10138, HB 10135 and HB 10391, respectively.

Monoclonal antibodies 1E7 and 2G7 according to the present invention are produced by hybridoma cell lines 1E7 and 2G7 and comprise two preferred monoclonal antibodies that fall within Group (A) Monoclonal Antibodies of the invention. The Group (A) Monoclonal Antibodies have the following identifying characteristics:

(1) do not bind significantly to normal resting endothelial cells;

(2) do not bind significantly to normal resting or IL-1 activated epidermal keratinocytes or resting or IL-1 activated fibroblasts;

(3) do not bind significantly to a mixture of resting or IL-1 activated granulocytes or mononuclear cells comprising T-cells, B-cells and monocytes;

(4) do not or partially inhibit binding of T-cells to activated IL-1 endothelial cells in vitro;

(5) do not inhibit binding of granulocytes to IL-1 activated endothelial cells in vitro; and

(6) specifically bind to 1E7/2G7 sialoglycoprotein antigen found on the surface of IL-1 activated endothelial cells and defined by the cDNA sequence shown in Figures 34A to 34D.

Of the above-identifying characteristics, a subgroup of monoclonal antibodies which includes monoclonal antibody 1E7 do not inhibit binding of T-cells to IL-1 activated endothelial cells in vitro and a subgroup of monoclonal antibodies which includes monoclonal antibody 2G7 partially inhibit binding of T-cells to IL-1 activated endothelial cells in vitro.

A further identifying characteristic of a subgroup of Group (A) Monoclonal Antibodies include that it partially inhibits binding of THP-1 myelomonocytic cells to IL-1 activated endothelial cells in vitro. This characteristic is exhibited by a subgroup of Group (A) Monoclonal Antibodies that includes the preferred monoclonal antibody 2G7.

Further identifying characteristics of preferred monoclonal antibody 1E7 are that the monoclonal antibody is a murine monoclonal antibody and has an isotype of IgG2a.

Further identifying characteristics of preferred monoclonal antibody 2G7 are that the monoclonal antibody is a murine monoclonal antibody and has an isotype of IgG1. Also, monoclonal antibody 2G7 partially inhibits binding of THP-1 myelomonocytic cells to IL-1 activated endothelial cells in vitro.

Monoclonal antibody 3A2 according to the present invention is produced by hybridoma cell line 3A2 and comprises one preferred monoclonal antibody that falls within Group (B) Monoclonal Antibodies of the invention. The Group (B) Monoclonal Antibodies have the following identifying characteristics:

(1) do not bind significantly to normal resting endothelial cells;

(2) do not bind significantly to normal resting of IL-1 activated epidermal keratinocytes or resting or IL-1 activated fibroblasts;

(3) do not bind significantly to a mixture of resting or IL-1 activated granulocytes or mononuclear cells comprising T-cells, B-cells and monocytes; and

(4) specifically bind to CMP-170 antigen present in the cytoplasm of resting and activated IL-1 endothelial cells and on the surface of IL-1 activated endothelial cells.

Further identifying characteristics of Group (B) Monoclonal Antibodies include that they bind specifically to CMP-170 found in resting fibroblasts and leucocytes.

Further identifying characteristics of preferred monoclonal antibody 3A2 are that the monoclonal antibody is a murine monoclonal antibody and has an isotype of IgM.

Monoclonal antibodies 7A9 and 3B7 according to the present invention are produced by hybridoma cell lines 7A9 and 3B7 and comprise two preferred monoclonal antibodies that fall within Group (C) Monoclonal Antibodies of the invention. The Group (C) Monoclonal Antibodies have the following identifying characteristics:

(1) do not bind significantly to normal resting endothelial cells;

(2) do not bind significantly to normal resting or IL-1 activated epidermal keratinocytes or resting or IL-1 activated fibroblasts;

(3) do not bind significantly to a mixture of resting or IL-1 activated granulocytes or mononuclear cells comprising T-cells, B-cells and monocytes;

(4) partially inhibit binding of T-cells to activated endothelial cells in vitro;

(5) completely or partially inhibit binding of granulocytes to IL-1 activated endothelial cells in vitro;

(6) specifically bind to the N-terminal 20% of ELAM-1 sialoglycoprotein antigen present on the surface of IL-1 activated endothelial cells; and

(7) does not bind to the 1E7/2G7 sialoglycoprotein antigen present on the surface of IL-1 activated endothelial cells.

With respect in the above-identified characteristics, a subgroup of monoclonal antibodies which includes monoclonal antibody 7A9 completely inhibit binding of granulocytes to IL-1 activated endothelial cells in vitro and a subgroup of monoclonal antibodies which includes monoclonal antibody 3B7 partially inhibit .binding of granulocytes to IL-1 activated endothelial cells in vitro.

Further identifying characteristics of preferred monoclonal antibody 7A9 are that it is a murine monoclonal antibody and has an isotype of IgG1.

Further identifying characteristics of preferred monoclonal antibody 3B7 are that it is a murine monoclonal antibody and its isotype is IgG2a.

Binding site-containing fragments of any of the above-described monoclonal antibodies can be obtained by numerous methods known in the art, such as, for example, pepsin digestion used to produce $F(ab')_2$ fragments. See Parham, J. Immunol., 131: 2893-2092, 1983.

A specific assay for determining the binding to normal resting endothelial cells is described in Example 2B.

However, in general, the assay can be conducts according to several known procedures in the art such as, for example, those described in Handbook of Experimental Immunology 4th Edition, 1986, D.M. Weir, editor, Vols 1-4, Blackwell Scientific Publications, Oxford, England.

Specific assays for determining binding to normal resting or IL-1 activated epidermal keratinocytes or resting or IL-1 activated fibroblasts are set forth in Example 3(B).

However, the assay can generally be conducted according to known methods in the art, such as those described in, for example, Handbook of Experimental Immunology 4th Edition, 1986, D.M. Weir, editor, Vols 1-4, Blackwell Scientific Publications, Oxford, England.

An assay for determining whether the above-described monoclonal antibodies bind to a mixture of resting or IL-1 activated granulocytes or mononuclear cells comprising T-cells, B-cells and monocytes is described in Example 3(B).

However, in general, several known methods can be used for this determination, such as those described in, for example, Handbook of Experimental Immunology 4th Edition, 1986, D.M. Weir, editor, Vols 1-4, Blackwell Scientific Publications, Oxford, England.

A specific assay for determining whether the above-described monoclonal antibodies inhibit binding of T-cells to IL-1 activated endothelial cells in vitro is set forth in Example 2(E).

However, any of several known methods is suitable, such as described in, for example, Masuyama and Kano, J. Clin. Invest., 77: 1596-1605, 1986.

A specific method for determining whether the above-described monoclonal antibody inhibits binding of granulocytes to IL-1 activated endothelial cells in vitro is described in Example 2(D).

However, any of several known methods is suitable, such as those described in, for example, Pober et al, J. Immunol., 136: 1680-1687, 1986.

Whether the above-described monoclonal antibodies specifically bind to the 1E7/2G7 sialoglycoprotein antigen, CMP-170 antigen and ELAM-1 sialoglycoprotein antigen can be determined by conducting flow cytometry as described in Example 2(B).

A specific method for determining whether the above-described monoclonal antibodies bind to the N-terminal 20% of the ELAM-1 sialoglycoprotein antigen is described in Example 5.

## Novel Antigens and Antigenic Fragments

The present invention also provides novel substantially pure antigens and antigenic fragments of antigens found on IL-1 activated endothelial cells. The antigens and antigenic fragments are designated 1E7/2G7, CMP-170 and novel antigens which are antigenic fragments of the known antigen ELAM-1.

The 1E7/2G7 antigen has the following identifying characteristics:

(1) is a sialoglycoprotein;

(2) molecular mass, as determined by SDS-polyacrylamide gel electrophoresis under reducing conditions;

(a) major band at about 114 kDa,

(b) minor band at about 95 kDa;

(3) molecular mass, as determined by SDS-polyacrylamide gel electrophoresis under non-reducing conditions:

(a) major band at about 100 kDa,

(b) minor band at about 93 kDa;

(4) molecular mass, a determined by SDS-polyacrylamide gel electrophoresis under reducing conditions, reduced by about 1 to 2 kDa after removal of O-linked sugars;

(5) isoelectric point about 4.8 to 4.9, as determined by 2-D gel analysis;

(6) not expressed on constitutive or thrombin stimulated induced peripheral blood mononuclear cells, granulocytes, platelets, fibroblasts or keratinocytes;

(7) expression on IL-1 activated endothelial cells stimulated by E. coli lipolysaccharide (lps) and tumor necrosis factor alpha (TNF) but not by gamma interferon ($IFN_{\gamma}$);

(8) exhibits chronic kinetics as determined in vitro by ability of monoclonal antibody 1E7 and/or 2G7, to bind to human endothelial cells pretreated for increasingly longer periods of time with IL-1, the monoclonal antibody 1E7 being produced by hybridoma cell line 1E7 having ATCC Deposit No. HB 10136 and said monoclonal antibody 2G7 being produced by hybridoma cell line 2G7 having ATCC Deposit No. HB 10137;

(9) exhibits chronic and acute kinetics as determined in vivo by expression of the antigen in blood vessels of tissues undergoing either chronic or acute inflammation;

(10) does not bind to antibodies that bind specifically to ELAM-1 or ICAM-1;

(11) binds to the monoclonal antibody 1E7 and/or the monoclonal antibody 2G7; and

(12) expressed by COS cells transfected with the cDNA sequence shown in Figures 34A to 34D.

Useful fragments of the 1E7/2G7 antigen include an antigenic fragment comprising an epitope that binds to the monoclonal antibody 1E7 and not the to the monoclonal antibody 2G7 as well as an antigenic fragment comprising an epitope that binds to the monoclonal antibody 2G7 but not the monoclonal antibody 1E7.

Additional identifying characteristics of the 1E7/2G7 antigen include that its molecular mass, as determined by SDS-polyacrylamide gel electrophoresis under reducing conditions, after tunicamycin treatment to prevent carbohydrate attachment, is about 80 kDa.

The CMP-170 antigen has the following identifying characteristics:

(1) molecular mass, as determined by SDS-polyacrylamide gel electrophoresis under reducing and non-reducing conditions: about 170 kDa;

(2) not expressed on surface of resting or IL-1 stimulated peripheral blood mononuclear cells, granulocytes, fibroblasts or keratinocytes;
(3) present in cytoplasm of resting endothelial cells, fibroblasts and leucocytes;
(4) expression at cell surface of activated endothelial cells inhibited by cycloheximide and actinomycin D;
(5) does not localize to Weibel-Palade bodies in endothelial cells;
(6) associates at cell surface with ELAM-1 in IL-1 stimulated endothelial cells;
(7) exhibits acute kinetics as determined in vitro by ability of monoclonal antibody 3A2 to bind to human endothelial cells pretreated for increasingly longer periods of time with IL-1, the monoclonal antibody 3A2 being produced by hybridoma cell line 3A2 having ATCC Deposit No. HB 10138; and
(8) binds to the monoclonal antibody 3A2.

The antigen which consists of an antigenic fragment of the ELAM-1 antigen has the following identifying characteristics:

(1) comprises about the N-terminal 31% of the ELAM-1 antigen;
(2) binds to monoclonal antibody 7A9 produced by hybridoma cell line 7A9 having ATCC Deposit No. HB 10135;
(3) binds to monoclonal antibody 3B7 produced by hybridoma cell line 3B7 having ATCC Deposit No. HB 10391; and
(4) not expressed on constitutive or thrombin stimulated peripheral blood mononuclear cells, granulocytes, platelets, fibroblasts or keratinocytes; and
(5) expression of IL-1 activated endothelial cells stimulated by E. coli lipopolysaccharide (lps) and tumor necrosis factor alpha (TNF) but not by gamma interferon (IFN$\gamma$).

The method for determining molecular mass by SDS-polyacrylamide gel electrophoresis under reducing and non-reducing conditions is well known in the art and is described, for example, in Two Dimensional Electrophoresis and Immunological Techniques, Bonnie S. Dunbar, Plenum Press, New York, 1987.

In describing the molecular mass, the terms "major band" and "minor band" are relative terms based on the intensities of the bands in a given experiment. However, the skilled artisan can readily determine whether a band is a major band or a minor band by, for example, observing the figures in the present application.

The isoelectric point of the antigen is also determined by methods well known in the art such as by 2-D gel analysis as described in Garrels, Methods Enzymol., 100: 411-423, 1983 and in Two Dimensional Electrophoresis and Immunological Techniques, Bonnie S. Dunbar, Plenum Press, New York, 1987.

O-linked sugars can be removed by known methods such as that described in Example 4(F).

Other methods that can be used to remove O-linked sugars are described in Handbook of Experimental Immunology 4th Edition, 1986, D.M. Weir, editor, Vols 1-4, Blackwell Scientific Publications, Oxford, England.

A specific example of an assay for determining whether the antigens of the present invention are or are not expressed on resting or thrombin or IL-1 stimulated peripheral blood mononuclear cells, granulocytes, platelets, fibroblasts or keratinocytes is set forth in Example 4(J) and (M).

Other methods that can be used to determine whether the antigens are or are not expressed on resting or thrombin or IL-1 stimulated cells are described in Handbook of Experimental Immunology 4th Edition, 1986, D.M. Weir, editor, Vols 1-4, Blackwell Scientific Publications, Oxford, England.

A specific method for determining whether the expression of the antigens of the present invention is stimulated on IL-1 activated endothelial cells by lps, TNF and/or IFN$\gamma$ is described in Example 4(K).

A method for determining whether the antigens according to the present invention exhibit chronic kinetics and/or acute kinetics in vitro is described in Example 3(C) and 4(I).

Whether the antigens according to the present invention exhibit chronic and/or acute kinetics in vivo can be determined by observing expression of the antigen in blood vessels of tissues undergoing either chronic or acute inflammation. This is a conventional determination made by a trained pathologist knowledgeable of the patient's history and is based upon the number and nature of infiltrating leucocytes, and other standard criteria.

Whether the antigens bind to particular monoclonal antibodies can be determined by conducting flow cytometry as described in Example 2(B).

A specific method for determining whether an antigen is present in the cytoplasm of resting endothelial cells is described in Example 4(P).

Briefly, cells are permeabilized with, for example, acetone or ethanol, in order to allow antibody to react with antigen present inside the cells. Cells are then visualized by conventional methods after reacting with an indirect probe that reacts with the antibody. See, for example, Figure 26, where alkaline phosphatase conjugated to goat anti-mouse antibody was used as an indirect probe.

A specific method for determining whether expression of antigens at the cell surface of activated endothelial cells is inhibited by cycloheximide and actinomycin D is described in Example 4(O).

Other methods that can be used in place of the one described above include employing flow cytometry conducted as described in Example 2(B).

Conventional methods can be used to determine whether an antigen localizes to Weibel-Palade bodies. The methods involve determining whether the antigen co-localizes (as observed using light microscopy) with von-Willibrand Factor (vWF) -- the major component of Weibel-Palade bodies.

Briefly, suitable cells, such as quiescent human endothelial cells, are cultured, detached using, e.g., PBS-EDTA, and cytospun onto glass microscope slides. The cells are fixed with acetone and dried to permeabilize the cells. The cells are then stained by a double staining protocol, such as described in Example 4(P), with the antibody of interest and anti-vWF antibody (commercially available). The stained cells are examined for double staining of vWF.

Additionally, from cells stained only with the antibody of interest, the skilled artisan can readily determine whether the antibody is in Weibel-Palade bodies.

Two specific methods for determining whether an antigen associates at the cell surface with ELAM-1 in IL-1 stimulated endothelial cells are described in Example 4(Q).

Other methods that can be used in place of those described above include use of cross-linking reagents at the cell surface and co-immunoprecipitation as described in Handbook of Experimental Immunology 4th Edition, 1986, D.M. Weir, editor, Vols 1-4, Blackwell Scientific Publications, Oxford, England.

The novel antigens and antigenic fragments can be prepared and purified by conventional methods some of which are described in detail in the Examples.

The purification of the ELAM-1 fragments can be by the immunoprecipitation protocol given in Example 4(B). For a larger scale purification, the 1E7 or 2G7 monoclonal antibody (e.g., 5 mg) can be conjugated to Affigel-10 beads (e.g., 5 ml) (commercially available) by the standard protocol supplied by the manufacturer and fluids (supernatants of cells producing the ELAM-1 fragments) allowed to interact with the antibody coated beads. The beads are rinsed with PBS-TWEEN to remove non-specifically bound material, and the antigen fragments are eluted by several standard techniques such as described for ICAM-1 fragments in Marlin et al, Nature, 344: 70-72, 1990.

For use in a soluble form, the antigens or antigenic fragments should be synthetically produced. The antigens can be cloned and sequenced according to known methods (see, for example, Bevilacqua et. al, Science, 243: 1160-1164, 1989). Determination of the active site or sites is also by standard techniques (see, for example, Peterson and Seed, Cell, 54: 65-72, 1988) as is synthesis of the peptide fragments.

The novel antigens and antigenic fragments can be used to assay for blocking binding of white blood cells to activated endothelial cells in order to substantiate whether the monoclonal antibodies according to the present invention block such binding. The skilled artisan can readily determine suitable assay conditions.

As an example, the mononuclear cells whose binding is being assayed are preincubated with the antigen or antigenic fragment in a suitable buffer and at a suitable concentration and temperature for a time sufficient to saturate all antigen binding sites. After preincubation, the mononuclear cells, in the continued presence of peptide, are added to activated endothelial cells. The non-adherent monocellular cells are washed off by rinsing with buffer and the number of cells bound to the activated endothelial cells is determined. As a control, a parallel-assay can be performed using unactivated endothelial cells.

Detection can be performed in a quantitative manner by incorporating a radioactive tracer, e.g., $^{51}Cr$, by methods known in the art, into the cytoplasm of the cells whose binding is being studied, washing to remove any extracellular counts and placing these labeled cells over the endothelial cell monolayer.

One assay according to the present invention for detecting blocking of binding of white blood cells to activated endothelial cells is described in detail in Example 4(U).

The novel antigens of the present invention are useful in their soluble form to block the interaction of white blood cells with activated endothelial cells, as described in more detail below in the section entitled "Medicaments and Methods for Treating Inflammatory Responses".

## Medicaments and Methods for Treating Inflammatory Responses

The present invention also provides medicaments and methods for treating inflammatory responses associated with activated endothelial cells.

In one embodiment, the medicament comprises:

(I) a pharmaceutically effective amount of a monoclonal antibody or binding site-containing fragment thereof or pharmaceutically acceptable salts of the monoclonal antibody or the fragment, wherein the monoclonal antibody is at least one of the monoclonal antibodies from Group (A) which partially inhibits binding of T-cells to activated endothelial cells in vitro of the monoclonal antibodies from Group (C), both described previously, and

(II) a pharmaceutically acceptable carrier, diluent or excipient.

In a second embodiment, the medicament comprises:

(I) a pharmaceutically effective amount of a substantially pure antigen or antigenic fragment thereof, or pharmaceutically acceptable salts of the antigen or fragment, wherein the antigen is at least one antigen selected from the group consisting of the previously described 1E7/2G7 antigen, the CMP-170 antigen and the antigenic fragment of ELAM-1.

Similarly, in one embodiment the method comprises administering to a subject in need of treatment a pharmaceutically effective amount of a monoclonal antibody or binding site-containing fragment thereof, or pharmaceutically acceptable salts of the monoclonal antibody or the fragment, wherein the monoclonal antibody is at least one of the above-described monoclonal antibodies from Group (A) which partially inhibits binding of T-cells to activated endothelial cells in vitro or the above-described monoclonal antibodies from Group (C).

In a second embodiment, the method comprises administering to a subject in need of treatment a pharmaceutically effective amount of a substantially pure antigen or antigenic fragment thereof, or pharmaceutically acceptable salts of the antigen or fragment, wherein the antigen is at least one antigen selected from the above-described 1E7/2G7 antigen, the CMP-170 antigen and the antigenic fragment of ELAM-1.

The monoclonal antibodies useful as medicaments and in the methods of treatment are the same as those described above that are capable of blocking binding of white blood cells to IL-1 activated endothelial cells in vitro, and in preferred embodiments, the monoclonal antibodies are 2G7, 7A9 and 3B7, described above.

Chimeric antibodies comprising the complementarity determining regions (CDR) of the antibodies of the present invention and the non-CDR regions of a suitable human immunoglobulin can also be used (Queen et al, Proc. Natl. Acad. Sci. U.S.A., 86: 10029-10033, 1989).

The antigens useful as a medicament and in the methods of treatment are all of the novel antigens described above. However, the antigens described above to which the monoclonal antibodies 2G7, 7A9 and 3B7 specifically bind are expected to be especially useful.

Suitable pharmaceutically acceptable salts can readily be determined by the skilled artisan.

Suitable pharmaceutically acceptable carriers, diluents or excipients can readily be determined by the skilled artisan. Examples include isotonic saline (0.15M NaCl) and Ringer's glucose intravenous solution.

The medicaments can be administered by methods readily determined by the skilled artisan, e.g., intravenously.

Suitable doses to be administered might vary depending upon the nature of the inflammatory disorder, but can readily be determined by the skilled artisan.

In general, a suitable dose for intravenous injection to humans is about 20 to 50 mg of the monoclonal antibody or binding site-containing fragment thereof or antigen or antigenic fragment thereof.

Figure 1 is a simplified schematic representation of how the medicaments and methods of treatment according to the present invention are believed to work.

The medicaments and methods of treatment are applicable to any inflammatory response associated with activated endothelial cells, and can be used for acute as well as chronic inflammations.

Of course combinations of the various medicaments can always be used, as long as the novel antigen is not combined in one medicament with its corresponding monoclonal antibody.

One skilled in the art can readily determine the inflammatory responses for which the medicaments and methods of treatment of the present invention will be useful. Examples include tumor-cell-mediated vascular damage, rheumatoid arthritis, post-reprefusion myocardial injury (damage by granulocytes) and adult respiratory distress syndrome (macrophages and granulocytes). Examples are also disclosed in Simpson, P.J. et al, J. Clin. Invest., 81: 624-629, 1988; Vedder, N.B. et al, J. Clin. Invest., 81: 939-944, 1988; Simon and Ward "Adult Respiratory Distress Syndrome" in Inflammation: Basic Principles and Clinical Correlates - (Gallin, J. I., Goldstein, I.M. and Snyderman. R.: eds) Raven Press, New York, 1988, page 815; Kadison and Barton "Vasculitis: Mechanisms of Vessel Damage Id. page 703; and Harris "Pathogenesis of Rheumatoid Arthritis: A Disorder Associated with Dysfunctional Immunoregulation Id. page 751.

## Method of Detecting Inflammatory Responses

The present invention also provides a method for detecting inflammatory responses associated with activated endothelial cells, the method comprising:

(I) contacting a biological fluid with at least one of the above-described monoclonal antibodies from Group (A), (B) or (C) or binding site-containing fragments thereof, and

(II) assaying for the specific binding of the monoclonal antibody or binding site-containing fragment thereof to antigen in the biological fluid.

The method of producing and purifying the monoclonal antibodies has already been described above.

The phrase "biological fluid" means serum, urine, synovial fluid and any other body fluid expected to contain antigens produced from inflammation.

Detection can occur either in vitro or in vivo. In vitro detection can be carried out using any of the well known in vitro immunological assays, such as those described by Young, W.W. et al, J. Exp. Med., 150: 1008-1019 (1979) and Kannagi, R. et al, Cancer Res., 43: 4997-5005 (1983). Further, in vivo, detection can be carried out using any of the well known in vivo immunological assays, such as those described in Burchell, J. et al, Int. J. Cancer. 34: 763-768 (1984); Epenetos, A.A. et al, Lancet, 2: 999-1004 (1982); Chatal, J.-F. et al, J. Nuclear Med., 26: 531-537 (1985).

An especially preferred method for the detection of antigen in biological fluids is a sandwich ELISA technique. This method in preferred form consists of the attachment of monoclonal antibodies to one epitope of the protein of interest to a plastic NUNC ELISA dish. This is followed by adding bovine serum albumin in a 1% solution to block non-specific binding of proteins in biological fluids. After rinsing wells to remove unbound proteins, the biological fluids are added (blood, urine, etc.) to the dish for a 1 hour period. After washing to remove unbound biological fluid, the second monoclonal antibody to another epitope of the protein of interest is added. The second monoclonal antibody is conjugated with a suitable reporter molecule such as alkaline phosphatase. After a period of incubation, the wells are washed to remove unbound protein and a suitable enzyme substrate (if the reporter molecule is an enzyme), such as aminoethyl carbazole, is added and the reaction product is quantitated on an ELISA plate reader. An example of conducting a sandwich ELISA is described in Lokeshwar and Lin, J. Immunol. Methods. 123: 123-129 (1989).

The method can be used, for example, to detect early graft rejection or subclinical infections or vasculitis.

## cDNA Sequence and Vector Carrying cDNA of 1E7/2G7 Antigen

The present invention also provides a cDNA sequence, or fragment thereof, and vector carrying the cDNA sequence, or fragment thereof, that codes for antigen 1E7/2G7.

The cDNA sequence comprises the sequence substantially as shown in Figures 34A to 34D, provided that bases 952 to 1227 are present substantially as shown in Figure 34B.

By "substantially as shown" it is meant that the cDNA sequence shown in Figures 34A to 34D can have deletions, additions and/or substitutions of the bases:

(1) as long as the cDNA sequence can still be used as a probe to isolate mRNA that directs the synthesis of the antigen 1E7/2G7 or antigenic fragments thereof, or

(2) as long as the ligand binding properties of the ligand for which the cDNA sequence codes are maintained.

Fragments of the cDNA sequence substantially as shown or as shown in Figures 34A to 34D, are fragments of the cDNA sequence that code for fragments of the 1E7/2G7 antigen that maintain the ligand binding properties.

Whether a cDNA sequence or fragment thereof can still be used as a probe to isolate mRNA that directs the synthesis of the antigen 1E7/2G7 or antigenic fragments thereof can readily be determined by the skilled artisan by conventional methods and without undue experimentation.

Whether a cDNA sequence or fragment thereof codes for a protein that maintains its ligand binding properties can also be readily determined by the skilled artisan by conventional methods and without undue experimentation as follows.

First the skilled artisan would establish that the solubilized form of 1E7/2G7 can be made, and that in that form it does bind to cells. The soluble form is made by deleting the cytoplasmic and transmembrane regions. Basically a stop codon is inserted in the coding sequence, preventing these regions from being translated. COS cells are transfected with this detected form of the cDNA by conventional methods, e.g., $Ca^{++}$ shock, and are grown in the presence of the $^{35}S$ isotopes of cysteine and methionine which is incorporated into the growing COS cells and therefore into the translated antigen. After about 48 hours the supernatant of these cells is harvested and the binding of the antigen to the cell surface, by comparison to mock transfected cells, is measured as follows.

A suitable cell type such as the cell lines RAMOS and JURKAT, available from the ATCC, or normal human peripheral blood mononuclear cells (one million) are incubated with 1 ml of the culture supernatant of the mock or transfected cell for about 1 hour on ice. After the incubation cells are washed by centrifugation with Dulbecco's PBS containing 1% RIA grade BSA. The washed cells are lysed by placing them in 100 µl of 1% SDS detergent at room temperature for 5 minutes. The entire lysed cell pellet is then added to suitable scintillation fluid and the bound radioactivity is measured in a scintillation counter. The specifically bound cpm is determined by subtracting the cpm obtained from cells incubated in the presence of supernatant from that obtained from mock transfected cells.

The minimum size binding fragment is then determined by constructing a series of deleted cDNA molecules by placing the stop codon closer and closer to the N terminus, expressing the protein, and testing its binding in the above assay.

The cDNA sequence can be prepared as described in Example 5, using commercially available mammalian expression vectors such as pEUK-C1, pEUK-C2 and pMAM-neo in place of the plasmid pCDN-1.

The vector carrying the cDNA sequence can be any of numerous vectors known in the art.

If the goal is simply to amplify the cDNA sequence, the vector can be any of numerous known vectors such as pBR322, pSP72 and pUC, all of which are commercially available.

If the goal is to express the cDNA sequence, the vector can be any of numerous known mammalian expression vectors that contain expression control sequences, such as pEUK-C1 , pEUK-C2 and pMAMneo, all of which are commercially available.

The cDNA sequences can be inserted into the vectors by conventional methods and the vectors can be propagated and expressed by conventional methods.

## EXAMPLES

The present invention will now be described by reference to specific examples, which are not meant to be limiting.

Unless otherwise specified all percents, ratios, parts, etc. are by volume.

The reagents used in the Examples were obtained and/or treated as follows: TRITON X-114 was obtained from Kodak. It was extracted three times with 10 mM Tris buffer pH 7.4, 0.15M NaCl to remove impurities. TRITON X-100, porcine intestinal heparin, and collagenase type 1 were purchased from Sigma. TWEEN 20 was purchased from Aldrich. Phenylmethylsulfonyl fluoride (PMSF) was purchased from Calbiochem. Gelatin was purchased from Bio Rad. Biotinylated goat anti-mouse light chain reagent and FITC-goat anti-mouse reagent was purchased from Jackson Laboratories. Affinity purified goat anti-mouse IgG was purchased from Kirkegaad and Perry Laboratories, and $^{125}$I-streptavidin and $^{35}$S-cysteine were purchased from Amersham. Recombinant human IL-1 beta was a gift from Dr. Y. Hirai of the Tokushima Research Institute. The material was purified from E. coli and gave a single band at 17 kDa on SDS-polyacrylamide gel (15% acrylamide) electrophoresis under reducing conditions: IL-1 suitable for use in this and following examples can also be obtained commercially. Tumor necrosis factor alpha (TNF) was purchased from Genzyme. Human $\alpha$ thrombin, with a specific activity of 3008 U/mg protein, was obtains courtesy of John W. Fenton. E. coli lipopolysaccharide (lps) was obtained from Difco. Tunicamycin as its B2 homologue was purchased from Boehringer-Mannheim. T4, T8, B1 and Mo2 monoclonal antibodies were purchased from the Coulter Corporation. The W6/32 antibody was purchased from Accurate Chemical and Scientific Co., the RR/1 antibody was a gift of Dr. Timothy Springer, the 10E5 antibody was a gift of Dr. Barry Coller, and the MOPC-21 myeloma protein was purified from culture supernatants of the P3X63Ag8 cell line obtained from ATCC.

## EXAMPLE 1

## PREPARATION OF MONOCLONAL ANTIBODIES SPECIFIC TO ACTIVATED HUMAN ENDOTHELIAL CELLS

**(A) Cell Culture**: Discarded umbilical cords, obtained with permission of the hospital review board, were processed according to the procedure of Jaffe (J. Clin. Invest., 52: 2745-2756, 1973) with minor modifications to obtain human umbilical vein endothelial cells (HUVEC). Briefly, cells were stripped from the blood vessel walls by treatment with collagenase and cultured in gelatin coated tissue culture flasks in the medium described below. Cells were determined to be greater than 95% endothelial by virtue of the presence of Weibel-Palade bodies and uptake of acetylated low density lipoprotein by viable cells. Cells were cultured in M199 medium containing 20% low endotoxin fetal calf serum (FCS, Hyclone), 90 µg/ml preservative-free porcine heparin, 20 µg/ml endothelial cell growth supplement (ECGS) (from collaborative Research), glutamine and antibiotics (hereinafter "growth medium"). ECGS was prepared from bovine hypothalamus by saline extraction in a chilled blender, followed by stirring for 2 hours at 4°C and centrifugation at 14,000g to remove insoluble material. After removal of lipid and filtration through 0.45 micron filters, aliquots were freeze dried. Human granulocytes were obtained from heparinized peripheral blood from which the mononuclear cells had been removed by centrifugation over FICOLL-HYPAQUE. The granulocyte/red cell pellet was sedimented over 3% dextran to remove the majority of red cells. Remaining red cells, when necessary, were lysed with hypotonic saline. Purity of granulocytes was greater than 95%, as determined by Weight-Giemsa staining. Human T-cells were prepared from the mononuclear layer after FICOLL-HYPAQUE separation. These cells were depleted of B-cells and monocytes by passage over nylon-wool columns. The resulting cells contained fewer than 5% Mol or B1 positive cells, and the sum of the T4 plus T8 bearing cells was 85-90% as determined by flow cytometry with commercially available monoclonal reagents. Normal human epidermal keratinocytes were obtained from the Clonetics Corp.; fibroblasts were obtained by explant from human foreskin tissue and human platelets were obtained from acid-citrate-dextrose anticoagulated blood of normal donors. Platelets were washed several times and resuspended in $Ca^{++}$-free PBS containing 1% BSA.

**(B) Hybridoma Production:** Female Balb/c mice at 8 weeks of age were injected intraperitoneally with $3x10^6$ IL-1 (4 hours) activated HUVEC on 4 occasions, 10 days apart, the last injection being 3 to 4 days prior to fusion. Spleens were aseptically removed, minced with a syringe plunger, washed and mixed with the SP2/O fusion partner (Fazekas de St. Groth, S. and Scheidegger, D., J. Immunol Methods 35: 1-21, 1980) in a ratio of five to 1. The cells were fused with polyethylene glycol as previously described (Kohler, G. and Milstein, C., Nature, 256: 495-497, 1975) and plated into 6 to 8 96-well microtiter plates containing resident peritoneal exudate cells from Balb/c mice as a feeder layer. Screening was performed on confluent monolayers of resting or IL-1 activated HUVEC (activated by addition of IL-1 at 1 ng/ml for 4 hrs) also in 96-well plates. After allowing 60 minutes at 4°C for the culture supernatants to react with the monolayers of HUVEC, cells were washed and incubated with biotinylated goat anti-mouse light-chain antibody for 30 minutes at 4°C. After 3 more rinses, cells were incubated for 15 minutes at 4°C with 0.3 µCl of $^{125}$I-streptavidin per well. After final rinses, cells were lysed with 1% TRITON X-100 detergent solution, and aliquots counted in a gamma counter. Hybridomas were selected based upon their ability to secrete antibodies which reacted exclusively with IL-1 treated but not with untreated HUVEC.

According to this method, five hybridoma cell lines were obtained that produced the desired monoclonal antibodies. These hybridoma cell lines and the monoclonal antibodies they produced were designated 1E7, 2G7, 3A2, 7A9 and 3B7. The hybridomas have been deposited with the American Type Culture Collection in Rockville, Maryland and have Deposit Nos. HB 10136, HB 10137, HB 10138, HB 10135 and HB 10391, respectively.

In addition, the above protocol was performed 13 separate times on a total of 71 microtiter plates of candidate hybridomas. In the first fusion the 1E7 hybridoma cell line was obtained. In the 8th, the 2G7 and 3A2 hybridoma cell lines were obtained. The 7A9 hybridoma cell line was obtained in the 10th and the 3B7 hybridoma cell line in the 13th fusion. Therefore two of 13 experiments yielded a monoclonal antibody (1E7 and 2G7) of the desired characteristics for the monoclonal antibodies of Group (A) described in the Detailed Description of the Invention; one of 13 experiments yielded a monoclonal antibody (3A2) of the desired characteristics for the monoclonal antibodies of Group (B) described in the Detailed Description of the Invention; and two of 13 experiments yielded a monoclonal antibody (7A9 and 3B7) of the desired characteristics for the monoclonal antibodies of Group (C) described in the Detailed Description of the Invention. This is not undue experimentation for the skilled artisan, and there are no critical steps missing in this experimental procedure. Thus the method of producing the monoclonal antibodies of Group (A), (B) and (C) described above and in the Detailed Description of the Invention is a reproducible method.

Unless otherwise specified, all monoclonal antibodies used in subsequent examples were purified by HPLC on DEAE ion-exchange columns or by affinity chromatography on protein A-Sepharose by conventional methods.

## EXAMPLE 2

## CHARACTERIZATION OF MONOCLONAL ANTIBODIES 1E7, 2G7, 3A2, 7A9 and 3B7

**(A) Isotype Determination:** Isotype was determined by using "ScreenType", a kit containing all of the necessary reagents, from Boehringer-Mannheim, Indianapolis, IN. The isotype determination was performed according to the manufacturer's instructions.

The results, which are summarized in Table I, were as follows: 1E7 -- IgG2a; 2G7 -- IgG1; 3A2 --IgM; 7A9 -- IgG1 and 3B7 -- IgG2a.

**(B-1) Binding of Monoclonal Antibodies 1E7, 2G7, 7A9 and 3B7 to Resting and IL-1 Activated Endothelial Cells:** Antibodies 1E7, 2G7, 7A9 and 3B7 were developed in plate binding assays of IL-1 treated versus untreated HUVEC as described in Example 1. Their reactivity with control or IL-1 treated endothelial cells was analyzed by flow cytometry as described below and compared to an anti-HLA monoclonal antibody, W6/32, and an antibody to ICAM-1, RR/1. All antibodies were used at a saturating doses determined in preliminary experiments.

Briefly, endothelial cells activated for 4 hours with IL-1 were treated in microtiter wells with concentrations of antibody ranging from 0.1 to 10 µg/ml, washed, and then incubated with FITC-goat anti-mouse indirect reagent. The cells were analyzed by flow cytometry and the minimum dose at which maximum fluorescence was obtained was taken as the saturating dose.

Flow cytometry was carried out as follows: Endothelial cells were detached from culture vessels with PBS containing 2 mM EDTA for 5 minutes at 37° C. A single cell suspension was made by gently pipeting the detaching cells with PBS/EDTA containing chilled 1% radioimmune assay (RIA) grade BSA and 0.02% sodium azide. One half million cells were incubated in round bottom 96-well plates in a volume of 100 µl containing a saturating dose of monoclonal antibody in PBS-1% BSA and 0.02% sodium azide (wash buffer). After 30 minutes incubation at 4° C, cells were washed 3 times and incubated in 100 µl of wash buffer containing FITC-goat anti-mouse reagent (Jackson ImmunoResearch Laboratories) at 1.5 µg/ml. For controls, cells were incubated with second-step reagent only. After an additional 30 minutes incubation at 4° C, cells were washed 3 times and resuspended for analysis. In practice the FITC-goat and-mouse reagent is used to set the background level of fluorescence. The cutoff point is set to include 95% of the cells treated with FITC-avidin alone. The proportion of cells shifted to greater fluorescence intensity after specific ligand binding determines the percent positive cells. Flow cytometry was performed on a Coulter Model 541 equipped with an argon laser and a quartz flow cell.

The results are shown in Figure 2 which shows detached cells stained with FITC-goat and-mouse (FGαM) reagent above (Figure 2A #1) or W6/32 anti-HLA plus GαM (Figure 2A #2) or FGαM following the monoclonal antibodies 1E7 (Figure 2B), 2G7 (Figure 2C), 7A9 (Figure 2D), 3B7 (Figure 2E) or RR1 (ICAM-1) (Figure 2F). All reagents were used at saturating doses. Each histogram represents an analysis of 25,000 cells. On the horizontal axis, every 25 channels represents an approximate doubling of fluorescence intensity a.u. = arbitrary units. Cell number in arbitrary units is indicated on the vertical axis. Uninduced (...) and IL-1 induced (—) HUVEC.

Figure 2 shows that the ICAM-1 protein is present on unstimulated HUVEC and increases approximately 8-fold after IL-1 stimulation, as previously reported (Dustin and Springer, J. Cell Biol., 107: 321-331, 1988), while the HLA protein does not increase after IL-1 stimulation. However, all four of the new antibodies assayed are undetectable on unstimulated cells and show a heterogeneous distribution of antigen, from weakly to strongly positive, within the stimulated HUVEC population. All these antigens, at their maximum fluorescence intensity, are comparable in their abundance to HLA proteins (Figure 2A #2) whose mean fluorescence intensity is approximately at channel 150. The heterogeneity in the expression of these activation antigens of HUVEC precluded a meaningful Scatchard analysis of the numbers of sites/cell. This heterogeneity was also evidenced when cells in monolayers on cover slips were examined, and was not influenced by the time or dose of IL-1 exposure (data not shown).

**(B-2) Binding of Monoclonal Antibodies 1E7, 2G7 and 3B7 to 1E7/2G7 cDNA Transfected COS Cells:** Binding was performed on 1E7/2G7 cDNA or mock transfected COS cells, prepared as described in Example 5. COS cells (2 x $10^4$/well) were plated into 96-well flat bottom microtiter wells 24 hours prior to assay and 48 hours after transfection. Cells were incubated with medium alone (control) or separately with each of the monoclonal antibodies at 5 µg/ml for 60 minutes at 4° C. After 3 rinses, cells were then incubated with biotinylated goat anti-mouse antibody for 30 minutes at 4° C. After 3 more rinses, cells were incubated for 15 minutes at 4° C with 0.3 µl of $^{125}$I-streptavidin per well. After final rinses, cells were lysed with 1% TRITON X-100, and aliquots were counted in a gamma counter.

The results are shown in Figure 3. Figure 3 shows that monoclonal antibodies 1E7 and 2G7, but not 3B7 (anti-ELAM-1) bind to the 1E7/2G7 cDNA transfected COS cells.

**(C) Blocking Activity of Monoclonal Antibodies:** Endothelial cells were obtained from discarded umbilical cords and cultured as described in Example 1. Cells multiplied rapidly and were subdivided (passage) up to 6 times into other flasks before their differentiated properties declined, perhaps as a result of the in vitro growth conditions. By the second passage sufficient cells were available to perform blocking assays. Recombinant human IL-1 beta was added at a concentration of 1 ng/ml to cells in culture for 4 to 6 hours while the cells were maintains as usual in a humidified incubation chamber containing 6% $CO_2$, 94% air. Controls consisted of culturing endothelial cells in the absence of IL-1. After the incubation period, cells were rinsed with medium to remove unused IL-1, and the respective monoclonal antibodies were added at concentrations ranging from 0.0016 to 10 µg/ml for 1 hour at 37°C. The $F(ab')_2$ fragment of monoclonal antibody 2G7, obtained by conventional methods, was also used for this assay. The cells whose binding was being studied were overlaid for 30 to 60 minutes to allow attachment. The cells used were human monocytes, granulocytes or a mixture of mononuclear cells comprising T-cells, B-cells and NK cells. These cells were isolated and purified by methods known in the art. The non-adherent cells were washed off by rinsing with growth medium, and the difference in the number of cells bound to endothelial cells (EC) untreated or treated with IL-1 was observed.

This assay was performed in a quantitative manner by incorporating a radioactive tracer, e.g., $^{51}Cr$, by methods known in the art, into the cytoplasm of the cells whose binding was being studied, washing to remove any extracellular counts, and placing these labeled cells over the EC monolayer. The $^{51}Cr$ label used for this purpose showed minimal leakage during the 30 to 60 minutes of the binding assay. After rinsing to remove non-adherent cells, the monolayers were solubilized with detergent. The radioactivity counted from each well was a measure of the number of cells bound.

The results showing inhibition of mononuclear cell binding by different doses of the $F(ab')_2$ fragment of monoclonal antibody 2G7 and 10 µg/ml of monoclonal antibody 1E7 are shown in Figure 4. The results show that monoclonal antibody 2G7, but not monoclonal antibody 1E7, when used to pretreat IL-1 activated endothelial cells, causes dose-dependent partial inhibition of the binding of human mononuclear cells to endothelial cells. The $F(ab')_2$ fragment of monoclonal antibody 7A9 also showed dose-dependent inhibition of cells as shown in Table I.

The results for all four monoclonal antibodies tested are summarized in Table I.

TABLE I

**Comparison of Monoclonal Antibodies of Present Invention With Published Antibodies**

| Name | Origin or Ref. | Isotype | Species | Blocking Activity | Immunogen* | Reference |
|---|---|---|---|---|---|---|
| H4/18 | Pober et al | IgG1 | Mouse | None | IL-1-EC | J.Immunol, 1366: 1680, (1986) |
| H18/7 | Bevilacqua et al | IgG2a | Mouse | Partial Granulocytes | TNF-EC | P.N.A.S., 84: 9238, (1987) |
| 1E7 | Invention | IgG2a | Mouse | None | IL-1-EC | |
| 2G7 | Invention | IgG1 | Mouse | Partial T-Cells and Perhaps B-Cells or Granulocytes | | |
| S12 | McEver et al | IgG1 | Mouse | Not Done | Platelets | J.Biol.Chem., 259: 9799, (1984) |
| KC4 | Hsu-Lin et al | IgG1 | Mouse | Not Done | Platelets | J.Biol.Chem., 259: 9121, (1984) |
| 3A2 | Invention | IgM | Mouse | Not Done | IL-1-EC | |
| 4D10 | Goerdt et al | IgG2a | Rat | None | 1ps 24h | Exp.Cell.Biol., 55: 117, (1987) |
| 7A9 | Invention | IgG1 | Mouse | Granulocytes Total, Monocytes Partial | IL-1-EC | |

* Abbreviations are as follows: IL-1-EC (interleukin-1 activated endothelial cells); TNF-EC (tumor necrosis factor activated endothelial cells); lps 24h (24h endotoxin (lipopolysaccharide, LPS) activated endothelial cells).

Table I compares properties of the monoclonal antibodies according to the present invention with those of the closest published antibodies. H4/18 is the first antibody raised against IL-1 stimulated endothelial cells (Pober. et al, J. Immunol., 136: 1680-1687, 1986). H18/7 is a monoclonal antibody raised against IL-1 stimulated endothelial cells and it partially blocks granulocyte adhesion to IL-1 stimulated endothelial cells (Bevilacqua et al, Proc. Natl. Acad. Sci., 84: 9238-9241, 1987). S12 is a monoclonal antibody that binds to activated platelets and is most closely compared to monoclonal antibody 3A2 of the present invention (McEver and Martin, J. Biol. Chem., 259: 9799-9804, 1984). Unexpectedly, however, monoclonal antibody 3A2 has an IgM isotype while that of S12 is IgG1. The IgM isotype is somewhat unusual for a monoclonal antibody. KC4 is an antiplatelet monoclonal antibody (Hsu-Lin et al, J. Biol. Chem., 259: 9121-9126, 1984). Of published monoclonal antibodies, KC4 has the closest specificity to that of monoclonal antibody 3A2 of the present invention. However monoclonal antibody KC4 has an isotype of IgG1 whereas monoclonal antibody 3A2 has the unexpected isotype of IgM. 4D10 is a rat monoclonal antibody having specificity for IL-1 stimulated endothelial cells (Goerdt et al, Expl. Cell Biol., 55: 117-126, 1987). Monoclonal antibody 4D10 has no obvious relation to the antibodies of the present invention. However, the antigen to which monoclonal antibody 4D10 binds is closest in molecular weight to the antigen to which monoclonal antibody 7A9 binds.

The comparisons in Table I demonstrate that the monoclonal antibodies according to the present invention have unexpected properties over the closest published monoclonal antibodies.

**(D) Inhibition of Granulocyte Binding:** The relative importance of the four epitopes on the two proteins, 1E7/2G7 and 3B7/7A9 (ELAM-1), in the adherence of unstimulated granulocytes to IL-1 activated HUVEC was examined as follows.

HUVEC at passage 4 or less were plated onto gelatin precoated 24-well plates and cultured for 48 hours or until confluent. Cell cultures were exposed to 1 ng/ml of IL-1 in RPMI-1640 containing 10% FCS, or cells were cultured in RPMI-1640 plus 10% FCS (wash medium) alone. After 4 hour exposure to IL-1 at 37°C, cultures were rinsed once with wash medium. Monolayers were incubated at this point with $F(ab')_2$ fragments of antibodies diluted in wash medium for 30 minutes at 37°C. (Preliminary results had established the necessity of using $F(ab')_2$ fragments to avoid bridging of HUVEC and granulocytes via FCS receptors on the latter.) Control wells contained wash medium only or 10 $\mu$g/ml of the purified myeloma protein, MOPC-21. All experimental points were performed in triplicate. Monolayers were overlaid, in the presence of antibody, with approximately $10^6$ $^{51}$Cr-labeled granulocytes in a volume of 0.5 ml. After 45 minutes at 37°C, monolayers were gently rinsed twice with wash medium at room temperature. The remaining adherent granulocytes were lysed with 300 $\mu$l of 1% TRITON X-100 in PBS. One half this volume (150 $\mu$l) was counted in a gamma counter. The percent inhibition of binding of granulocytes by monoclonal antibodies was calculated as follows: [(experimental-basal cpm) ÷ (IL-1 induced cpm - basal cpm)] x 100. The IL-1 minus basal value represents the inducible degree of binding. The amount of $^{51}$Cr taken up by $10^6$ washed granulocytes was used to convert data from cpm/well to number of cells bound/well. Monolayers were always inspected prior to lysis to ensure that no detachment of HUVEC had occurred.

$F(ab')_2$ fragments were prepared according to the procedure of Parham (Parham, J. Immunol., 131, 2895-2902, 1983). Pepsin was added to purified monoclonal antibody in 0.1 M citrate buffer, pH 4.1 at a w/w ratio of 1:50. Digestion proceeded overnight at 37°C. Removal of pepsin was accomplished by ion exchange chromatography. Digestion was monitored in reducing and non-reducing SDS-polyacrylamide gels (10% acrylamide), and binding activity to IL-1-activated HUVEC was established by flow cytometry (conducted as described above) to be equivalent to that obtained with undigested antibody.

Methods and reagents for performing reducing and non-reducing SDS-polyacrylamide gel electrophoresis are described in Two Dimensional Electrophoresis and Immunological Techniques, Bonnie S. Dunbar, Plenum Press, New York, 1987.

The results for a representative experiment are shown in Figure 5. Results are expressed as cells bound per well ± s.e.m. (n = 13).

The results indicate that the anti-ELAM-1 antibodies are more inhibitory than are antibodies 1E7 and 2G7. Summarizing results from six experiments, 1E7 and 2G7 gave 0% and 3% inhibition of granulocyte binding at the highest dose tested (10 $\mu$g/ml), while the 3B7 antibody gave a mean of 81% inhibition and the 7A9 antibody gave a mean of 95%, i.e., complete, inhibition, both at 10 $\mu$g/ml. Even though the 3B7 and 7A9 antibodies define non-overlapping epitopes of ELAM-1, their effects do not seem to be additive (data not shown). For both 7A9 and 3B7, significant inhibition of granulocyte binding was seen with a dose as low as 0.4 $\mu$g/ml. Since granulocytes do not express either the 1E7/2G7 or the 3B7/7A9 (ELAM-1) antigen (Table IV), the skilled artisan can conclude that the effects seen are due to the action of antibodies at the level of the endothelial cell.

**(E) Inhibition of T-cell binding:** The ability of monoclonal antibodies 1E7, 2G7, 7A9 and 3B7 to inhibit binding of T-cells to IL-1 activated endothelial cells was determined by the method described for inhibition of granulocyte binding except that nylon-wool non-adherent T-cells were substituted for granulocytes and 0.9 x $10^6$ T-cells were added to each well.

The results obtained when these four antibodies were tested for blocking ability in the adhesion of unstimulated nylon-wool non-adherent T-cells to IL-1-activated endothelial cells is shown in a representative experiment in Figure 6. In the combination experiments shown at the right in Figure 6, the final concentration of each antibody was 10 µg/ml. The phenotypic analysis showed 54% CD4+, 32% CD8+ , 2% Mo2+ (CD14), and 4% B1+ (CD20). Results are expressed as cells bound/well ± s.e.m. (n = 3).

Figure 6 shows that as with granulocytes, the 1E7 antibody shows no inhibition of binding at the highest dose tested (10 µg/ml). The MOPC-21 myeloma protein (IgG1) also showed no inhibition of binding of T-cells in separate experiments (not shown). However, partial inhibition was consistently seen with each of the other three antibodies in a dose-dependent manner. In six experiments, the mean percent inhibition seen with the 2G7 antibody was 34%, while values of 21% and 37% were obtained for the 3B7 and 7A9 antibodies, respectively. Inasmuch as these antibodies define distinct epitopes on different structures, and 3 of 4 are inhibitory, mixing experiments were performed and a representative experiment is shown to the right in Figure 6. Hence, the two anti-ELAM-1 antibodies, 3B7 and 7A9, each at 10 µg/ml, gave 31% inhibition, a value not additive of the results obtained with either 3B7 (19%) or 7A9 (40%), individually. However, when either anti-ELAM-1 antibody was combined with the 2G7 antibody (each at 10 µg/ml) additive values were obtained. Hence, the 2G7 plus 7A9 combination gave 68% inhibition (versus 29% with 2G7 and 40% with 7A9 individually) and 2G7 plus 3B7 gave 66% inhibition (versus 29% with 2G7 and 19% with 3B7). These results were confirmed in two additional experiments. The p values for comparison of the inhibition seen with the individual antibodies in Figure 6 relative to IL-1, show that the 2G7, 3B7, and 7A9 antibodies individually give statistically significant inhibition (p values 0.0009, 0.01, and 0.002, respectively). The combination of 2G7 with either 3B7 or 7A9 is significantly better than either antibody alone (p values of all combinations less then 0.001). The combination of 3B7 plus 7A9 is not significantly better than either antibody alone. Since neither the 1E7/2G7 nor the 3B7/7A9 antigen is present on peripheral blood lymphocytes, 70% of which are T-cells, the effects shown in Figure 15 can be attributed to the role of antigen on the endothelial cells.

## EXAMPLE 3

## CHARACTERIZATION OF SPECIFICITY OF MONOCLONAL ANTIBODIES OF PRESENT INVENTION WITH PUBLISHED ANTIBODIES

**(A) Antigen Size:** Confluent cultures of low passage (less than p6) human umbilical vein endothelial cells (HUVEC) in 100 mm tissue culture dishes were placed overnight in a low methionine containing medium. The next day, IL-1 was added to 1 ng/ml and $^{35}$S-methionine (Amersham) was added in the ratio of 250 µCi/$10^7$ cells. IL-1 was not added to controls. After 6 hours of culture at 37°C, the monolayers were rinsed three times with ice cold Dulbecco's phosphate buffered saline (D-PBS), and lysed with 250 µl of a 1% solution of TRITON X-100 lysing buffer.

Nunc ELISA 96-well microtiter plates were incubated with 25 µg of affinity purified goat anti-mouse antibody in 100 µl of 0.05M carbonate buffer, pH 9.5 for 1 hour at room temperature. Wells were rinsed ten times with PBS-0.05% TWEEN 20, followed by addition of 100 µl of 1% BSA for 1 hour at room temperature. To each well, in the presence of BSA, was added 5 µg of purified monoclonal antibody for an additional 1 hour at room temperature. Following ten washes with PBS-TWEEN, 1-3x$10^6$ cpm of lysate was added to each well at 4° C for 2 hours with gentle rocking. Wells were washed with PBS-TWEEN, dried and extracted with reducing sample buffer and applied to SDS-polyacrylamide (SDS-PAGE) gels, either reducing or non-reducing. The gels were 8% acrylamide for the 3A2 and 7A9 samples and 10% acrylamide for the 1E7 and 2G7 samples.

The results are shown in Figures 7 and 8 and are summarized in Table III. The results were as follows (the values in parenthesis are the average of results obtained from three experiments:

(1) monoclonal antibody 1E7 and monoclonal antibody 2G7 lysates showed a major band at 125 (114) kDa and a minor band at 97 (95) kDa under reducing conditions and a major band at 99 (100) kDa and a minor band at 87 (93) kDa under non-reducing conditions (results under reducing conditions are not shown in the figures);

(2) monoclonal antibody 3A2 lysates showed one band at 177 (170) kDa under reducing and non-reducing conditions, and;

(3) monoclonal antibody 7A9 lysates showed one band at 100 kDa under reducing conditions and one band at 90 kDa under non-reducing conditions.

As is shown in Example 4 below, the antigen to which monoclonal antibodies 1E7 and 2G7 specifically bind are the same antigen as are the antigens to which monoclonal antibodies 7A9 and 3B7 specifically bind.

**(B) Antigen Distribution:** A general binding assay was performed as follows: 20,000 HUVEC were plated in each well of a 96 well microtiter plate on a gelatin coated surface. Cells were left overnight in growth medium (See Example 1) to allow full attachment. The following day cells were exposed to 1 ng/ml IL-1 for 4 hours, washed and incubated separately with each of the monoclonal antibodies at 5 μg/ml. Controls consisted of HUVEC not exposed to IL-1, or exposed to IL-1 but not exposed to antibody. After 1 hour incubation at 4°C, cells were washed and incubated with biotinylated goat anti-mouse reagent at a 1/1000 dilution. After 30 minutes at 4°C, cells were rinsed and exposed to a 1/10 dilution of $^{125}$I-Streptavidin. After 15 minutes at 4°C, cells were rinsed and lysed with TRITON X-100 detergent (1%), and aliquots were counted in a gamma counter.

For assay of non-adherent cells such as T-cells, B-cells and granulocytes, plates were spun in a centrifuge to pellet cells prior to removal of medium. Cells were resuspended in appropriate medium for the next incubation.

The results are shown in Figure 9. Figure 9 shows that all four monoclonal antibodies, 1E7, 3A2, 7A9 and 2G7 bind to IL-1 activated human endothelial cells (HEC), but not significantly to normal resting HEC. This indicates that the antigens to which monoclonal antibodies 1E7, 3A2, 7A9 and 2G7 bind are found on IL-1 activated HEC in accordance with the results reported in Example 2.

The presence of the antigen corresponding to each of the four monoclonal antibodies was determined as in the general binding assay except that in addition to endothelial cells other cell types were used and the incubation time (as above) with IL-1 was for 4 hours. The other cell types were human fibroblasts (MRHF), keratinocytes (NHEK) and endothelial cells (HUVEC).

The results are shown in Table II below.

TABLE II

| ANTIGEN DISTRIBUTION OF MAbs 1E7, 2G7, 3A2 & 7A9 USING HUVEC, NHEK AND MRHF | | | | | |
|---|---|---|---|---|---|
| Cells + IL-1 | 1E7 | 2G7 | 3A2 | 7A9 | No MAb |
| HUVEC | 40,140±769 | 16,389±1888 | 6,425±343 | 15,891±362 | 439±86 |
| NHEK | 1,042± 72 | 768 ± 25 | 1,570±106 | 578±20 | 627±21 |
| MRHF | 1,189±309 | 511 ± 28 | 638± 92 | 513±38 | 391±27 |
| Cells - IL-1 | 1E7 | 2G7 | 3A2 | 7A9 | No MAb |
| HUVEC | 1,934±60 | 1,565±130 | 806±179 | 1,814±47 | 293±63 |
| NHEK | 959±51 | 696± 53 | 1,651±191 | 676±26 | 647±50 |
| MRHF | 863±60 | 521± 60 | 608± 69 | 448±29 | 495±73 |

The data in Table II show that all four monoclonal antibodies, 1E7, 3A2, 7A9 and 2G7 bind to IL-1 activated HEC, but not significantly to normal resting or IL-1 activated human epidermal keratinocytes or resting or IL-1 activated human fibroblasts. This data indicates that the antigens to which monoclonal antibodies 1E7, 3A2, 7A9 and 2G7 bind are found on IL-1 activated HEC.

The same protocol as for the general binding assay was used except that instead of using endothelial cells, granulocytes and mononuclear cells (T-, B- and monocyte cells) were used. Granulocytes and mononuclear cells were isolated from human heparinized blood by Ficoll-Hypaque separation according to known methods.

The results are shown in Figure 10. Figure 10 shows that the four monoclonal antibodies 1E7, 3A2, 7A9 and 2G7 do not bind significantly to a mixture of normal resting or IL-1 activated human granulocytes or mononuclear cells.

This data along with that from Figure 9 and Table II indicate that the monoclonal antibodies 1E7, 3A2, 7A9 and 2G7 are specific for IL-1 activated HEC.

**(C) Antigen Kinetics In Vitro:** HUVEC were plated in different petri dishes (35 mm diameter) after gelatin coating of the plastic. Growth medium (described in Example 1) was added and the cells were cultured for the duration of the experiment in growth medium to which was added 1 ng/ml a IL-1. The times at which the cells were initially plated were staggered such that all cells were harvested on the same day but after different times of addition of IL-1 so that some cells were exposed to IL-1 for the last 12 hours only, the full 96 h, or for intermediate times. The HUVEC were detached from petri dishes using PBS/EDTA, washed twice in PBS-BSA and placed at 75,000 cells per well in round bottom microtiter plates. To these wells were added individually each of the four monoclonal antibodies, 1E7, 2G7, 3A2 and 7A9, at 5 $\mu$g/ml for 30 minutes at 4° C. After washing three times the cells were exposed to FITC-goat anti-mouse reagent at 1/40 dilution for 30 minutes at 4°C. After washing three additional times, the cells were examined for the degree of fluorescence on a Coulter model 541 EPICS flow cytometer according to established and routine procedures provided by the manufacturer. The results are shown in Figure 10 and we expressed as the % positive (cells) vs. the length of exposure to IL-1.

The data in Figure 11 show that the antigens defined by monoclonal antibodies 1E7 and 2G7 increase in the presence of IL-1 and continue to be expressed in the presence of IL-1. In contrast, the antigen defined by monoclonal antibody 3A2 disappears with time. No zero time point is shown in this figure, the first time point is 4 hours, by which point the 4 antigens we maximally exhibited.

Behavior such as that shown by the antigens defined by monoclonal antibodies 1E7 and 2G7 was designated as "chronic kinetics" to suggest that these antigens might be associated with chronic inflammation. Transient expression was designated as "acute kinetics" to suggest that these antigens might be associated with acute inflammation.

The data from Example 3 constitute additional identifying characteristics of the monoclonal antibodies of the present invention and of the novel antigen according to the present invention. The data we summarized in Table III and compared with similar data for the closest known published monoclonal antibodies.

TABLE III

| COMPARISON OF SPECIFICITY OF MONOCLONAL ANTIBODIES OF PRESENT INVENTION WITH PUBLISHED ANTIBODIES | | | | | |
|---|---|---|---|---|---|
| Name | Ag Size (Kd).[1/] | | Ag Distribution[2/] | Ag Kinetics | Remarks |
| | Reducing | Non-Reducing | | | |
| 1E7 | 125, 97 | 99, 87 | IL-1-EC | Chronic | Invention |
| 2G7 | 125, 97 | 99, 87 | IL-1-EC | Chronic | Invention |
| 3A2 | 177 | 177 | IL-1-EC | Acute | Invention |
| 7A9 | 100 | 90 | IL-1-EC | Chronic | Invention |
| H 4/18; H 18/7 | 115, 97[3/] | nd | IL-1-EC[4/] | Acute[5/] | Published |
| 4D10 | unknown | 81[6/] | IL-1-EC[7/] | Acute[8/] | Published |
| S12 | 148[9/] | 138[10/] | Platelets, EC[11/] | unknown | Published |
| E 1/6 | 110[12/] | nd | nd | Chronic | Published |

[1/] Underline signifies major band.
[2/] Abbreviations are as follows: IL-1-EC (interleukin-1 activated endothelial cells; EC (resting endothelial cells)
[3/] Taken from Bevilacqua, Proc. Natl. Acad. Sci., 84: 9238-9242, 1987.
[4/] Taken from Bevilacqua, Proc. Natl. Acad. Sci., 94: 9238-9242, 1987.
[5/] Taken from Pober et al, J. Immunol., 136: 1680-1687, 1986 and taken from Bevilacqua, Proc. Natl. Acad. Sci., 84: 9238-9242, 1987.
[6/] Taken from Goerdt, et al, Expl. Cell Biol. 55: 117-126, 1987.
[7/] Taken from Goerdt, et al, Expl. Cell Biol. 55: 117-126, 1987.
[8/] Taken from Goerdt, et al, Expl. Cell Biol. 55: 117-126, 1987.
[9/] Taken from McEver and Martin, J. Biol. Chem., 259: 9799-9804, 1984.
[10/] Taken from McEver and Martin, J. Biol. Chem., 259: 9799-9804, 1984.
[11/] Taken from McEver and Martin, J. Biol. Chem., 259: 9799-9804, 1984.
[12/] Taken from Rice et al, Science, 246: 1303-1305, 1989.

The data in Table III demonstrate that the monoclonal antibodies according to the present invention are unexpectedly different from the closest known published monoclonal antibodies.

## EXAMPLE 4

## DETAILED ANALYSIS OF THE ANTIGENS 1E7/2G7, 1A7/3B7 AND CMP-170

**(A) 2-D Gel Analysis of Membrane Proteins From Ouiescent and IL-1 Stimulated HUVEC:** In order to obtain an estimated of the number of new membrane proteins synthesized by endothelial cells alter short-term exposure to IL-1, detergent pellets from TRITON X-114 lysed cells were examined by 2-D gel analysis as follows.

Confluent cultures of low passage (less than p6) HUVEC in 100 mm tissue culture dishes were placed overnight in growth medium containing D-MEM (Gibco) without cysteine plus 20% dialyzed fetal calf serum and other components of endothelial cell growth medium. The next day, IL-1 was added at 1 ng/ml and $^{35}$S-cysteine (Amersham) was added in the ratio of 250 $\mu$Ci/$10^7$ cells. After 6 hours of culture at 37 °C, the monolayers were rinsed three times with ice cold Dulbecco's phosphate buffered saline (D-PBS), and lysed with 250 $\mu$l of a 1% solution of TRITON X-114 lysing buffer containing 10 mM Tris pH 7.4, 0.15M NaCl, and 2mM PMSF. The separation of hydrophobic from aqueous phase proteins was accomplished essentially as described by Bordier, J. Biol. Chem., 256: 1604-1607, 1981. After 5 minutes, cell lysates were scraped from the dish into a 1.5 ml Eppendorf tube, spun at 12,000 g for 10 minutes at 4 °C, and the supernatant layered over 300 $\mu$l of 6% sucrose, 10 mM Tris pH 7.4, 0.15M NaCl, and 0.06% TRITON X-114. The gradient was placed at 37 °C for exactly 1 minute to accomplish the phase separation, and centrifuged at 850 g for 10 minutes at room temperature. The supernatant aqueous phase was removed and combined with fresh 10% TRITON X-114 to bring the concentration to 1% detergent while at 4 °C. This re-extracted supernatant was placed over the original sucrose gradient/TRITON X-114 detergent pellet, brought to 37 °C for 1 minute, and recentrifuged as before. This operation finally consisted of the initial separation of detergent and aqueous phases followed by three reextractions of the aqueous phase by detergent re-addition. The combined detergent pellets for each sample were diluted by addition of 9 parts buffer (10 mM Tris pH 7.4, 0.15M NaCl) to one part pellet. This material was reextracted by placing it over a sucrose cushion, incubating for 1 minute at 37 °C, and recentrifuging as described above. The last aqueous phase and the sucrose cushion were discarded, and the detergent pellet frozen at -70 °C until the 2-D gel analysis.

This procedure has been shown to favor the partitioning of membrane proteins because of their hydrophobic transmembrane regions, into the hydrophobic (detergent) rather than the aqueous phase (Bordier, J. Biol. Chem., 256: 1604-1607, 1981). This applies as well to mitochondrial, plasma, and other membrane proteins. Preliminary determinations of the selectivity by which membrane proteins were found in the aqueous versus the detergent phase extracts was established by fractionation of cells whose surface had been biotinylated by incubation of $10^7$ cells in protein free PBS containing N-hydroxysuccinimido biotin at 100 $\mu$g/ml for 30 minutes on ice (4 °C). The action was stopped by addition of 1% BSA in PBS and rinsing cells by centrifugation and resuspension to remove unreacted chemical. Cells were lysed with TRITON X-100 and run on a 10% reducing SDS-polyacrylamide gel. This gel was then subjected to Western blotting with equivalent amounts of protein from the two phases onto nitrocellulose. This was followed by detection with streptavidin-beta-galactosidase which, while not quantitative, indicated that the great majority of the biotin was associated with the detergent phase (data not shown).

2-D gel analysis was performed by Protein Databases Inc. (PDI) as previously described (Garrels, J. Biol. Chem., 254: 7961-7977, 1979; Garrels, Methods Enzymol., 100: 411-423, 1983). Briefly, a sample of 20 $\mu$l or less containing 400,000 dpm of $^{35}$S-cysteine incorporated into cell protein (5-20 $\mu$g) was loaded onto a first dimension pH 3.5-10 gradient gel using LKB ampholytes. The second dimension was performed on SDS-PAGE gels of 10% acrylamide under reducing conditions. Films were exposed to dried gels together with a set of densitometric standards for varying periods. Molecular weights and isoelectric points were established by reference to proteins with known values. Computer-assisted analysis of gels was performed as described (Garrels, J. Biol. Chem., 264: 5269-5282, 1989).

The results are shown in Figures 12A and 12B, wherein membrane proteins from $^{35}$S-cysteine labeled endothelial cells are shown in Figure 11A and IL-1 activated endothelial cells are shown in Figure 11B. For each gel 400,000 cpm were loaded.. In this figure, film was exposed to dried gels for 22 days. The apparent molecular weights and isoelectric points are indicated, based upon reference to known proteins in a database.

Figure 12A shows the pattern of proteins which incorporate $^{35}$S-cysteine in quiescent and confluent cells in a 6 hour period. On the right is shown the proteins synthesized by cells exposed to IL-1 and $^{35}$S-cysteine for 6 hours. The three arrows on the upper left side of Figure 12A refer to clusters of spots which are absent from uninduced and present in induced cells. These patterns were very reproducible in a series of 6 such analyses and essentially equivalent to results obtained using $^{35}$S-methionine as a metabolic label. In this representative experiment, computer analysis identified a total of 652 spots in the two films, 300 of which were over 30 disintegrations per minute (dpm). Of those spots in the IL-1 induced 2-D gels with over 30 dpm, 19 were at dpm values 2-fold or higher over the levels in the uninduced 2-D gel. The three clusters of spots containing most of the 19 spots were the only major induced proteins in all six experiments.

**(B) Characterization of the 1E7/2G7 Antigen:** TRITON X-114 lysates (prepared as described above) of IL-1 activated or non-activated HUVEC were fractionated to obtain the detergent pellets. A portion of the IL-1-activated membrane pellet was used to immunoprecipitate the 1E7 and 2G7 antigens.

The immunoprecipitation was carried out as follows. Nunc ELISA 96-well plates were incubated with 25 µg of affinity purified goat anti-mouse antibody in 100 µl of 0.1M carbonate buffer, pH 9.5, for 1 hour at room temperature. Wells were rinsed ten times with PBS-0.05% TWEEN 20, followed by addition of 200 µl of 1% BSA for 1 hour at room temperature. Five micrograms of purified monoclonal antibody was added to each well in the presence of BSA for an additional hour at room temperature. Following five washes with PBS-TWEEN, 1-3x$10^6$ cpm of lysate was added to each well at 4 °C for 2 hours with gentle rocking. Wells were washed with PBS-TWEEN, and the excess fluid was removed. The immunoprecipitated material was extracted with 2% SDS, 2% 2-mercaptoethanol and a portion of each of these precipitates was mixed with TRITON X-114 lysate membrane fractions for 2-D gel analyses.

Figure 13 shows the 2-D gel analysis of 1E7 and 2G7 immunoprecipitates alone and in mixtures of total membrane proteins from uninduced (-IL-1) and induced (+IL-1) HUVEC. In each of the eight squares is shown a portion of a 2-D gel which corresponds to the upper left quadrant of the total gel analyzed in Figure 12. Figures 13A and 13B are the immunoprecipitates alone with either the 1E7 (top) or 2G7 (bottom) antibody. No additional spots were seen in other portion of these gels. Figures 13C and 13D show the total membrane proteins from uninduced (-IL-1, top) or induced (IL-1, bottom) HUVEC with no added immunoprecipitate. Figures 13E and 13F show the effect of addition of the 1E7 (top) or 2G7 (bottom) immunoprecipitate to the membrane proteins of uninduced HUVEC. Figures 13G and 13H show the effect of addition of the 1E7 (top) or 2G7 (bottom) immunoprecipitates to the membrane proteins from IL-1 induced HUVEC. Pus (+) indicates the acidic and negative (-) the basic end of each gel.

From Figure 13, several conclusions can be drawn. First, the pattern of spots immunoprecipitated by the 1E7 and 2G7 antigens are indistinguishable (Figure 13A, top and bottom, respectively). Second, mixing experiments with either the 1E7 or 2G7 immunoprecipitate show that they occupy an identical position with respect to size and charge, relative to the other spots in the total membrane lysates. This can be seen most easily where the 1E7 or 2G7 immunoprecipitates are added to the uninduced (-IL-1) lysate (Figures 13E and 13F). Third, the 1E7 and 2G7 antigens are identical, and define the protein present in the induced membrane lysate with which these immunoprecipitates overlap when added to the IL-1 induced lysate (Figures 13G and 13H). These spots correspond to the middle arrow in Figure 11 consistently the most prominent set of spots in 2-D gels from cysteine (or methionine) labeled cells. From these mixing experiments as well as the 2-D gel shown in Figure 12 the conclusion, based upon the molecular mass and isoelectric point of known proteins run on 10% SDS-polyacrylamide reducing gels, is that the 1E7/2G7 antigen molecular mass centers at approximately 110 kDa with an isoelectric point range of 4.8 to 4.9.

**(C) Comparison of the 3B7/7A9 Antigen With the 1E7/2G7 Antigen:** Preliminary analysis in 2-D gels of immunoprecipitates of the 3B7 and 7A9 antigens indicated that they had identical, and in mixing experiments, overlapping profiles. The immunoprecipitation, 2-D gel electrophoresis and mixing experiments were conducted as described immediately above. Each precipitate consisted of approximately 10 spots whose molecular mass range was approximately 110-120 kDa and whose isoelectric point range was approximately 4.3 to 5.0. It could be tentatively concluded therefore that the 3B7 and 7A9 antibodies each define the same polypeptide. In order to test the relatedness of the 1E7/2G7 protein to the 3B7/7A9 and ICAM-1 proteins, and to place each of these in the context of the IL-1 inducible membrane proteins shown in Figure 11, a 2-D gel analysis, conducted as described above, of mixtures of different immunoprecipitates was performed. Equal cpm (approximately $10^4$) of immunoprecipitates from IL-1 activated and $^{35}$S-cysteine labeled HUVEC were analyzed. The antibodies used for the immunoprecipitations were: RR1 (anti ICAM-1), 2G7, 7A9, RR1 + 2G7, RR1 + 7A9 and 2G7 + 7A9.

The results are shown in Figure 14: Figure 14A, RR1 (anti-ICAM-1); Figure 14B, 2G7; Figure 14C, 7A9; Figure 14D, RR1 + 2G7; Figure 14E, RR1 + 7A9 and Figure 14F, 2G7 + 7A9. Only the upper left hand quadrant of each of the six 2-D gels is shown. No additional spots were present in other regions.

The individual immunoprecipitates for ICAM-1, 2G7, and 7A9 are shown in Figures 14A, 14B, and 14C, respectively. Figure 14D shows the distinctness of both the 2G7 and the ICAM-1 proteins and their relation to one another. Figure 14E shows a comparison of 7A9 and ICAM-1 while Figure 14F shows the 2G7 and 7A9 comparison. Only in the last case is there a partial overlap of spots with the 7A9 showing a slightly more acidic and higher molecular weight profile. The results allow identification of the three arrows in Figure 12. The left most arrow identifies the acidic end of the 3B7/7A9 protein, the middle arrow identifies the region of the 1E7/2G7 protein, and the right arrow identifies the ICAM-1 region. The corresponding induced proteins are apparent in Figure 12 (+IL-1). The distinctiveness of the 1E7/2G7 and 3B7/7A9 proteins, and their relation to the ELAM-1 structure previously identified (Bevilacqua et al, Proc. Natl. Acad. Sci., 84: 9238-9242, 1987; Bevilacqua et al, Science, 243: 1160-1165, 1989) was tested in the following experiments.

The coding sequence of the ELAM-1 cDNA cloned by polymerase chain reaction was inserted into the pCDN-1 expression vector (Figure 15) and used to transfect COS cells.

ELAM-1 cDNA cloning by polymerase chain reaction (PCR) was conducted as follows. The following PCR primers were used to amplify the ELAM-1 coding region:

Primer 1 5'-GGGGGGCTCG AGTGAAGTCA TGATTGCTTC ACAGTTTCTCTC-3'

Primer 2 5'-GGGGGGACGC GTAACTTAAA GGATGTAAGA AGGCTTTTGGTA-3'

The two primers were chosen such that they encompass the coding region of ELAM-1 mRNA, and include the start and stop codons, respectively. Also, restriction enzyme sites, Xho-I and Mlu-I, were built into these primers for ease of cloning of the PCR amplified product into the expression vector, PCR-amplified ELAM-1 cDNA was prepared by two different approaches. In method 1, polyA RNA from IL-1-activated HUVEC was used to make cDNA using an Invitrogen Copy Kit. Total RNA from HUVEC was isolated by acid phenol extraction (Chomczynski and Saccri, Anal. Biochem., 152: 156-159, 1987). PolyA RNA was isolated by oligo dT cellulose chromatography (Aviv and Leder, Proc. Nat. Acad. Sci., USA, 69: 1408-1412, 1972). The cDNA was then used to amplify the ELAM-1 coding sequence by PCR (Saiki et al, Science, 239: 487-491, 1988) using the two primers described above and a gene amplification kit obtained from Cetus Corp. PCR amplification was carried out for 30 cycles using the following conditions: denaturation: 94°C, 1 minute; annealing: 50°C, 2 minutes; elongation: 72°C, 3 minutes. During the last cycle, the elongation time was for 7 minutes. In method 2, 500 ng of polyA RNA from IL-1-activated HUVEC was reverse transcribed in a total reaction volume of 20 µl with PCR primer 1 and MuLV reverse transcriptase obtained from BRL using the conditions prescribed by the vendor. This cDNA was then used to amplify the ELAM-1 coding sequences according to the conditions of method 1. The PCR amplified product from both methods was separated by agarose gel electrophoresis. The expected 1.85 Kb product of the ELAM-1 coding sequence was obtained in both cases.

The 1.85 Kb band was electroeluted and treated with Kienow fragment of DNA Polymerase I (BRL) and ligated to the plasmid pSP72A, linearized with Nru-I and treated with calf intestine alkaline phosphatase (Boehringer Mannheim).

The plasmid pSP72A is a modified version of the plasmid pSP72 obtained from Promega Biotech. Specifically, pSP72 (Promega Biotech; Cat # P2191) was modified by replacing the multiple cloning site (MCS) with a new multiple cloning site containing the following sites: NdeI, NotI, XhoI, HindIII, NruI, ClaI, MluI, BamHI, XbaI, EcoRI, SmaI, SacII, SnaBI, SalI, EagI and BglII. The synthesis of oligonucleotide coding for the modified MCS was synthesized in a gene assembler machine using a standard protocol. The oligonucleotide was inserted in NdeI/BglII digested pSP72 to derive the vector pSP72A.

Clones containing ELAM-1 coding sequence insert were obtained by colony hybridization using a synthetic oligomer probe within the coding region of ELAM-1 cDNA. Six clones were isolated and sequenced using eight different synthetic oligomer probes spaced at regular intervals along the coding sequence of ELAM-1 cDNA. All the clones contained randomly distributed sequence variations different from the published sequence of ELAM-1 cDNA (Bevilacqua et al, Science, 243: 1160-1165, 1989) which could be due to error during PCR amplification. However, two variations at nucleotide position 1518 and 1916 of the published sequence were present in all of the clones of PCR amplified ELAM-1 coding sequence. Therefore the correct sequence is believed to be this new sequence. One of the clones, SPELAM-4 contained the same sequence as that of the published sequence except the differences at nucleotide positions 1518 and 1916. The ELAM-1 coding sequence insert was released from this clone following digestion with Not-I and Sma-I and cloned into the expression vector pCDN-1.

The resulting ELAM-1 expression plasmid is shown in Figure 15. Figure 15 shows the plasmid (pCDN-1; T.V. Gopal, unpublished work) containing SV40 early promoter (area with diagonal bars) and SV40 "t" splice and polyA addition sequences (dotted area) derived from pSV2 NEO (Southem and Berg, J. Mol. Applied Genetics, 1: 327-341, 1982). The rest of the plasmid contained pBR322 (—) sequences. Important restriction sites are identified in the figure. MCS indicates multiple cloning sites. Other commonly available mammalian expression vectors such as pEUK-C1, pEUK-C2 and pMAM-neo (Clontech Laboratories, CA) can be used in place of pCDN-1 to study transient expression of ELAM-1 in COS cells.

Mock or ELAM-1 transfected COS cells were used to test the reactivity of the 4 monoclonal antibodies. Preliminary experiments, not shown, indicated that approximately 2-5% of ELAM-1 transfected cells bound to HL-60 tumor targets, whereas the mock transfected cells displayed only occasional HL-60 binding. Mock transfected and ELAM-1 transfected cells were screened in a plate binding assay analogous to that described in Example 1.

Briefly, COS cells (2 x $10^4$/well) were plated into microtiter wells 24 hours prior to assay and 48 hours after transfection. Cells were incubated successively with medium alone (control) or 1 μg/ml each of the four purified monoclonal antibodies followed by biotinylated goat anti-mouse antibody and $^{125}$I-streptavidin.

Transfection was carried out as follows. Twenty micrograms of pCDN ELAM-1 plasmid DNA was transfected into approximately 1 x $10^6$ COS cells by the DNA-$CaPO_4$ coprecipitation method of Wigler et al, Cell, 16: 777-785, 1979. Four hours after the addition of the DNA-$CaPO_4$ precipitate the cells were treated with 15% glycerol for two minutes at room temperature according to the method of Parker and Stark, J. Virol., 31: 360-396, 1979. The same amount of the vector plasmid DNA without any insert was used as control for transfection into 1 x $10^6$ cells to obtain mock transfected cells. Cells were harvested approximately forty eight hours after glycerol shock and used for antibody binding studies. ELAM-1 transfected but not mock-transfected cells efficiently bound HL60 cells (data not shown).

The data from one of three experiments with identical results is shown in Figure 16. In this figure, the results are expressed as the mean (± s.e.m. n = 3) of the total cpm/well. This figure demonstrates that neither the 1E7 nor 2G7 antibodies are reactive with the ELAM-1 gene product, while both 3B7 and 7A9 do react. This pattern of reactivity was confirmed by immunoprecipitation analysis of these same cells after metabolic labeling. Only the 3B7 and 7A9 antigens precipitated a protein of molecular mass approximately 100 kDa from ELAM-1 transfected but not from mock transfected cells (data not shown). For simplicity, therefore, only the 2G7 and 7A9 antibodies were used in most subsequent comparisons of the two proteins.

**(D) Comparison of ICAM-1, 2G7 and 7A9 Proteins After Tunicamycin Treatment:** A further comparison of the ICAM-1, 2G7, and 7A9 proteins with respect to the degrees of N-linked carbohydrate was undertaken. Confluent HUVEC were treated for 6 hours in the presence of 1 ng/ml of IL-1 and in the absence or presence of 1 μg/ml tunicamycin B2. Tunicamycin was added at the time of addition of IL-1 together with $^{35}$S-cysteine. TRITON X-100 lysates were immunoprecipitated as described above except that the wells were extracted with reducing sample buffer in a 60°C water both for five minutes. The immunoprecipitates were analyzed on 10% SDS-polyacrylamide reducing gels. TRITON X-100 lysates were prepared as described above for the TRITON X-114 lysates, except that the phase separation procedure was omitted.

The results are shown in Figure 17. Figure 17 shows that the ICAM-1 glycoprotein is present, after tunicamycin treatment, as a mixture of native and core protein structures, the latter at 50 kDa. The 2G7 immunoprecipitate with major and minor bands at 114 kDa and 95 kDa, is present after tunicamycin treatment as a mixture of native 114 kDa and core protein at 80 kDa. The reduced expression of the 114 kDa band suggests that tunicamycin may have caused some reduction in the synthesis of this protein. The 7A9 (ELAM-1) antigen, consistent with the complex pattern seen in 2-D gels, is a broad band in the region of 110 kDa which after tunicamycin treatment is almost entirely reduced to a protein of molecular mass approximately 67 kDa.

**(E) Sialic Acid Content of 2G7 and 7A9 (ELAM-1) Antigens:** The pattern of spots shown in the 2-D gel analysis of the immunoprecipitates of the 2G7 and 7A9 (ELAM-1) antigens (Figures 12 and 13) suggest charge heterogeneity that might be due to variable degrees of sialic acid content. HUVEC were therefore activated with IL-1 for 6 hours in the presence of $^{35}$S-cysteine and detached with either PBS/EDTA (control) or by neuraminidase treatment. Viability of such cells was always greater than 95%. TRITON X-100 extracts (obtained as described above) of these cells were immunoprecipitated by the method described above with either the 2G7 or the 7A9 antibody. 2-D gel analyses (conducted as described above) of these immunoprecipitates were performed.

The neuraminidase treatment of the HUVEC was performed as follows. Cells were rinsed once with D-PBS and 0.05 U of neuraminidase in PBS was added to approximately 3 x $10^6$ cells for 20 minutes at 37°C. The detached and fully viable cells were centrifuged and lysed by the addition of 1% TRITON X-114 as described above.

Figure 18 shows a portion of each of the four 2-D gels. For both antigens there was a significant decrease in the numbers of detectable spots plus the appearance of a new spot(s) reduced in molecular weight by approximately 5 kDa and increased in charge by approximately 0.5 isoelectric point. This is evidence that both proteins contain sialic acid in varying degrees of substitution.

**(F) O-linked Carbohydrate Content of 2G7 and 7A9 (ELAM-1) Antigen:** $^{35}$S-cysteine immunoprecipitates (prepared as described above in microtiter wells) of both antigens were first treated with digestion buffer alone or with buffer containing 1 U/ml of neuraminidase (Boehringer Mannheim) for 1 hour at 37°C. Digestion buffer consisted of 0.155 M sodium phosphate, pH 6.0, containing 1 mM calcium acetate. In addition the digestion buffer contained a cocktail of protease inhibitors as described by Ronnett et al (Ronnett et al, J. Biol. Chem., 259: 4566-4575, 1984) consisting of leupeptin, benzamidine, aprotinin, antipain, pepstatin and chymostatin. After neuraminidase treatment, wells were left untreated or treated with endo-alpha-N-acetylgalactosaminidase (O-glycanase) at 25 mU/ml in the same buffer as used for neuraminidase treatment. O-glycanase treatment proceeded at 37°C overnight. The next day wells were rinsed twice with PBS-TWEEN, extracted as described above with sample buffer and the extracts were analyzed on 10% SDS-polyacrylamide reducing gels.

The results are shown in Figure 19. As shown in Figure 19, there was a reduction in mass of approximately 5 kDa for both the 2G7 and 7A9 (ELAM-1) antigens after neuraminidase treatment, consistent with the results obtained by treatment of intact cells (Figure 18). Treatment to remove O-linked sugars caused only a slight reduction in mass, approximately 1-2 kDa for both the 2G7 and 7A9 antigens in two experiments.

**(G) Kinetics of Antigen Synthesis:** In order to further show the similarities and differences between the 2G7 and 7A9 glycoproteins, the glycoproteins were immunoprecipitated as described above at different times alter the addition of IL-1 to the HUVEC cultures. $^{35}$S-cysteine was added at the time of IL-1 addition. TRITON X-100 lysates were made and immunoprecipitates were formed with either the 2G7 or the 7A9 antibody. Immunoprecipitates were analyzed on 10% SDS-polyacrylamide reducing gels.

The results, shown in Figure 20, indicate that at 1 hour only the 7A9 (ELAM-1) antigen can be detected, though weakly. It has presumably already undergone some degree of glycosylation since its mass at this time is approximately 79 kDa, not the 67 kDa seen alter tunicamycin treatment. By 2 hours the 2G7 immunoprecipitate shows two bands of equal intensity at approximately 95 kDa and 114 kDa while at this time the 7A9 (ELAM-1) antigen is at its "mature" form of a broad band at around 95 kDa. By 6 hours the 2G7 antigen shows the major band at 114 kDa.

**(H) Competition Binding Assays:** In order to test the spatial relatedness of the four epitopes defined on these two proteins by the different monoclonal antibodies, a competition binding assay was performed wherein each of the purified monoclonal antibodies was coupled with FITC and tested in flow cytometry for their ability to compete in homologous and heterologous combinations.

FITC conjugates of each monoclonal antibody, 1E7, 2G7, 7A9 and 3B7, were tittered on IL-1-activated endothelial cells to determine a slightly subsaturating dose. This dose of FITC antibody was premixed with a 50-fold excess of each of the four unconjugated monoclonal antibodies. This mixture was then added to a single cell suspension of IL-1-activated HUVEC at 4°C for 30 minutes in PBS containing 1% BSA and 0.02% sodium azide. Cells were washed three times in the same buffer and resuspended for flow cytometric analysis.

FITC conjugation of monoclonal antibodies at 4 mg/ml in 0.1M carbonate buffer at pH 9.5 was performed as described (Hardy, in Handbook of Experimental Immunology, Vol. 1, 4th Edition, 1986 - (Weir, D.M. ed.) Blackwell Scientific Publications, Oxford, England, 3101-3112).

The results are shown in Figure 21. The particular FITC-antibody used for each of the four experiments shown was as follows: Figure 21A : 1E7; Figure 21B : 2G7; Figure 21C : 3B7; Figure 21D : 7A9. On the vertical axis is the percent positive cells.

The results indicate that at a 50-fold excess 1E7, 2G7, and 3B7 compete only for the binding of the homologous FITC-monoclonal antibody. The 7A9 antibody is different in that it reproducibly interferes with the binding of the 2G7 antibody, a maximum of 62% at a 1:50 molar ratio 2G7:7A9.

**(I) Kinetics of Antigen Expression at the Cell Surface:** HUVEC were cultured for different periods of time in 1 ng/ml of IL-1, and analyzed for the 2G7 and 7A9 antigens using the 96-well plate binding assay essentially as performed for the hybridoma screening assay. All cells were analyzed at the same time.

The results are shown in Figure 22, wherein closed circles represent antibody 2G7 and closed triangles represent antibody 7A9. The results are expressed as the mean c.p.m. ± s.e.m. (n = 3).

The results shown in Figure 22 demonstrate that by 2 hours the 7A9 (ELAM-1) protein is maximally expressed, while the 2G7 antigen is only half maximally expressed. By 4 hours the 2G7 antigen has reached the maximum level. This result is consistent with the somewhat earlier expression of the 7A9 (ELAM-1) protein in biosynthetic labeling experiments (Figure 20). Interestingly, in the continued presence of IL-1, both proteins are expressed at the cell surface at high levels for up to seven days. If IL-1 is withdrawn, they return to baseline levels within 24 hours (data not shown).

**(J) Cellular Distribution of the 2G7 and 7A9 Antigens:** A variety of cells in primary culture were tested, with or without IL-1 pretreatment, for expression of the 2G7 or 7A9 glycoproteins. These experiments were performed in 96-well plates essentially as was done in the hybridoma screening assay. Specifically, the indicated cell types were placed in 96-well plates. The following cells were seeded at the indicated concentration 24 hours prior to assay: fibroblasts, keratinocytes and HUVEC, 2 x $10^4$/well; on the day of assay peripheral blood monoclonal cells were seeded at 5 x $10^4$/well, granulocytes at $10^5$/well and platelets at $10^5$/well. Platelets were stimulated with 1 U of human alpha-thrombin for 1 hour prior to assay of antigen expression.

The results are shown in Table IV.

TABLE IV

| Cellular Distribution of the 2G7 and 7A9 (ELAM-1) Proteins | | | |
|---|---|---|---|
| **Cell Type** | **Antibody** | **cpm bound, no IL-1** | **cpm bound, IL-1** |
| HUVEC | 2G7<br>7A9 | 1650 ± 62<br>1971 ± 281 | 47161 ± 1367<br>59309 ± 640 |
| Granulocytes | 2G7<br>7A9 | 1672 ± 351<br>1383 ± 295 | 1298 ± 157<br>1215 ± 166 |
| Peripheral blood mononuclear cells | 2G7<br>7A9 | 2047 ± 295<br>2210 ± 346 | 1511 ± 69<br>1575 ± 37 |
| Keratinocytes | 2G7<br>7A9 | 849 ± 45<br>832 ± 42 | 813 ± 29<br>800 ± 29 |
| Fibroblasts | 2G7<br>7A9 | 521 ± 35<br>448 ± 17 | 511 ± 16<br>513 ± 22 |
| Platelets | Medium Only<br>2G7<br>7A9<br>10E5 | 5688 ± 228<br>5141 ± 155<br>6241 ± 36<br>17267 ± 76 | 5736*<br>5377*<br>6843*<br>17455* |

*Thrombin stimulation, 1 U/ml

The results in Table IV indicate that these antigens are not expressed at a detectable level on fibroblasts, granulocytes, unfractionated peripheral blood mononuclear cells, keratinocytes or platelets (with or without thrombin stimulation). Platelets appeared to have a high level of non-specific binding of $^{125}$I-streptavidin.

**(K) Effects of TNF, LPS and IFNγ on 1E7/2G7 and 7A9/3B7 Antigen Expression:** The effect of lps stimulation for 4 hours, TNF stimulation for 6 hours, and long term IFNγ stimulation on the expression of the 1E7/2G7 antigen and the 7A9/3B7 antigen was studied. Specifically, HUVEC were expressed to various inducing agents as follows: gamma-IFN, 100 µ/ml, 2.5 days; lps 1 µg/ml for 6 hours, TNF 1 µ/ml for 4 hours; and IL-1 1 ng/ml for 4 hours. After washes to remove these agents, the indicated antibodies were tested at saturating doses for antigen expression in the plate-binding assay.

The results are shown in Table V and are expressed as mean ± s.e.m. (n = 3).

Table V

| Effect of Various Agents on 1E7/2G7 and 7A9/3B7 Antigen Expression | | | | |
|---|---|---|---|---|
| **Antibody** | **IFN-Gamma** | **lps** | **TNF** | **IL-1** |
| Control | 181 ± 19 | 244 ± 20 | 644 ± 42 | 253 ± 3 |
| 1E7 | 818 ± 44 | 3217 ± 207 | 12,726 ± 350 | 9,069 ± 457 |
| 2G7 | 763 ± 5 | 4684 ± 292 | 13,335 ± 232 | 10,483 ± 1115 |
| 3B7 | nd* | nd | 18,319 ± 450 | 21,595 ± 2297 |
| 7A9 | nd | nd | 20,067 ± 402 | 26,583 ± 898 |
| HLA-DR | 5140 ± 374 | 178 ± 12 | nd | 226 ± 4 |

*nd = not done

As has been shown previously for ELAM-1, lps and TNF stimulation result in the expression of the 1E7/2G7 protein. IFNγ, however, after a 2.5 day incubation was ineffective at induction of 1E7/2G7, though HLA-DR was expressed as expected.

**(L) Surface Expression of the 3A2 Antigen:** A flow cytometric analysis was conducted of endothelial cells pretreated for 4 hours with 1 ng/ml IL-1 and stained with 50 µg/ml 3A2 antibody. The cell number was determined in arbitrary units from the relative fluorescent intensities. For comparison the expression of the HLA and ICAM-1 antigens was also determined using the W6/32 anti-HLA antigen and the anti-ICAM-1 antibody RR/1. The flow cytometry was conducted as described above. The staining was conducted by conventional methods using FITC labeled antibodies.

The results are shown in Figure 23. Figure 23 shows quiescent (...) or IL-1 stimulated (-) endothelial cells stained with the indirect reagent only (Figure 23A), the W6/32 anti-HLA antigen (Figure 23B), the anti-ICAM-1 antibody RR/1 (Figure 23C) and 3A2 antibody (Figure 23D).

The antigen defined by the 3A2 antibody is not detectable on the surface of quiescent endothelial cells, but after exposure to IL-1 is detectable within 2 hours. The results show that unlike either the HLA or ICAM-1 protein, the 3A2 antibody detects a heterogeneous distribution of antigen ranging from channel 50 to channel 150, an approximately 8 fold range of fluorescent intensity. The most intensely positive cells are equivalent in fluorescence intensity to the HLA antigen, though less than ICAM-1. As previously reported (Dustin and Springer, J. Cell Biol., 107: 321-331, 1988), ICAM-1 shows an approximately 8-fold increase on endothelial cells after 4 hours of IL-1 exposure.

**(M) Tissue Expression of 3A2 Antigen:** Granulocytes, peripheral blood mononuclear cells (PBL), keratinocytes, and fibroblasts were tested for membrane expression of the 3A2 antigen before or after exposure for 4 hours to 1 ng/ml of IL-1. The methods used were those described above for the 1E7/2G7 and 7A9/3B7 antigens except that cells were stimulated with IL-1 instead of thrombin.

The results are shown in Table VI.

Table VI

| Cell Surface Distribution of the CMP-170 Protein | | |
|---|---|---|
| **Cell Type** | **cpm bound, no IL-1** | **cpm bound, IL-1** |
| HUVEC | 943 ± 28 | 44,381 ± 1875 |
| Granulocyte | 1,607 ± 70 | 1,429 ± 157 |
| PBL | 2,459 ± 306 | 2,067 ± 142 |
| Keratinocytes | 1,651 ± 191 | 1,570 ± 106 |
| Fibroblasts | 608 ± 40 | 638 ± 53 |

Table VI shows that only endothelial cells showed inducibility of the expression of the 3A2 antigen.

**(N) 3A2 Antigen Characterization:** Early passage endothelial cells were cultured in the presence or absence of 1 ng/ml IL-1 and 250 µCi $^{35}$S-cysteine for 4 hours and $^{35}$S-cysteine labeled cells were lysed with 1% TRITON X-100 and immunoprecipitated as described above with the antibodies 3A2, 7A9 (anti-ELAM-1) or 2G7 directed to the endothelial T-cell adhesion structure. The immunoprecipitates were run on 8% SDS-polyacrylamide gels under reducing conditions.

The results are shown in Figure 24 wherein (-) indicates untreated and (+) indicates IL-1 treated cells. The results indicate that cells not treated with IL-1 show no ELAM-1 antigen, but that IL-1 treated cells precipitate a 110-120 kDa molecule reactive with the 7A9 antibody, as expected, and a 114 kDa protein reactive with the 2G7 antibody as shown above. The 3A2 antibody precipitates a 170 kDa protein in the absence and presence of IL-1. in addition the 3A2 antibody precipitates a faint band in the region of the 2G7 and 7A9 proteins only from the IL-1 treated cells. This result suggests that the 3A2 antigen complex might be composed of two proteins, or subunits, one of which is constitutive and the other inducible. While expression of the 3A2 antigen by IL-1 at the cell surface is inducible (Figure 23), expression of the antigen(s) in the cytoplasm might be constitutive.

**(O) Expression of the 3A2, 2G7 and 7A9 (ELAM-1) Antigens at the Plasma Membrane Requires RNA and Protein Synthesis:** Endothelial cells in monolayer culture in 96-well flat bottom plates were pretreated for 30 minutes with 1 μg/ml of cycloheximide or for 15 minutes with 1 μg/ml of Actinomycin D prior to the addition of IL-1. Drugs were left in cultures during the peril of IL-1 activation. After 4 hours the cultures were rinsed and incubated with the relevant monoclonal antibodies in a plate binding assay as described above. Viable cells in monolayer cultures were examined for surface expression of these three proteins.

The results are shown in Figures 25; Figure 25A: IL-1 ± cyoloheximide; Figure 25B: IL-1 ± actinomycin D (act D).

Figure 25 shows that all antigens tested exhibited dependence of expression on RNA and protein synthesis.

**(P) Light Microscopic Evaluation of 3A2 Antigen:** Quiescent and IL-1 stimulated endothelial cells were examined for cytoplasmic expression of the 3A2 antigen. HUVEC were cultured for 4 hours in the absence or presence of l ng/ml IL-1. The cultured HUVEC were detached by brief exposure to PBS-EDTA and cytospun onto glass microscope slides. Cells were fixed with. acetone for 10 minutes at room temperature and air dried to permeabilize the cells and stained with the 3A2 and 7A9 antibodies.

For staining, 100 μl of antibody containing 0.1 μg of antibody was placed on the fixed cells for 30 minutes at room temperature. The cells were rinsed with PBS-1% BSA, and incubated with 5 μg/ml goat anti-mouse antibody conjugated with alkaline phosphatase for 30 minutes. After rinsing with PBS-1% BSA, the slide was incubated with chronogen AEC to develop color and counter stained with hematoxylin. Slides were viewed after mounting with a coverslip.

The results are shown in Figure 26. Figure 26A: no IL-1, stained with antibody 7A9; Figure 26B: plus IL-1, stained with antibody 7A9; Figure 26C: no IL-1, stained with antibody 3A2; Figure. 26D: plus IL-1, stained with antibody 3A2. The larger patches in Figures 26B, 26C and 26D are due to red color from staining which indicates the presence of corresponding antigen. The small dark spots in Figure 26A are nuclei stained blue, but are not surrounded by any red cytoplasmic staining.

As shown in Figure 26, staining with 3A2 was seen in both untreated and IL-1 treated cells, while staining with the 7A9 (anti-ELAM-1) was seen only in the IL-1 treated HUVEC. Note the difference in the intensity and distribution of the 3A2 antigen in IL-1 treated as compared to untreated cells. The staining in IL-1 treated cells is more punctate and in some cases can be seen to be associated with the membrane. In additional experiments, antibody to von Willebrand Factor was employed in a double staining protocol (using a Zymed double staining kit for two mouse primary antibodies according to the manufacturer's instructions) with the 3A2 antibody on non-IL-1 treated endothelial cells; these antigens do not colocalize to the Weibel Palade bodies. Hence as suggested by the immunoprecipitation experiments in Figure 24, the 3A2 antigen must preexist within the cell as the 170 kDa polypeptide. For this reason this protein has been designated as cytoplasmic-membrane protein 170 (CMP-170).

**(O) Nature of the CMP-170 Coprecipitated Structure:** The purpose of this example is to identify the 110 - 120 kDa material that associates with CMP-170 in IL-1 treated endothelial cells. Preliminary studies with a battery of detergents such as TRITON X-100, CHAPSO, OCTYLCLUCOSIDE, TRITON X-114, CHAPS and Deoxycholate, showed that solubilization of the 3A2 antigen from IL-1 treated cells was routine but was only sometimes accompanied by coprecipitation of the smaller molecular weight band. The coprecipitation was found to be optimal and reproducible in the presence of the detergent TRITON X-114. Accordingly, untreated or IL-1 treated endothelial cells were lysed with 1% TRITON X-114 detergent and immunoprecipitated as described above with the 3A2 antibody or with control goat anti-mouse IgM reagent only. A portion of these immunoprecipitates was run on 8% SDS-polyacrylamide gels under nonreducing or reducing conditions. Additional wells containing a 3A2 immunoprecipitate was reextracted with TRITON X-100 to dissociate the 3A2 antigen complex and transferred to wells with either 7A9 or 2G7 antibody for a second immunoprecipitation. The samples were run on 8% SDS-polyacrylamide gels under reducing conditions.

The results are shown in Figure 27 wherein (-) indicates untreated and (+) indicates treated endothelial cell. Figure 27A: gel run under nonreducing conditions; Figure 27B: gel run under reducing conditions; Figure 27C, first and second lanes: immunoprecipitates with 7A9 or 2G7 antibodies from IL-1 activated cells (as controls); and Figure 27C, third and fourth lanes: second immunoprecipitation with 7A9 or 2G7 antibody.

The results shown in Figures 27A and 27B indicate first that the 3A2 antigen molecular weight is 170 kDa under non-reducing and reducing conditions. The sharpening of the 3A2 antigen under reducing conditions suggests that some multimerization of the antigen may occur. The presence of iodacetamide in the lysis and sample buffers makes it unlikely that this effect is artifactual. Second, the results in Figures 2A and 2B indicate that the coprecipitated structure, at molecular mass of 110-120 kDa is not covalently associated with CMP-170 since it appears under non-reducing as well as reducing conditions.

Both the cytokine inducibility and the similarity in molecular weight of the 7A9 (ELAM-1) and 2G7 antigens (in the range of 110-120 kDa) made them candidates for the coprecipitated structure. The identity of the additional band was determined by extracting the coprecipitated material with TRITON X-100, which does not favor their association, and immunoprecipitating with either the 2G7 or 7A9 antibody. The results shown in Figure 27C indicate that the 7A9 (ELAM-1) protein but not the 2G7 protein is coprecipitated. Immunoprecipitates of the 7A9 and 2G7 antigens are shown in the first two lanes of panel C for comparison.

Another approach was also taken to confirm the CMP-170: ELAM-1 association. This was possible because preliminary experiments had shown that CMP-170 was also present in the cytoplasm of untreated COS cells. This allowed testing as to whether ELAM-1 transfected COS cells, metabolically labeled and lysed with the TRITON X-114 detergent would reveal an association between CMP-170 and ELAM-1.

Specifically, COS cells (a monkey kidney cell line which has endogenous CMP-170) were subjected to mock transfection or were transfected by conventional methods with the ELAM-1 cDNA in the CDN plasmid. 43 Hours after transfection, $3 \times 10^6$ cells were metabolically labeled for 6 hours with 250 $\mu$Ci $^{35}$S-cysteine, lysed with 1% TRITON X-114 as described above, and immunoprecipitated as described above with antibodies 7A9 or 3A2. The immunoprecipitates were run on 10% SDS-polyacrylamide reducing gels.

The results are shown in Figure 28 where (-) indicates mock transfection and (+) indicates transfection with the ELAM-1 cDNA.

The results shown in Figure 28 indicate, as expected, that the ELAM-1 protein is precipitated from ELAM-1-transfected but not from mock-transfected COS cells, in accordance with previous experiments. (Bevilacqua et al, Science, 243: 1160-1165, 1989) In lane 5, the 3A2 antibody immunoprecipitated a 170 kDa structure not seen in lane 3 (control lane). Interestingly, there is a prominent additional band at approximately 100 kDa not seen in the control lane. In the last lane to the right (lane 6) the 3A2 antibody, in addition to the 170 kDa structure, clearly coprecipitated the ELAM-1 antigen. Whether the endogenous 3A2 associated COS protein seen at 100 kDa in lane 5 was displaced by ELAM-1 in transfected cells could not be determined due to similarity in their molecular weights. As can be seen from lane 1, the 7A9 antibody did not precipitate a protein from mock-transfected COS cells. Also, the immunoprecipitation data from COS cells (lane 2 in Figure 28) and endothelial cells (lane 1 from Figure 27C) with 7A9 did not show a band coprecipitated at 170 kDa. Hence the coprecipitation of ELAM-1 with CMP-170 is unidirectional.

**(R) Kinetics of 3A2:ELAM-1 Association:** $3 \times 10^6$ Endothelial cells were pulsed with 250 $\mu$Ci $^{35}$S-cysteine for either 45 or 120 minutes and either lysed immediately or chased for an additional 1 or 2 hours prior to lysis with TRITON X-114 as described above. Lysates were immunoprecipitated as described above with the 3A2 or 7A9 antibodies and the immunoprecipitates were run on 10% SDS-polyacrylamide reducing gels. Further, endothelial cells which had been exposed to IL-1 for 4 hours were detached with PBS-EDTA and labeled with $^{125}$I by the lactoperoxidase procedure described below. Cells were lysed with the TRITON X-114 detergent and immunoprecipitates made with the 3A2 or 7A9 antibody. The immunoprecipitates were run on 10% SDS-polyacrylamide reducing gels.

For cell surface iodination, five million endothelial cells were detached by treatment with PBS-EDTA, washed twice and resuspended in 200 $\mu$l of PBS at 4°C. To this tube was added 0.5 mCi of $^{125}$I, 50 $\mu$l of lactoperoxidase (0.2 mg/ml), and successive 20 $\mu$l additions of 30% $H_2O_2$ at one minute intervals at diluations of 1/27,000, 1/9,000, 1/3,000 and 1/1,000 as described by Hardy (R.R. Hardy, in Handbook of Experimental Immunology, Vol. 1, p 20.8, D.M. Weir, editor, Blackwell Scientific Publications, Oxford, England). Cells were washed three times and lysed with 1% TRITON X-114 detergent containing 0.15 M NaCl, 10 mM Tris pH 7.4, 2 mM PMSF and 1 mM iodoacetamide.

The results are shown in Figure 29. Figure 29A shows results for $^{35}$C-cysteine pulse labeled cells lysed after 45 minutes (first two lanes) or chased for an additional 1 or 2 hours, lanes 2 and 3 and lanes 4 and 5, respectively. Figure 29B shows the results of cell surface iodination.

The results shown in Figure 29A indicate that 45 minutes after the addition of IL-1, there is nascent 7A9 (ELAM-1) protein which can be immunoprecipitated. Likewise, the same 79 kDa molecular mass protein is coprecipitated with CMP-170. When cells are pulsed for 2 hours and chased for either 1 or 2 additional hours, the 3A2 antibody continues to coprecipitate the same molecular mass material precipitated by the 7A9 (ELAM-1) antibody. Hence the CMP-170/ELAM-1 association is detectable from the earliest points of ELAM-1 synthesis until it reaches its mature size. The results shown in Figure 29B indicate that the 3A2 antibody also coprecipitates the 7A9 (ELAM-1) protein from the cell surface, although as can be seen, the 3A2 antigen does not itself appear to label very well.

**(S) Kinetics of Cell Surface Expression of CMP-170 and 7A9 (ELAM-1) Antigens:** Endothelial cells grown to confluence in flat bottom microtiter plates were exposed to 1 ng/ml IL-1 for periods of time of from 0 to 48 hours and then tested in a plate binding assay analogous to that described above for hybridoma production for expression of the 7A9 and 3A2 antigens.

The results are shown in Figure 30. In Figure 30, the open circles represent the 7A9 antigen and the open triangles represent the 3A2 antigen. As can be seen from the results in Figure 30, the expression of both proteins occurs simultaneously, and peaks around 2 to 4 hours. However, the 7A9 (ELAM-1) antigen is maintained at high levels as long as IL-1 is present, while the 3A2 antigen declines to basal levels by 48 hours. Additional experiments (not set forth), indicate that CMP-170, like ELAM-1, is induced by exposure of endothelial cells to TNF and lipopolysaccharide but not gamma interferon.

**(T) Determination of Epitope of ELAM-1 to Which Monoclonal Antibodies 7A9 and 3B7 Bind and Preparation of Soluble Forms of ELAM-1:** Figure 31 represents various truncated forms of ELAM-1 which were prepared in order to determine the epitope to which monoclonal antibodies 7A9 and 3B7 bind. The truncated versions of ELAM-1 were constructed by PCR as described in Section (C) of this Example using PCR primers that contained a stop codon, e.g., either TAA or TAG, at the 3' end of the deleted versions of ELAM-1 cDNA. Construction of such primers is conventional in the art. The COOH ends were chosen such that the truncated versions of the ELAM-1 molecule would contain lectin (L-ELAM-1), lectin plus EGF (LE-ELAM-1), lectin plus EGF plus part of the complement regulatory domains (LEC-ELAM-1), and lectin + EGF + entire complement regulatory domains without the transmembrane and the cytoplasmic tail of the ELAM-1 molecule (ELAM-1 $Tm^-$). L-ELAM-1, LE-ELAM-1, LEC-ELAM-1, and the mature form of ELAM-1 were cloned into an expression vector that contained an RSV promoter driving the exogenously introduced gene (truncated versions of ELAM-1) and another dominant selectable marker, SV2 DHFR. Such a vector can be constructed by conventional methods using plasmids available from the ATCC (pRSVneo (ATCC No. 37198) and pSV2 DHFR (ATCC No. 67110)). The transmembrane deleted version of ELAM-1, ELAM-1 $Tm^-$, was cloned into the pCDN-1 vector. The plasmids were introduced into COS cells by the DNA-$CaPO_4$ co-precipitation method,described in Section (C) of this Example. 48 hours after DNA transfection, portions of the transfected cells were used for immunoprecipitation (as described in Section (C) of this Example) with ELAM-1 specific antibodies, 7A9 and 3B7, as well as the antibody 3A2. Both the transfected cell supernatants and the cell lysates were used for immunoprecipitation after metabolically labelling the cells with $^{35}$S-cysteine.

The results of the immunoprecipitation studies from the supernatant of these cells are shown in Figure 32. The results show that a fragment (LE-ELAM-1) as small as the N-terminal 30.7% of the molecule defined by the lectin and EGF-like domains, reacts with both the 7A9 and 3B7 antibodies.

**(U) Assay for Detecting Blocking of Binding of White Blood Cells to Activated Endothelial Cells:** Endothelial cells are obtained from discarded umbilical cords and cultured as described in Example 1. Cells multiply rapidly and are subdivided (passaged) up to 6 times into other flasks before their differentiated properties decline. By the second passage, sufficient cells are available to perform blocking assays. Recombinant human IL-1 beta is added at a concentration of 1 ng/ml to cells in culture for 4 to 6 hours while the cells are maintained as usual in a humidified incubation chamber containing 6% $CO_2$, 94% air. Controls consist of culturing endothelial cells in the absence of IL-1. After the incubation period, cells are rinsed with medium to remove unused IL-1.

Mononuclear cells, e.g., monocytes, granulocytes or a mixture of mononuclear cells comprising T-cells, B-cells and NK cells, are obtained from humans and isolated and purified by methods known in the art.

The mononuclear cells are preincubated in the presence of antigen 1E7/2G7 or antigenic fragments of ELAM-1 for 30 minutes at 37°C. The antigen is added in an amount sufficient to saturate the ligand binding sites on the mononuclear cells.

The mononuclear cells, in the continued presence of antigen, are then added to the endothelial cells for 30 to 60 minutes to allow attachment. The non-adherent cells are washed off by rinsing with growth medium, and the difference in the number of cells bound to endothelial cells (EC) untreated or treated with IL-1 is observed.

This assay is performed in a quantitative manner by incorporating a radioactive tracer, e.g., $^{51}Cr$, by methods known in the art, into the cytoplasm of the cells whose binding was being studied, washing to remove any extracellular counts, and placing these labelled cells over the EC monolayer. The $^{51}Cr$ label used for this purpose should show minimal leakage during the 30 to 60 minutes of the binding assay. After rinsing to remove non-adherent cells, the monolayers are solubilized with detergent. The radioactivity counted from each well is a measure of the number of cells bound.

## EXAMPLE 5

### cDNA SEQUENCE QF THE 1E7/2G7 ANTIGEN

Recently an adhesion molecule called VCAM-1 has been described (Osborn et al, Cell, 59: 1203-1211, 1989). Before attempting to clone the 1E7/2G7 antigen by cDNA expression cloning, VCAM-1 cDNA was cloned in order to determine whether the 1E7/2G7 antigen is related to VCAM-1 or not VCAM-1 cDNA was cloned using poly $A^+$ RNA from IL-1 activated HUVEC by PCR, as described in Example 4(C). The following PCR primers were chosen to only amplify the published coding sequences of VCAM-1 cDNA corresponding to a size of approximately 2.0 kb:

primer 1 5'GGGGGGCGGC CGCGCAACTT AAAATGCCTG GGAAGATG3'

primer 2 5'GGGGGCTCGA GCATTAGCTA CACTTTTGAT TTCTGTGA3'

Instead of obtaining a coding sequence of approximately 2.0 kb, a 2.3 kb band was obtained as the major product of PCR amplification. The 2.3 kb band was cloned into the expression vector pCDN-1, as described in Example 4(C), and several clones were isolated. Five clones were tested for 1E7/2G7 antibody binding in a transient expression assay using COS cells, as described in Example 4(B-2). All five clones showed strong binding to monoclonal antibodies 1E7 and 2G7.

Restriction analysis, performed using conventional methods, of these clones showed a pattern that was different from the expected bands if the PCR amplified clones contained the same sequence as that of the published VCAM-1 sequence. These studies also suggested an insert of nearly 282 bp roughly in the middle of the published gene. One clone was sequenced according to the method of Sanger (Sanger et al, Proc. Natl. Acad. Sci. USA, 74: 5463-5467, 1977) and this showed an insert of 94 amino acids at the amino acid position 308 in the VCAM-1 molecule (Figure 33). Thus the 1E7/2G7 antigen is different from the published VCAM-1 molecule. The cDNA sequence, along with the corresponding amino acid sequence of the molecule coding for the 1E7/2G7 antigen is shown in Figures 34A to 34D.

While the invention has been described in detail and with reference to specific examples thereof, it will be apparent to one skilled in the art that various changes and modifications can be made therein without departing from the spirit and scope thereof.

## STATEMENT QF DEPQSIT

Hybridomas 1E7, 2G7, 3A2 and 7A9 were deposited with the American Type Culture Collection, 12301 Parklawn Drive, Rockville, Maryland 20852, under the terms of the Budapest Treaty on the International Recognition of the Deposit of Microorganisms for the Purposes of Patent Procedure on May 9, 1989 and have ATCC deposit nos. HB 10136, HB 10137, HB 10138 and HB 10135, respectively. Hybridoma 3B7 was deposited with the American Type Culture Collection on March 21, 1990, and has ATCC deposit number HB-10391. All restrictions to access will be irrevocably removed upon grant of a United States patent on the instant application.

## Claims

1. Monoclonal antibody 1E7 or a binding site-containing fragment thereof, wherein said monoclonal antibody is produced by hybridoma cell line 1E7 having ATCC Deposit No. HB 10136.

2. Monoclonal antibody 2G7 or a binding site-containing fragment thereof, wherein said monoclonal antibody is produced by hybridoma cell line 2G7 having ATCC Deposit No. HB 10137.

3. Hybridoma cell line 1E7 having ATCC Deposit No. HB 10136.

4. Hybridoma cell line 2G7 having ATCC Deposit No. HB 10137.

5. A medicament for treating inflammatory responses associated with activated endothelial cells, the medicament comprising:
   (I) a pharmaceutically effective amount of a monoclonal antibody or a binding site-containing fragment thereof, wherein said monoclonal antibody is monoclonal antibody 2G7 produced by hybridoma cell line 2G7 having ATCC Deposit No. HB 10137; and
   (II) a pharmaceutically acceptable carrier, diluent or excipient.

6. The use of a pharmaceutically effective amount of a monoclonal antibody or a binding site-containing fragment thereof, wherein said monoclonal antibody is monoclonal antibody 2G7 produced by hybridoma cell line 2G7 having ATCC Deposit No. HB 10137 for the preparation of a medicament suitable for the treatment of inflammatory responses associated with activated endothelial cells.

**Patentansprüche**

1. Monoklonaler Antikörper 1E7 oder ein eine Bindungsstelle enthaltendes Fragment davon, worin der monoklonale Antikörper von der Hybridom-Zellinie 1E7 mit der ATCC-Hinterlegungsnummer HB 10136 produziert wird.

2. Monoklonaler Antikörper 2G7 oder ein eine Bindungsstelle enthaltendes Fragment davon, worin der monoklonale Antikörper von der Hybridom-Zellinie 2G7 mit der ATCC-Hinterlegungsnummer HB 10137 produziert wird.

3. Hybridom-Zellinie 1E7 mit der ATCC-Hinterlegungsnummer HB 10136.

4. Hybridom-Zellinie 2G7 mit der ATCC-Hinterlegungsnummer HB 10137.

5. Medikament zur Behandlung von Entzündungsreaktionen einhergehend mit aktivierten Endothelzellen, wobei das Medikament umfaßt:
   (I) eine pharmazeutisch wirksame Menge eines monoklonalen Antikörpers oder eines eine Bindungsstelle enthaltenden Fragmentes davon, worin der monoklonale Antikörper der monoklonale Antikörper 2G7, produziert von Hybridom-Zellinie 2G7 mit der ATCC-Hinterlegungsnummer HB 10137, ist; und
   (II) einen pharmazeutisch annehmbaren Träger, Verdünner oder Exzipienten.

6. Die Verwendung einer pharmazeutisch wirksamen Menge eines monoklonalen Antikörpers oder eines eine Bindungsstelle enthaltenden Fragmentes davon, worin der monoklonale Antikörper der monoklonale Antikörper 2G7, hergestellt von Hybridom-Zellinie 2G7 mit der ATCC-Hinterlegungsnummer HB 10137, ist, zur Herstellung eines Medikaments, das für die Behandlung von Entzündungsreaktionen, einhergehend mit aktivierten Endothelzellen, geeignet ist.

**Revendications**

1. Anticorps monoclonal 1E7 ou fragment de celui-ci contenant un site de fixation, dans lequel ledit anticorps monoclonal est produit pair la lignée cellulaire d'hybridome 1E7 dont le numéro de Dépôt ATCC est HB 10136.

2. Anticorps monoclonal 2G7 ou fragment de celui-ci contenant un site de fixation, dans lequel ledit anticorps monoclonal est produit par la lignée cellulaire d'hybridome 2G7 dont le numéro de Dépôt ATCC est HB 10137.

3. Lignée cellulaire d'hybridome 1E7 dont le numéro de dépôt ATCC est HB 10136.

4. Lignée cellulaire d'hybridome 2G7 dont le numéro de dépôt ATCC est HB 10137.

**5.** Médicament destiné au traitement des réponses inflammatoires associées à des cellules endothéliales activées, le médicament comprenant :

(I) une quantité pharmaceutiquement efficace d'un anticorps monoclonal ou d'un fragment de celui-ci contenant un site de fixation, dans lequel ledit anticorps monoclonal est l'anticorps monoclonal 2G7 produit par la lignée cellulaire d'hybridome 2G7 dont le numéro de Dépôt ATCC est HB 10137, et

(II) un porteur, un diluant ou un excipient pharmaceutiquement acceptable.

**6.** Utilisation d'une quantité pharmaceutiquement efficace d'un anticorps monoclonal ou d'un fragment de celui-ci contenant un site de fixation, dans lequel ledit anticorps monoclonal est l'anticorps monoclonal 2G7 produit par la lignée cellulaire d'hybridome 2G7 dont le numéro de Dépôt ATCC est HB 10137 pour la préparation d'un médicament approprié au traitement des réponses inflammatoires associées à des cellules endothéliales activées.

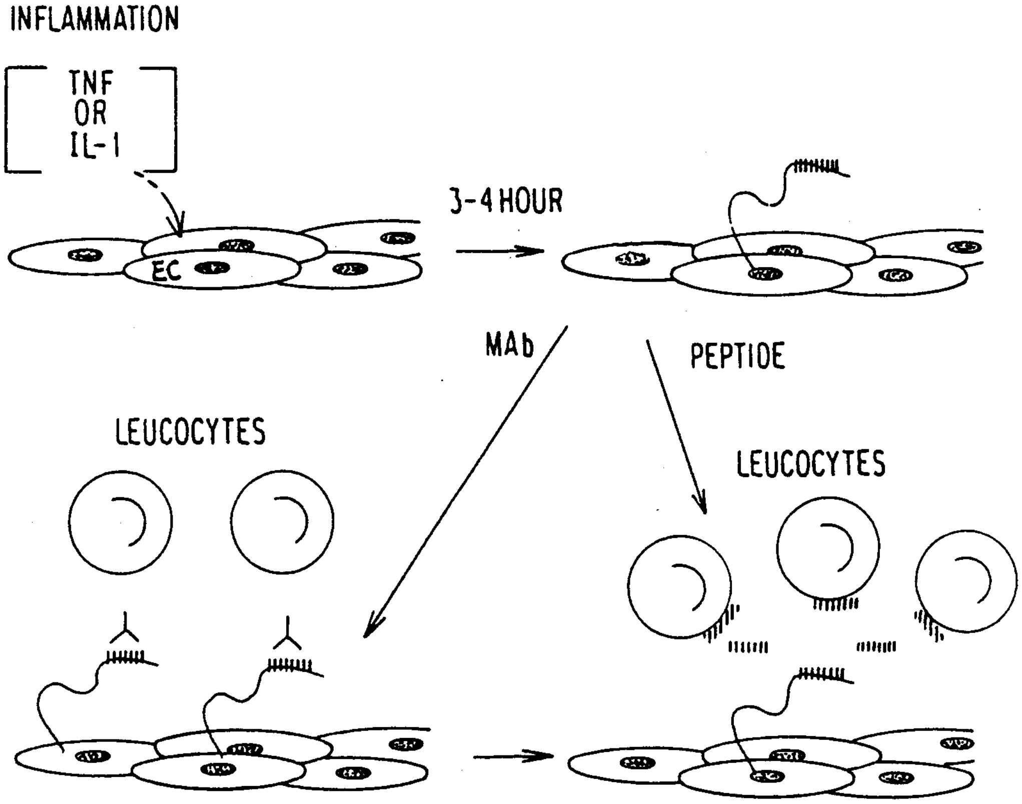
INFLAMMATION
TNF
OR
IL-1
EC
3-4 HOUR
MAb
PEPTIDE
LEUCOCYTES
LEUCOCYTES

FIG. 1

FIG.2A

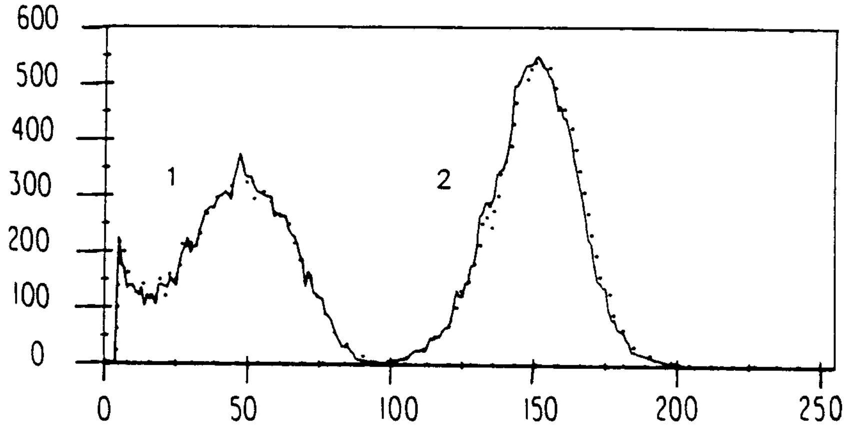


FIG.2B

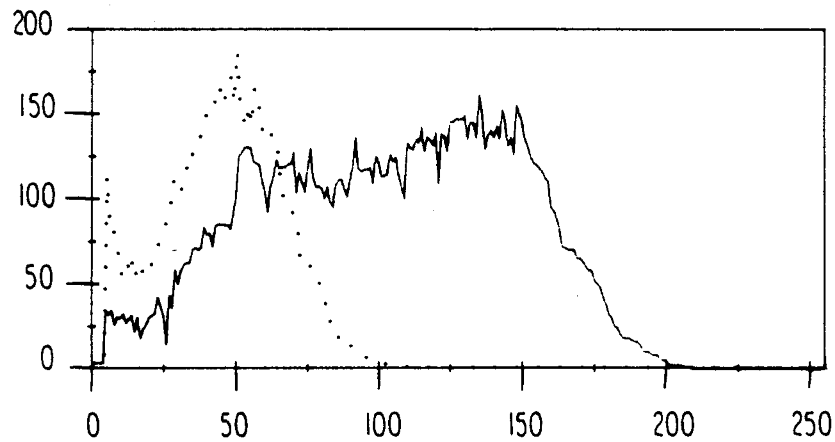


FIG.2C

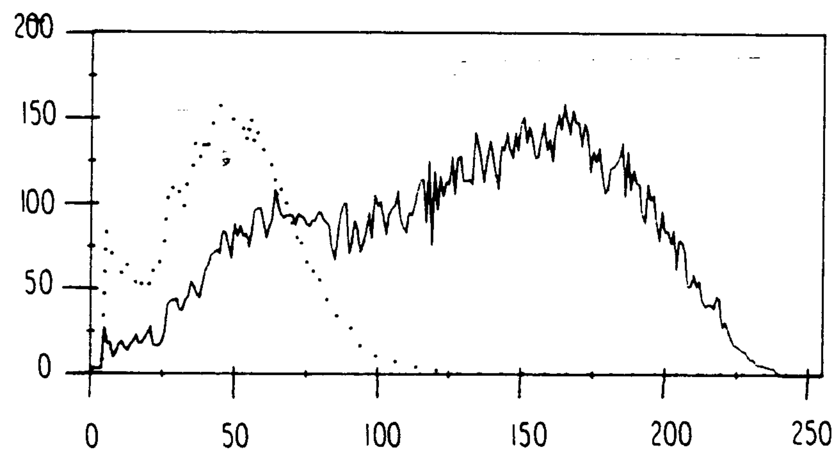

FIG.2D

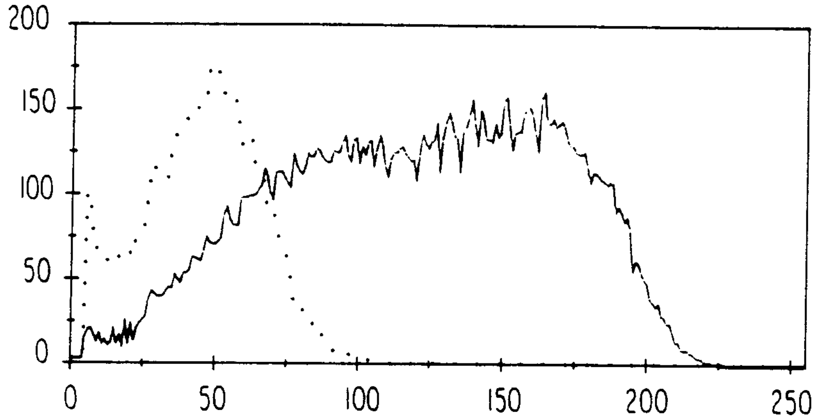


FIG.2E

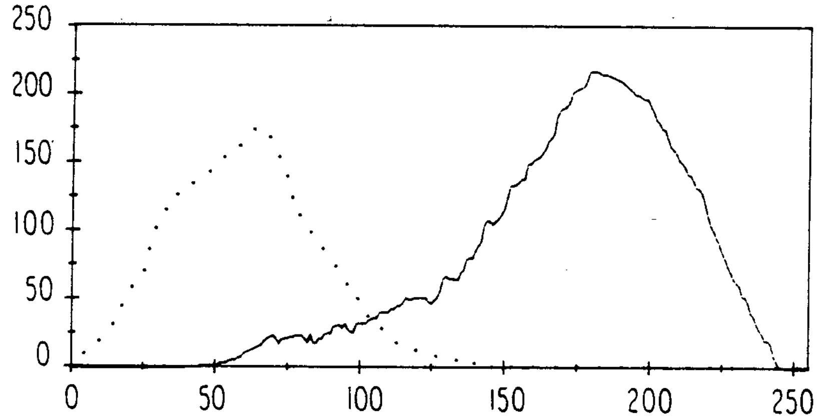


FIG.2F

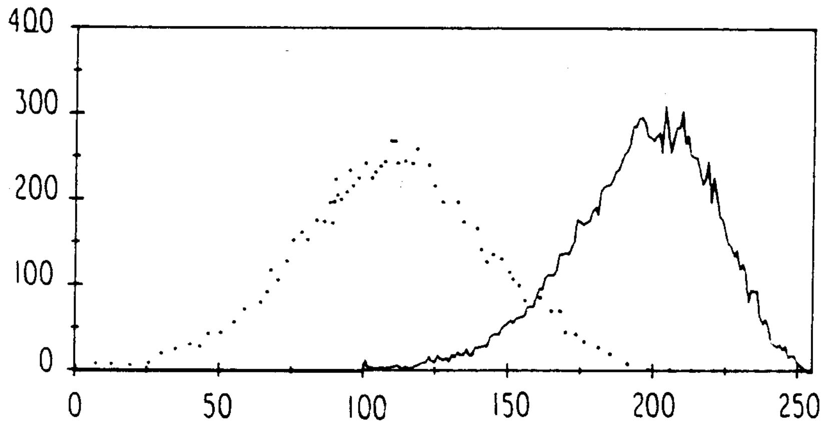

FIG. 3 THE REACTIVITY OF MONOCLONAL ANTIBODIES WITH 1E7/2G7cDNA TRANSFECTED COS CELLS.

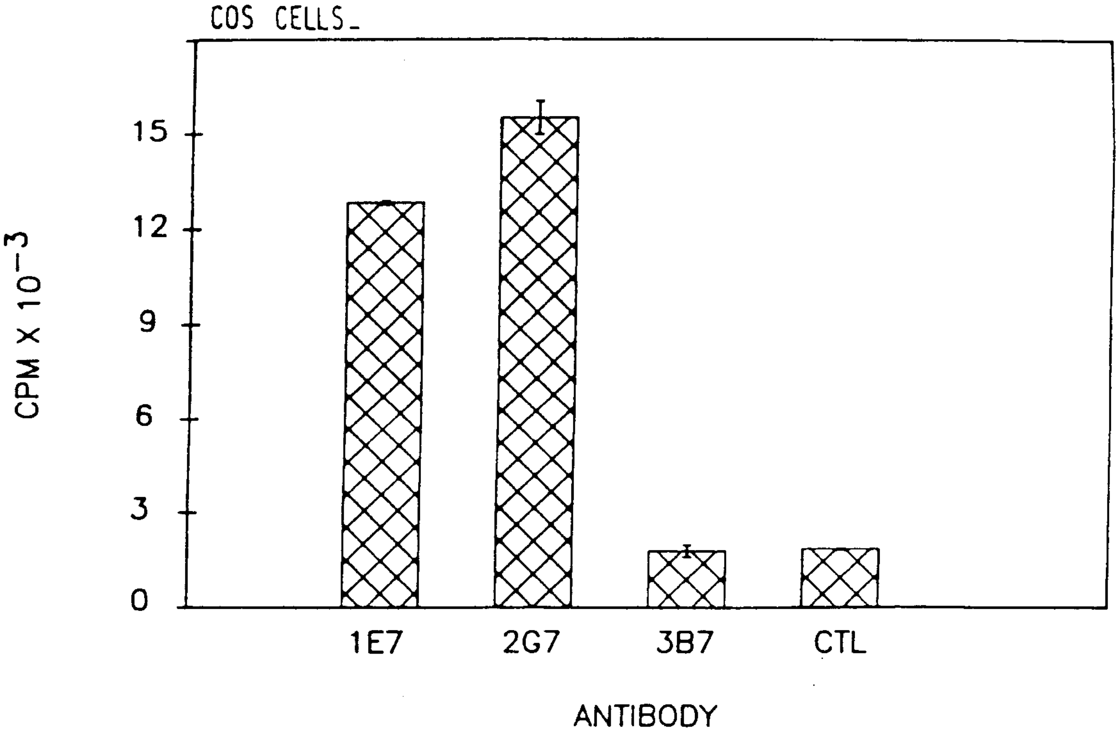


FIG. 4 INHIBITION OF MONONUCLEAR CELL BINDING TO IL-1 ACTIVATED HUVECS

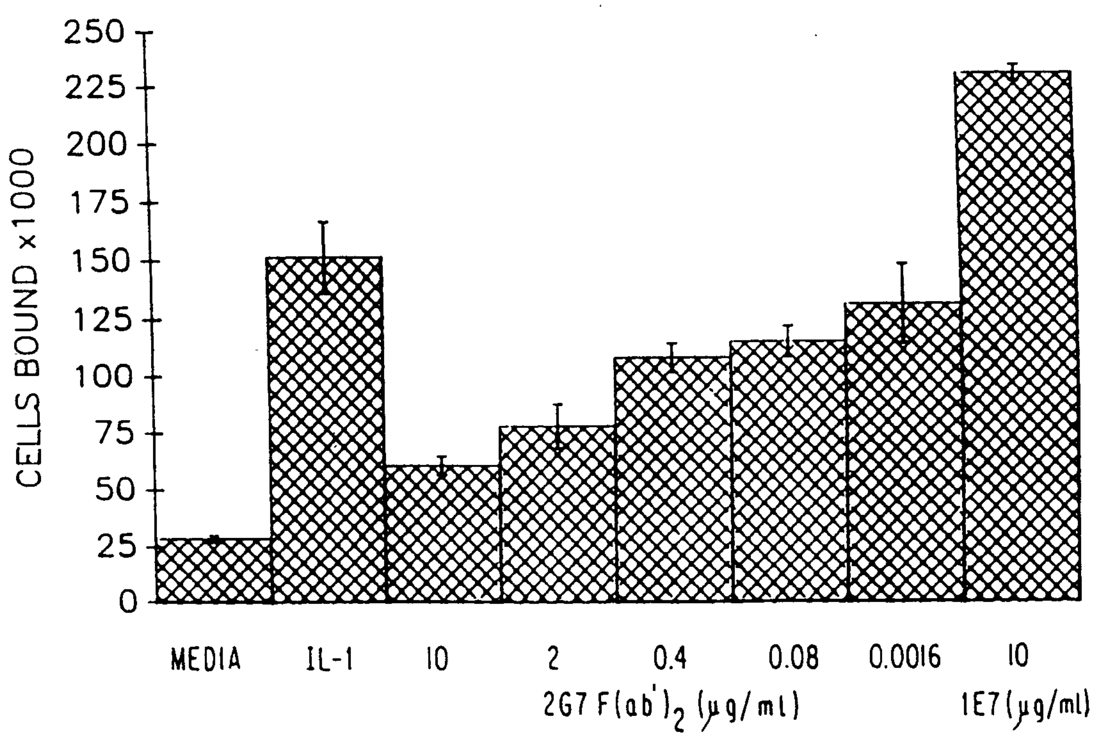

FIG. 5

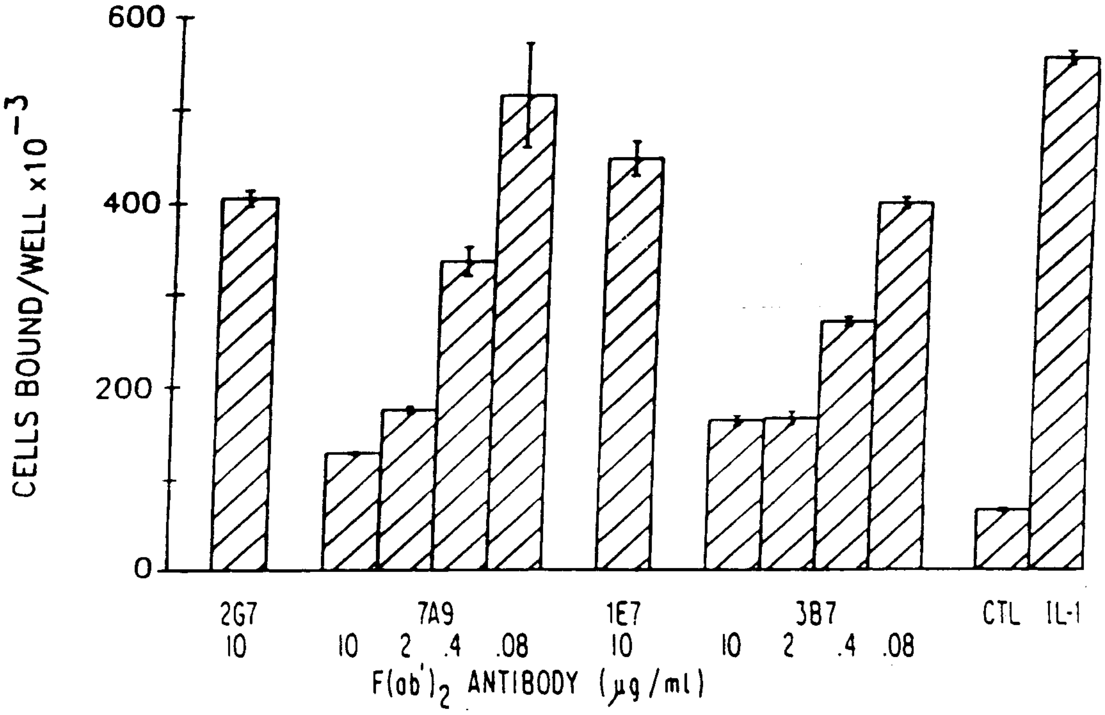
600
400
200
0
CELLS BOUND/WELL x10$^{-3}$
2G7
10
7A9
10 2 .4 .08
1E7
10
3B7
10 2 .4 .08
CTL IL-1
F(ab')$_2$ ANTIBODY (μg/ml)

FIG. 6

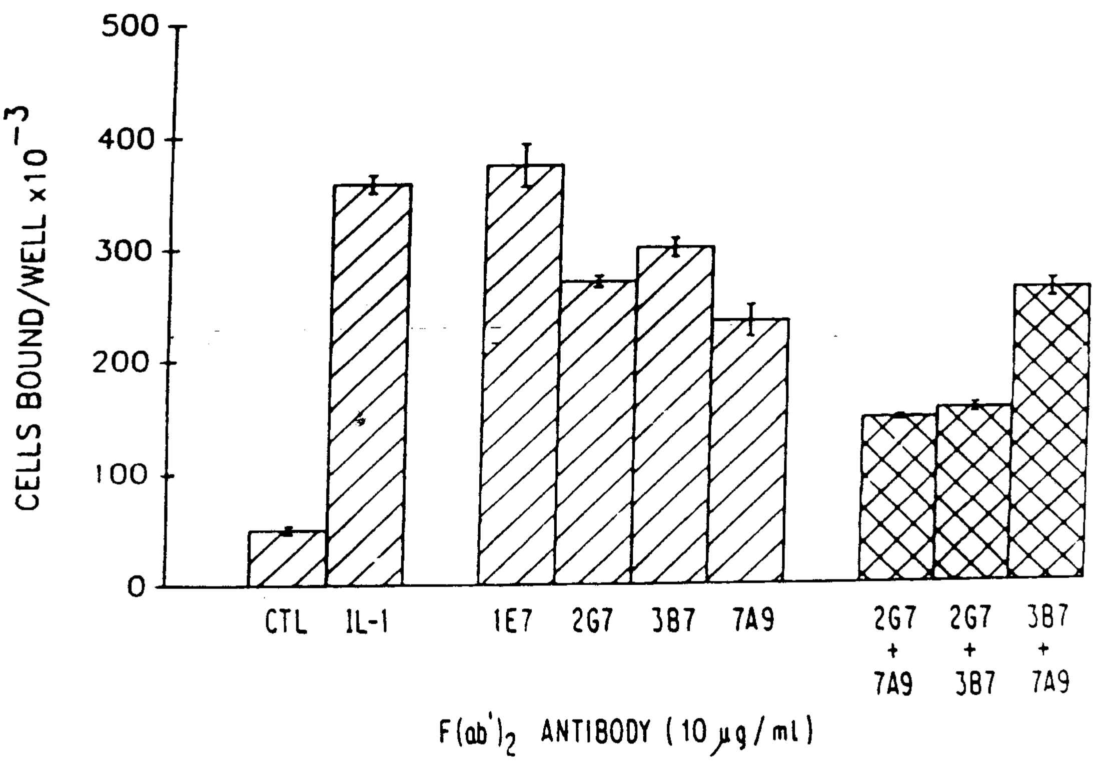
500
400
300
200
100
0
CELLS BOUND/WELL x10$^{-3}$
CTL IL-1
1E7 2G7 3B7 7A9
2G7 + 7A9
2G7 + 3B7
3B7 + 7A9
F(ab')$_2$ ANTIBODY (10 μg/ml)

FIG.7

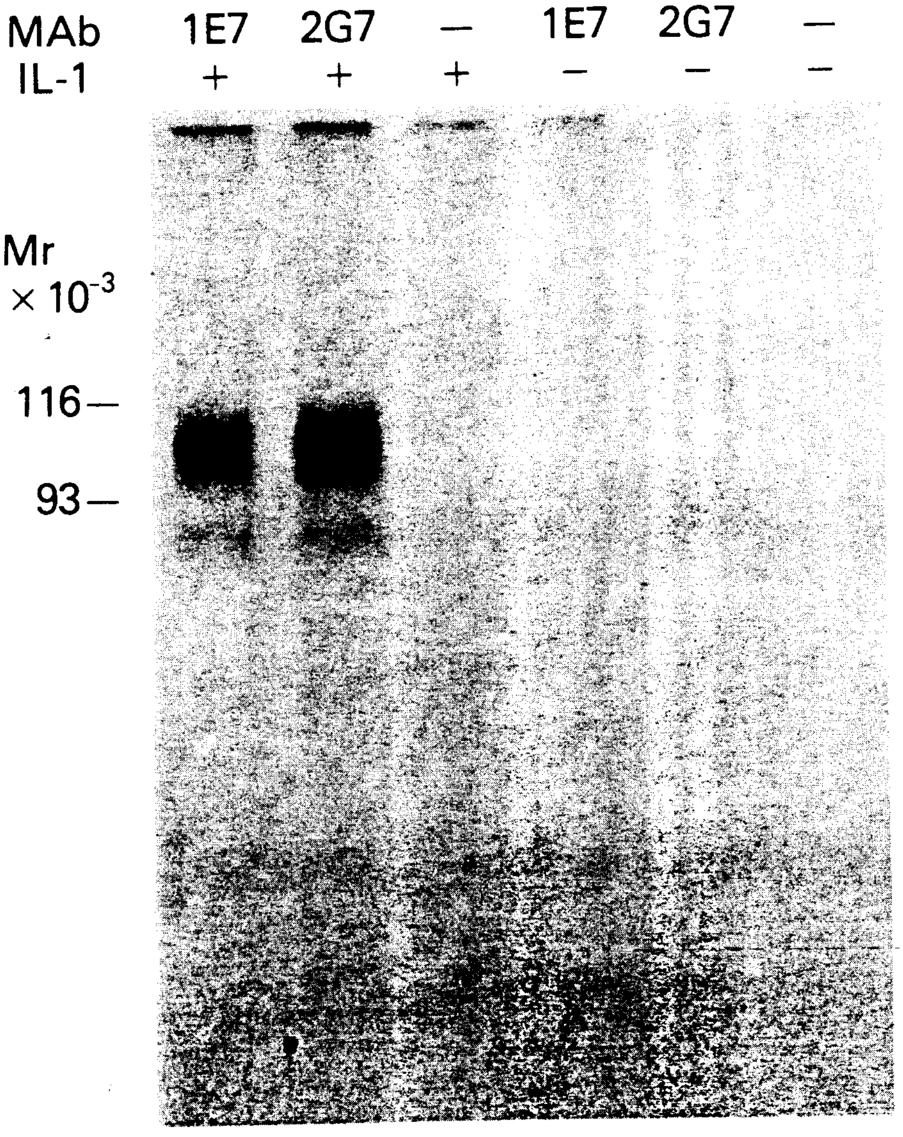

FIG.8A

FIG.8B

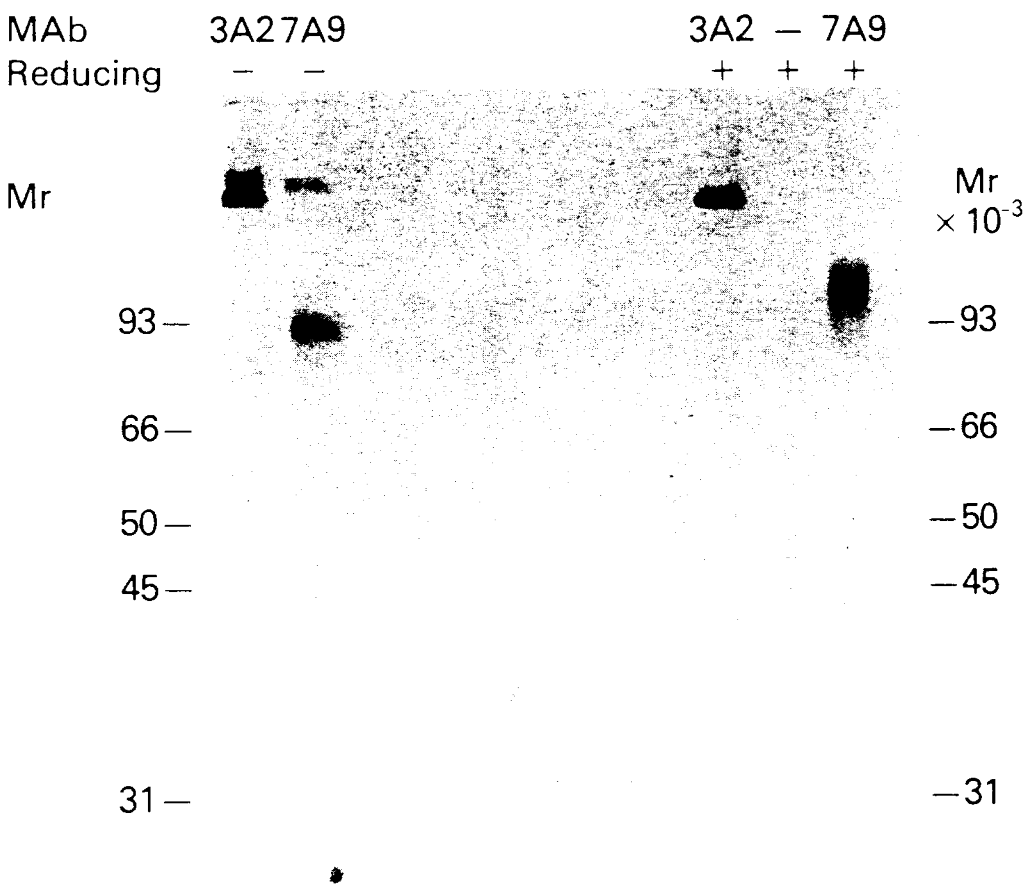
MAb
3A2
7A9
Reducing
–
–
Mr
93
66
50
45
31
3A2
–
7A9
+
+
+
Mr
× 10⁻³
93
66
50
45
31

FIG.9 EFFECT OF IL-1 ON EC ANTIGEN EXPRESSION.

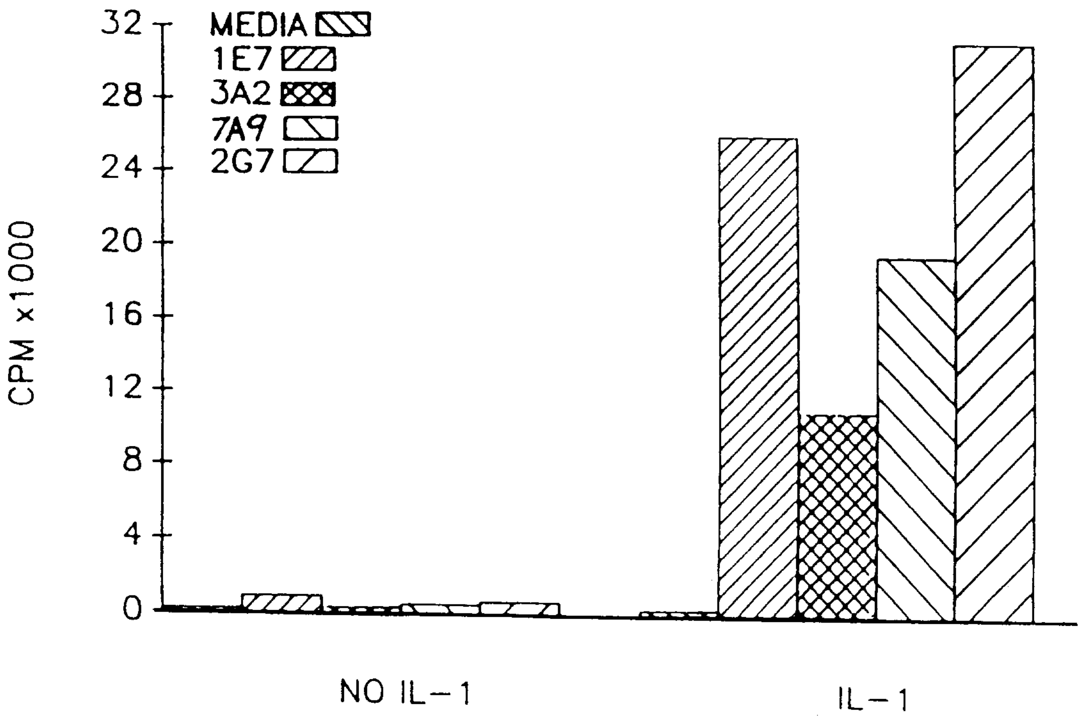


FIG.10 ENDOTHELIAL ANTIGEN EXPRESSION ON LEUCOCYTES.

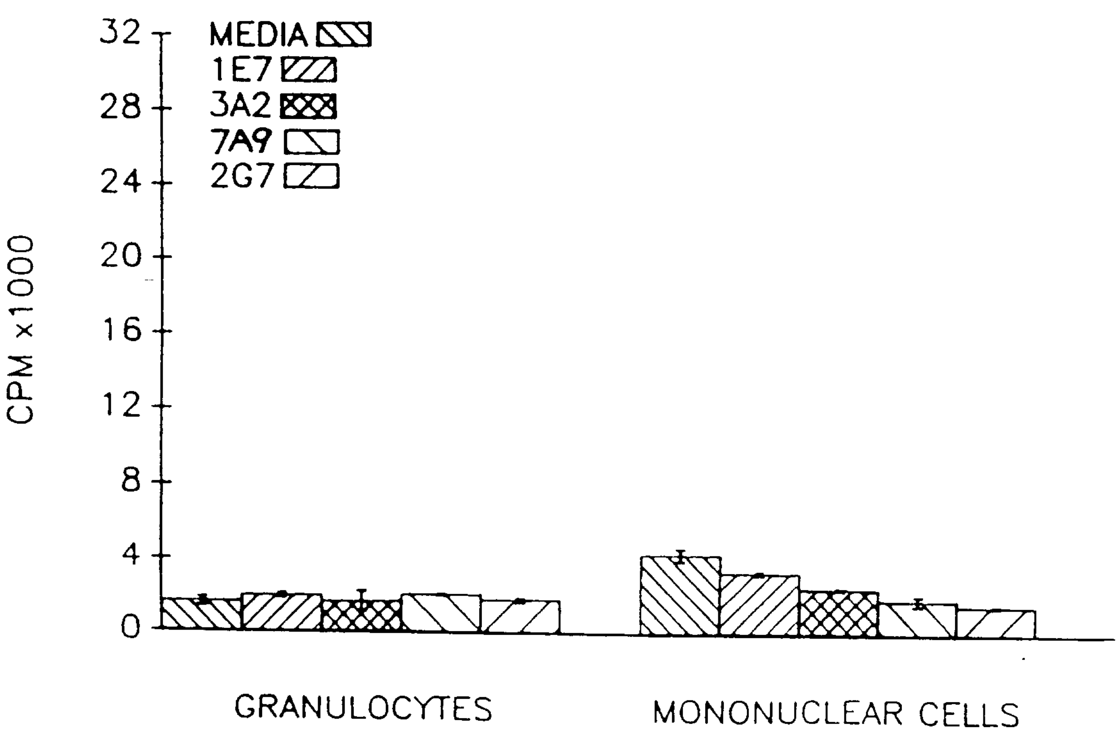

FIG. 11

HUVE CELLS INCUBATED WITH IL-1 FROM 2 HOURS TO 96 HOURS THEN STAINED WITH DIFFERENT ANTIBODIES

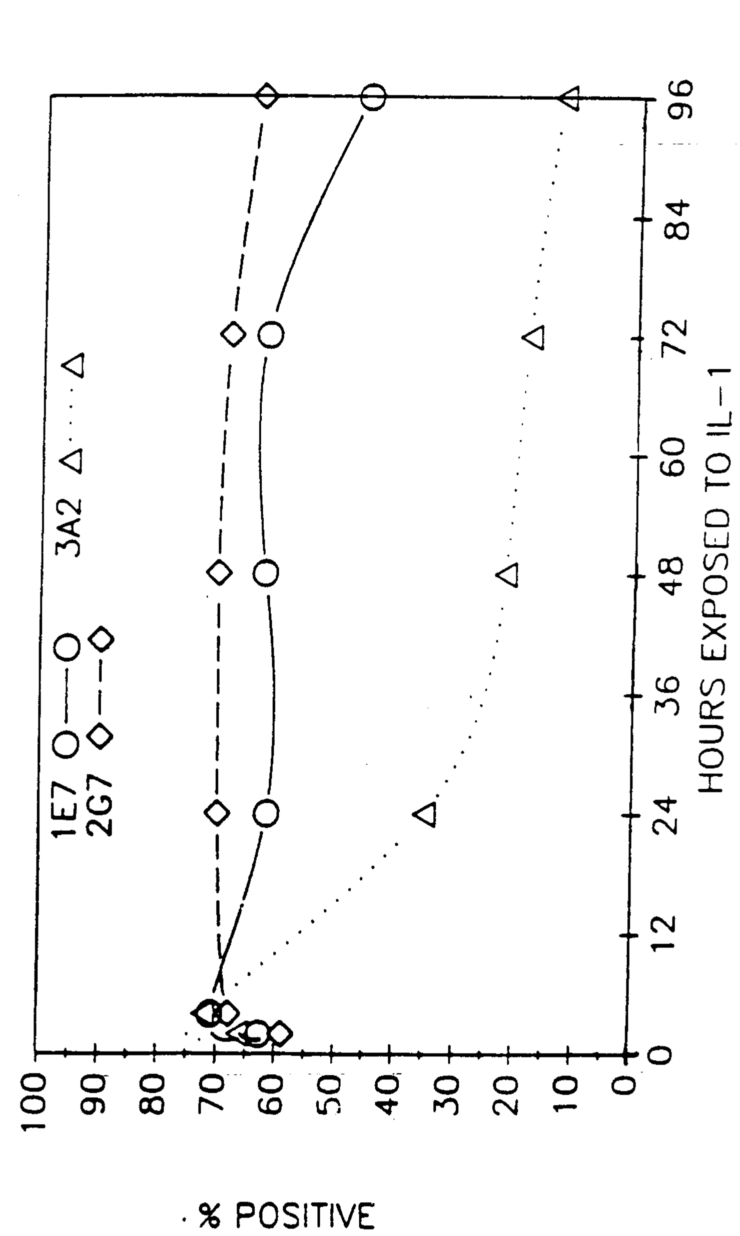

FIG.12B

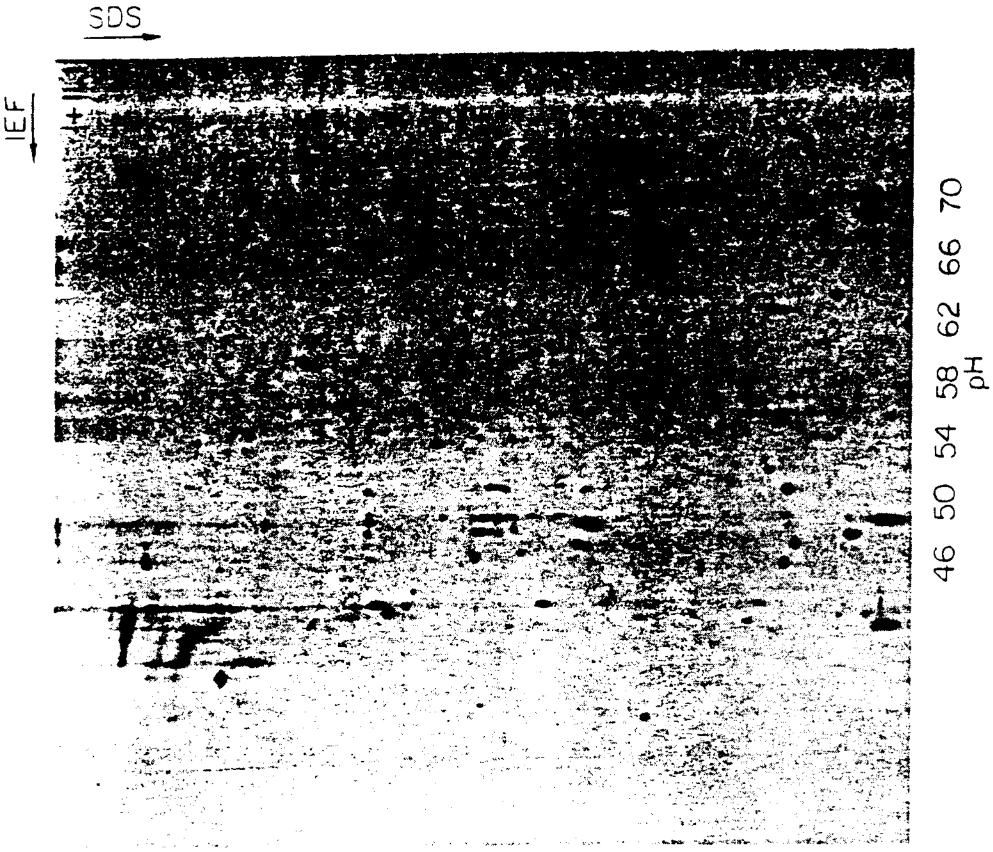


FIG.12A

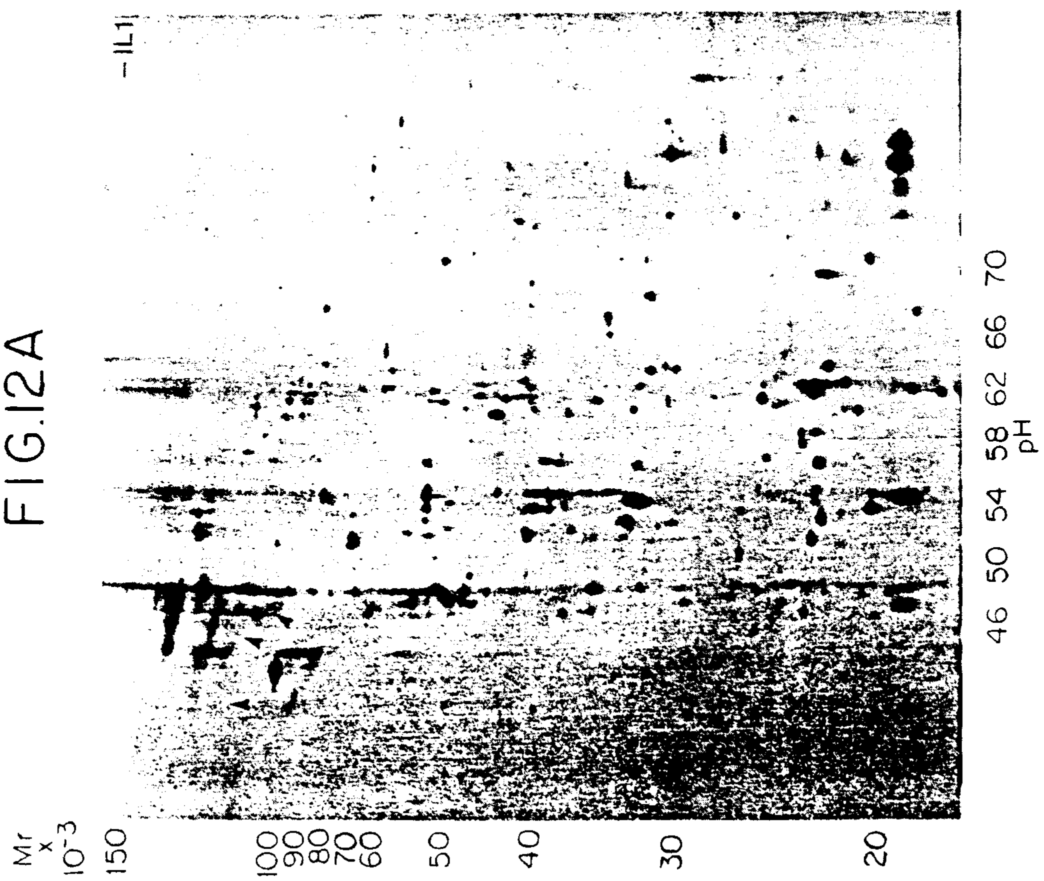

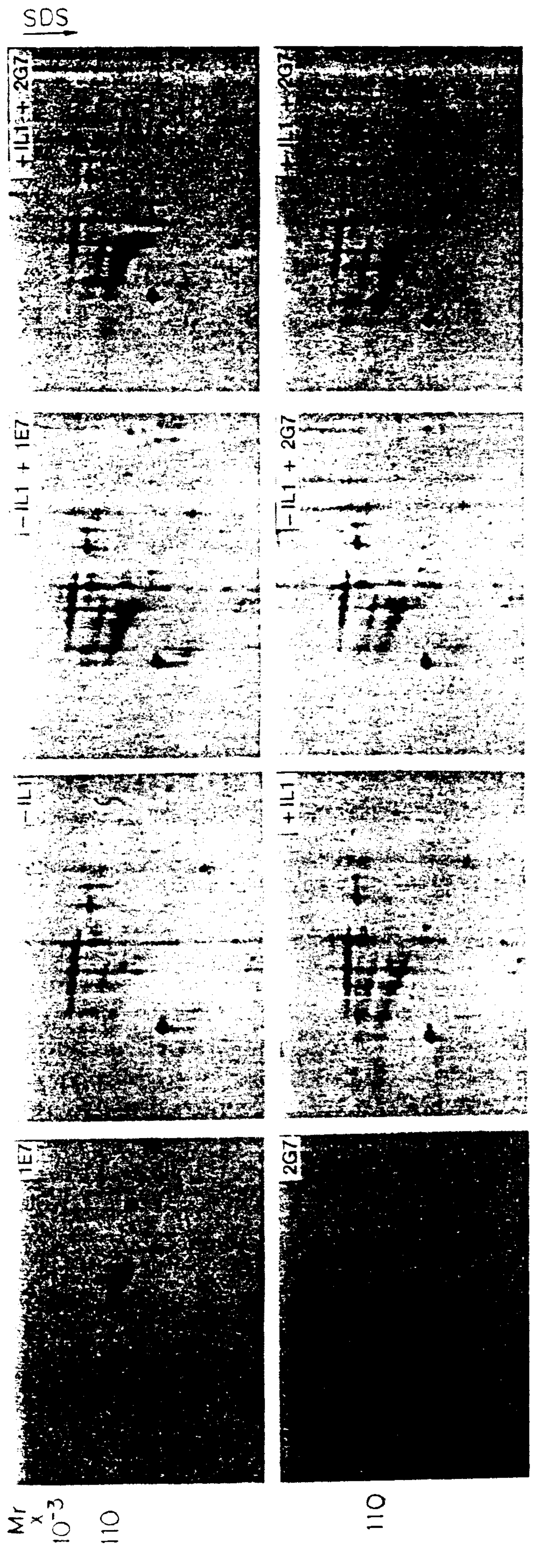


FIG.13A

FIG.13B

FIG.13C

FIG.13D

FIG.13E

FIG.13F

FIG.13G

FIG.13H

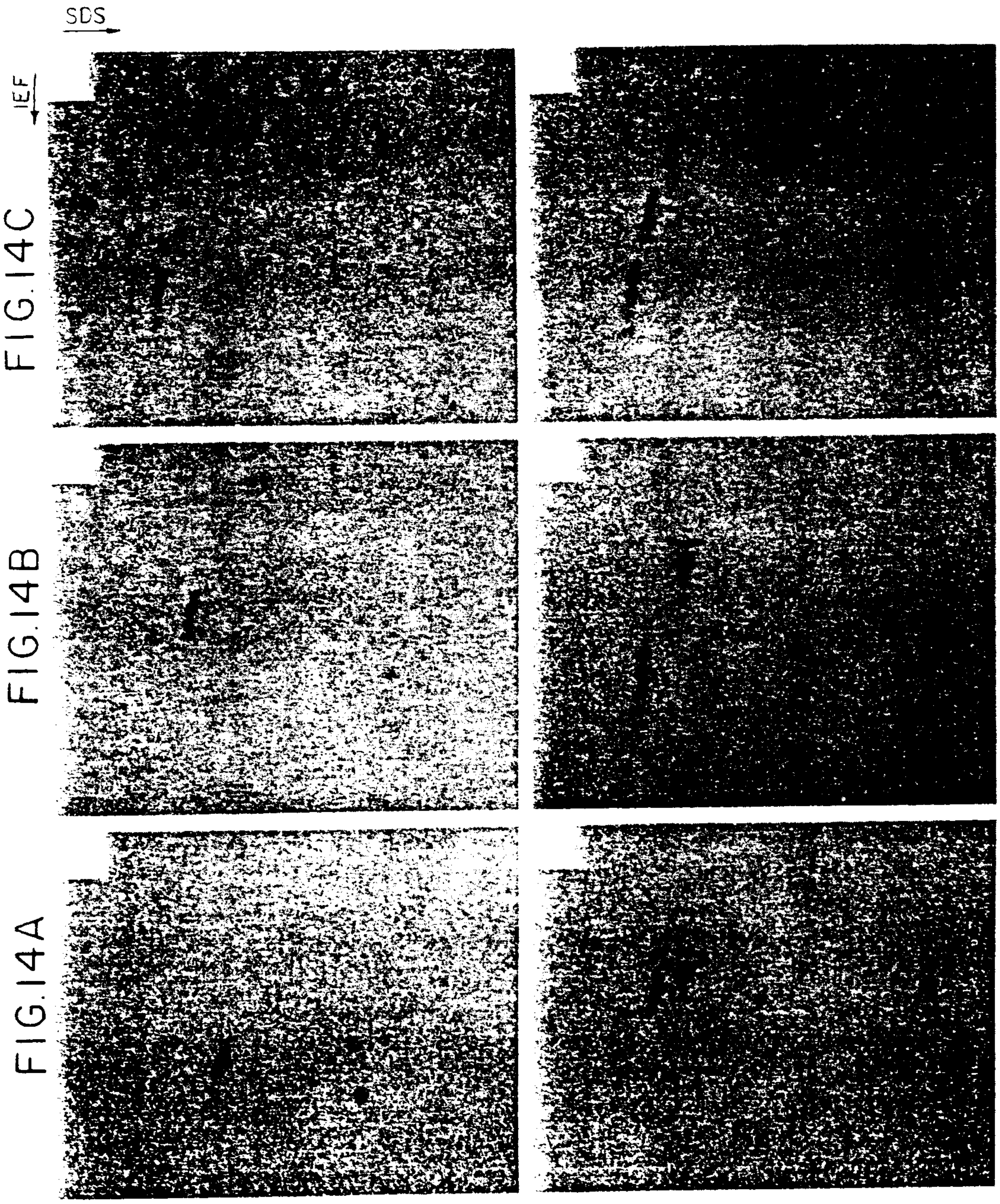


FIG.14A FIG.14B FIG.14C

FIG.14D FIG.14E FIG.14F

FIG.15

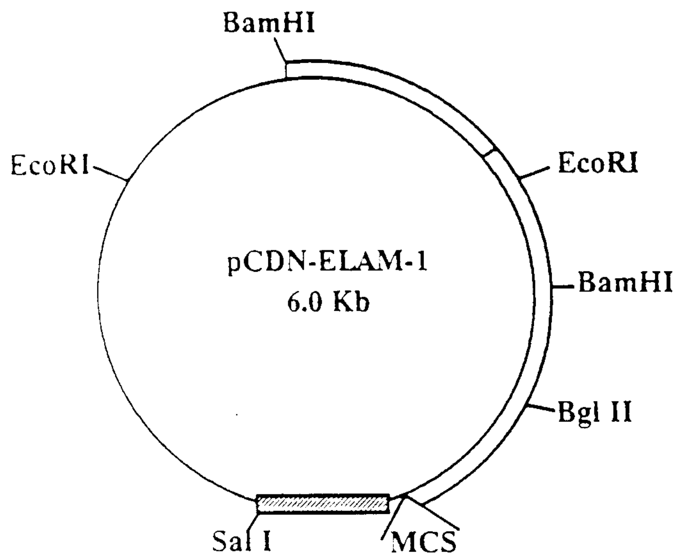
BamHI
EcoRI
EcoRI
pCDN-ELAM-1
6.0 Kb
BamHI
Bgl II
Sal I
MCS

FIG.16

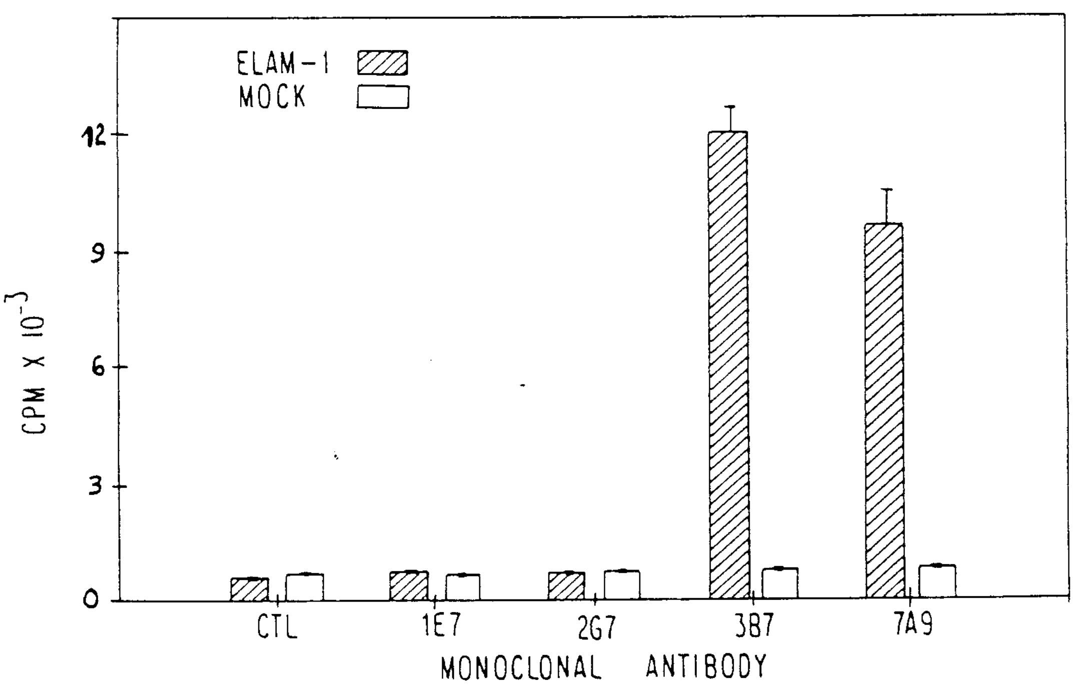
ELAM-1
MOCK
12
9
6
3
0
CPM X $10^{-3}$
CTL
1E7
2G7
3B7
7A9
MONOCLONAL ANTIBODY

FIG.17

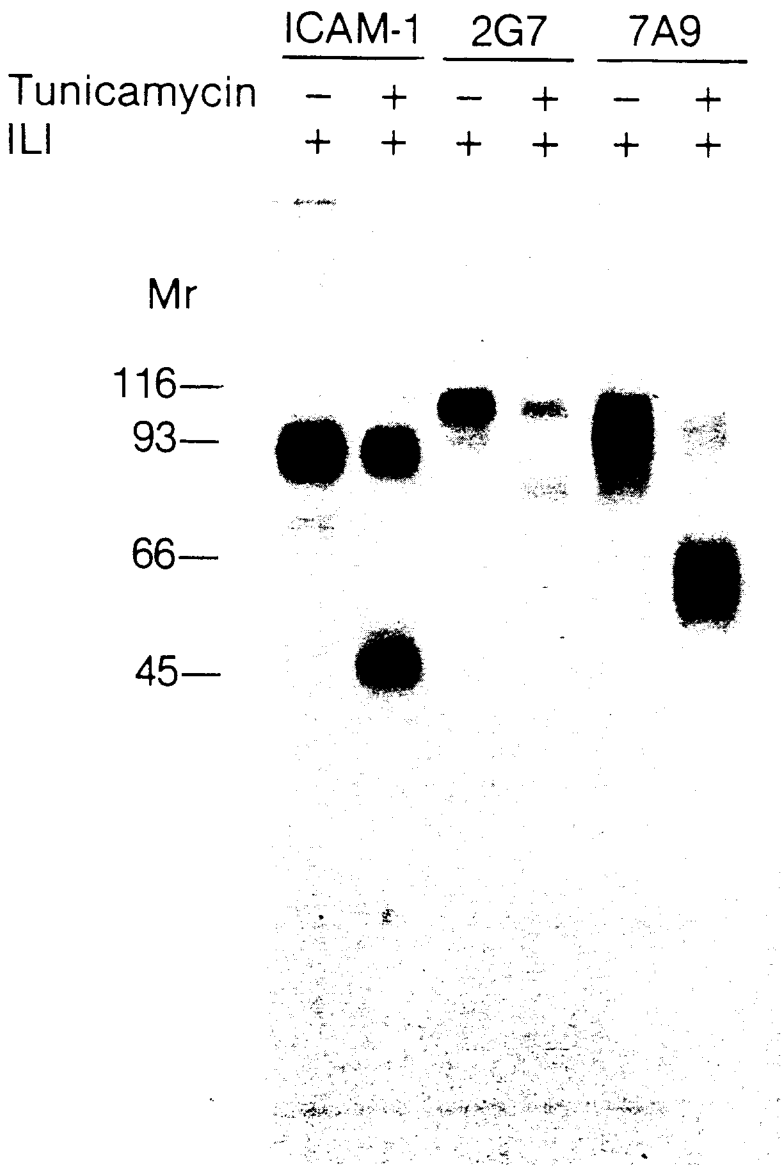

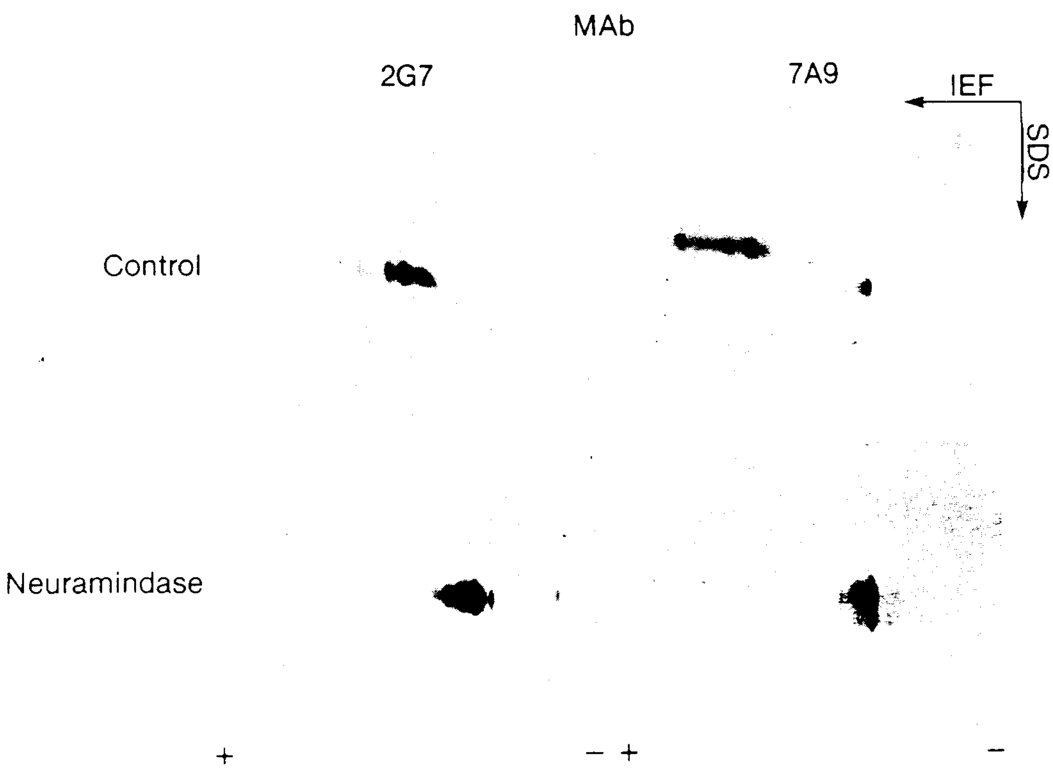


FIG.18A FIG.18B

FIG.19

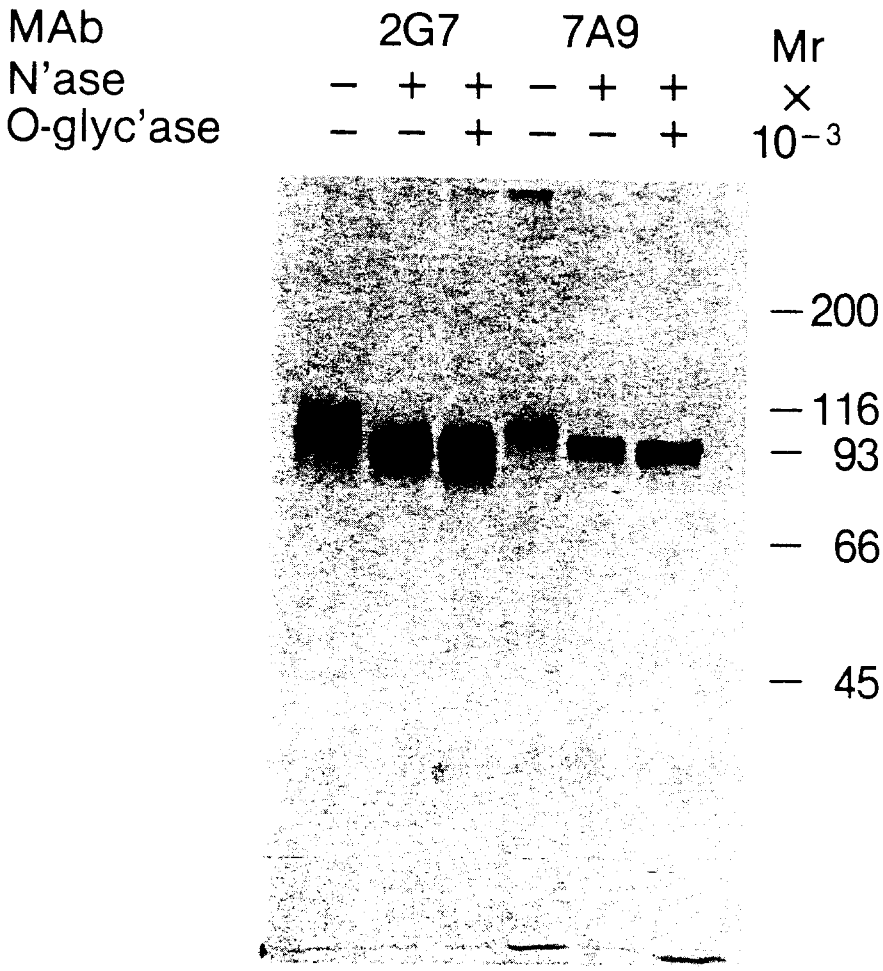

FIG.20

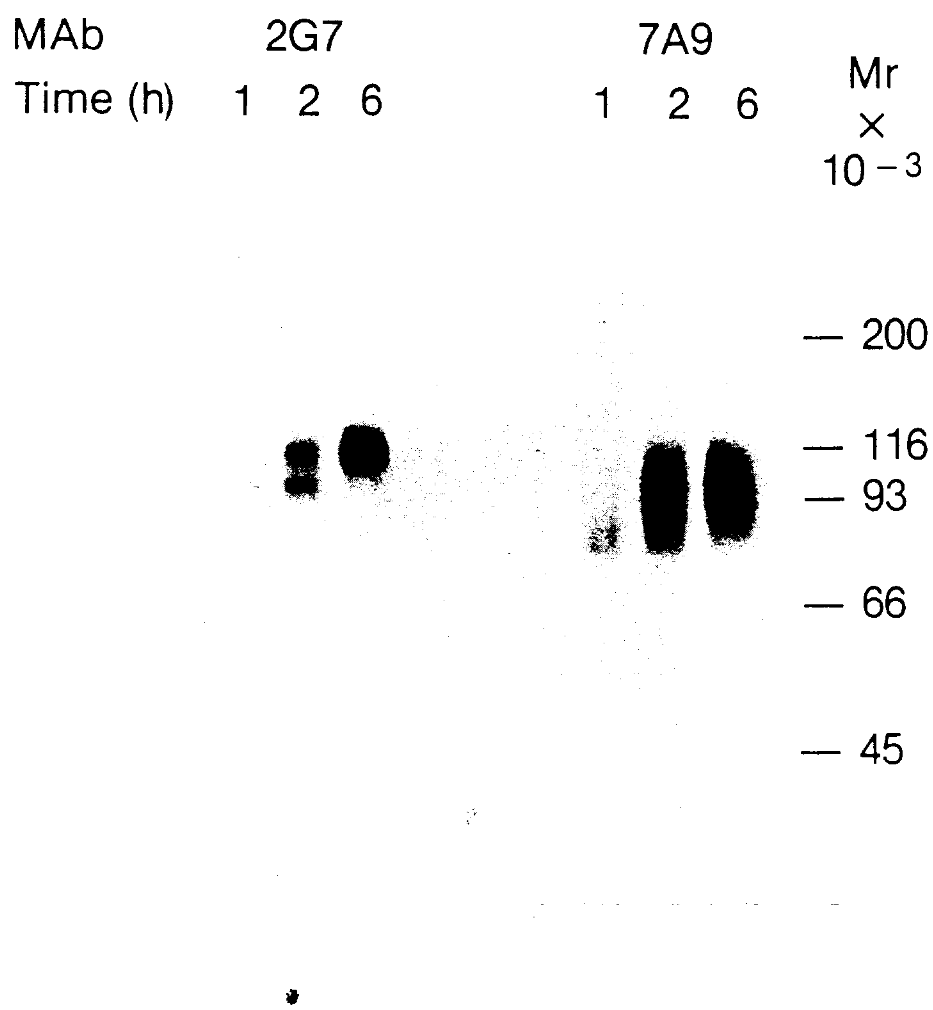

FIG. 21A

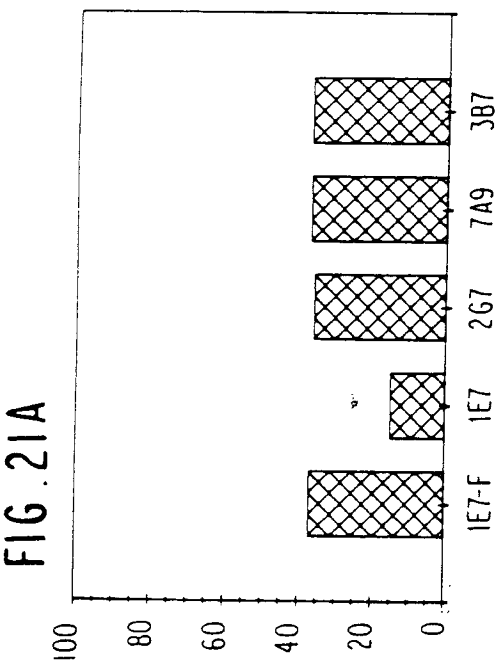


FIG. 21B

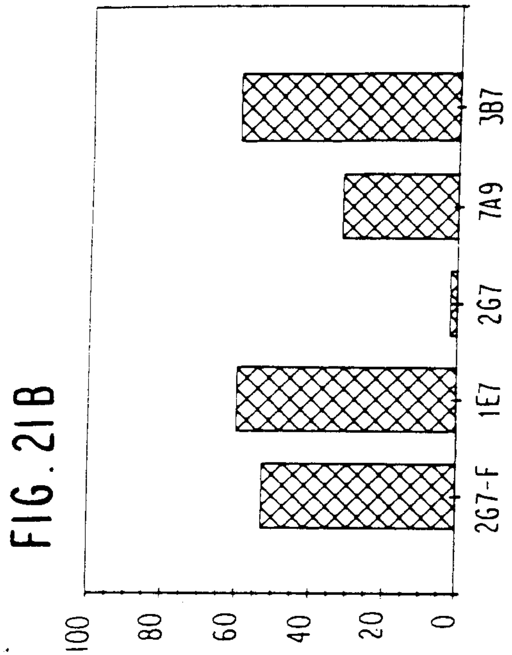


FIG. 21C

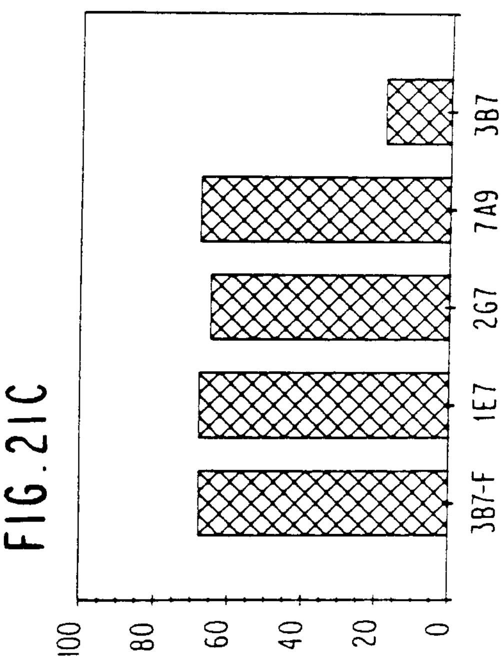


FIG. 21D

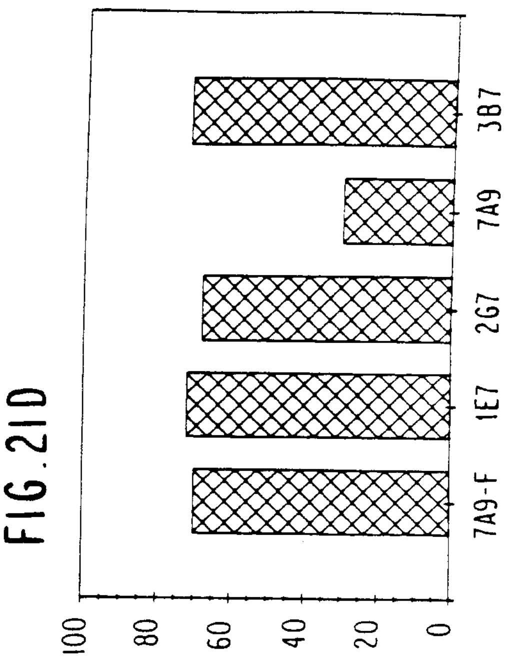

FIG. 22

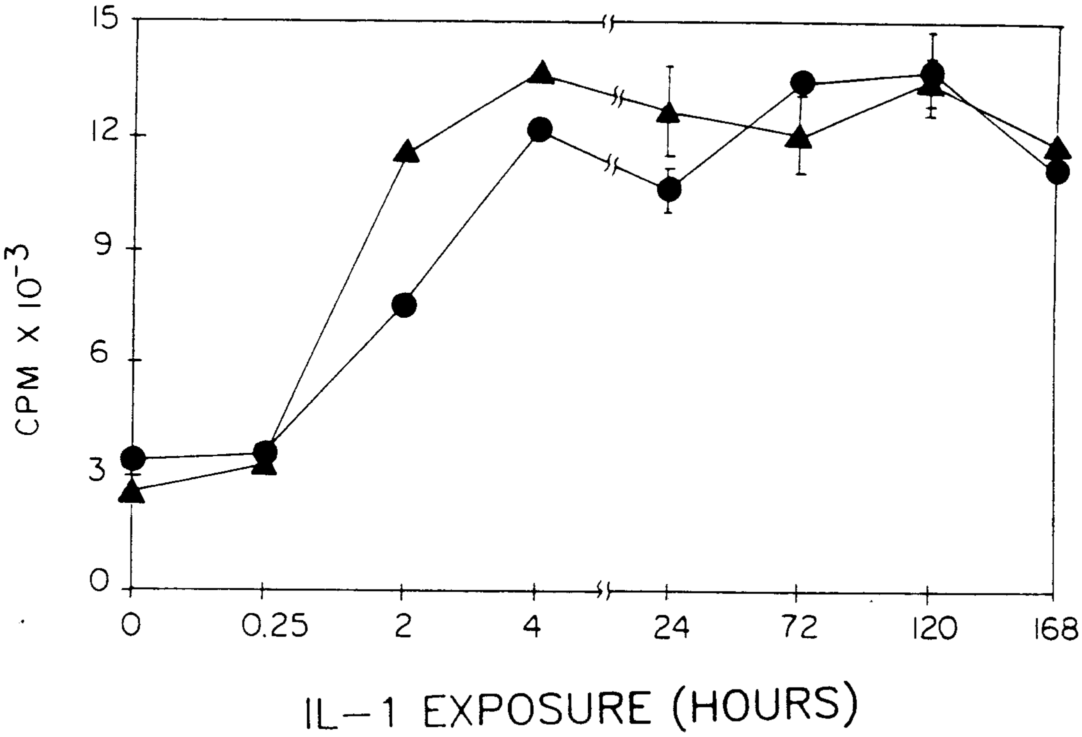

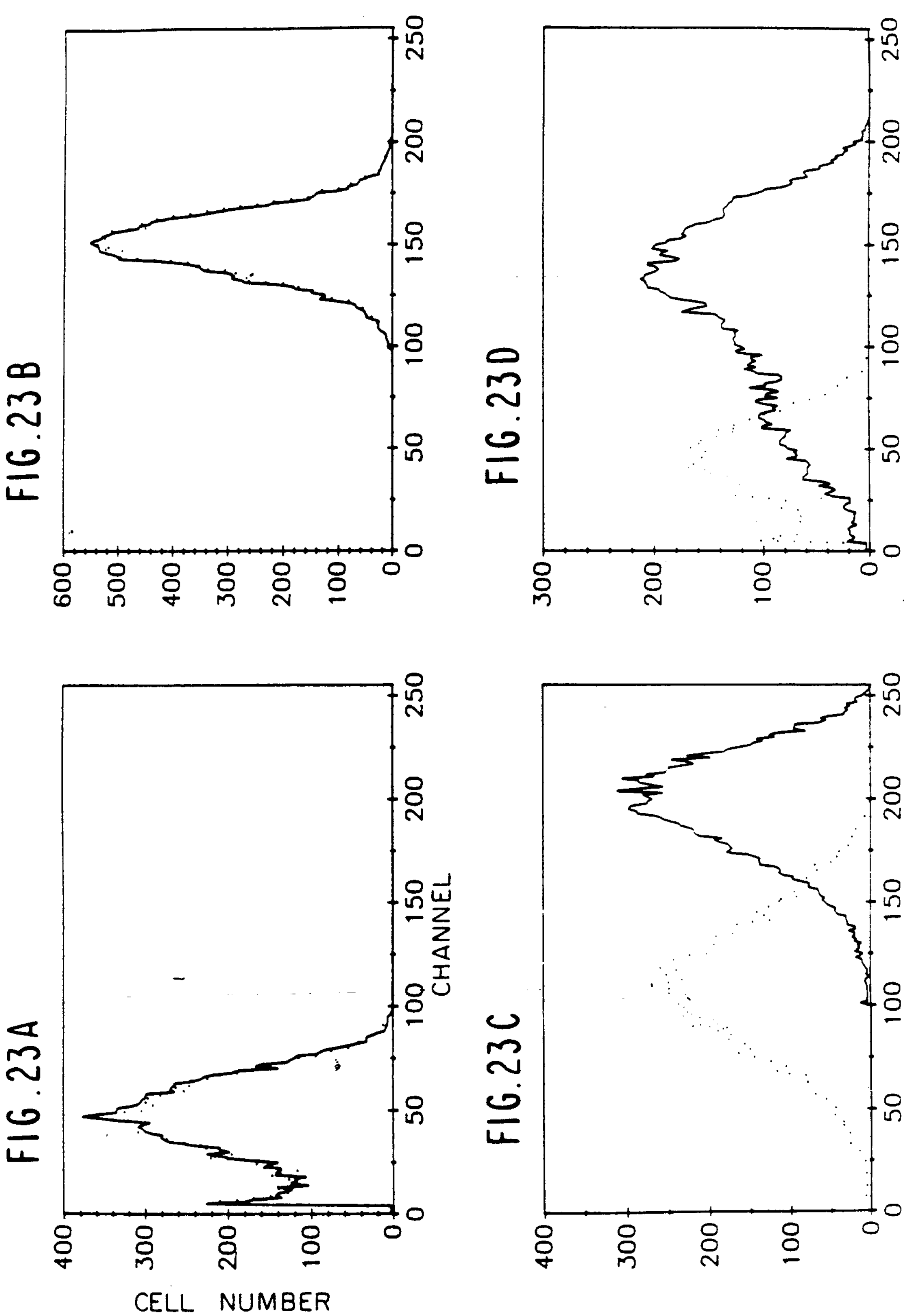


FIG. 23A
FIG. 23B
FIG. 23C
FIG. 23D

FIG.24

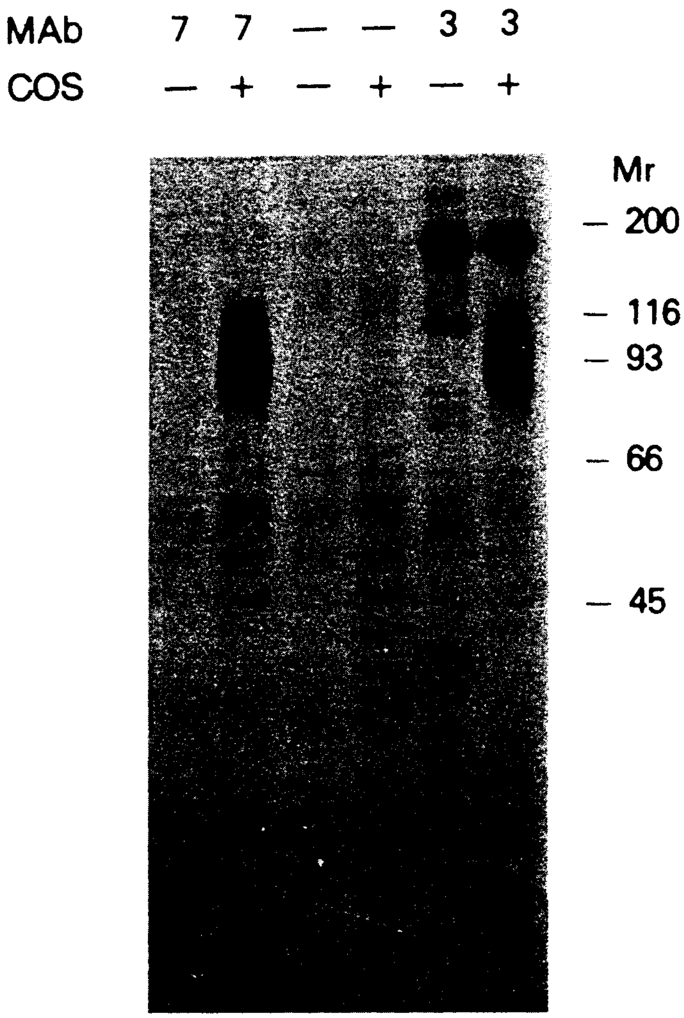

FIG.25A

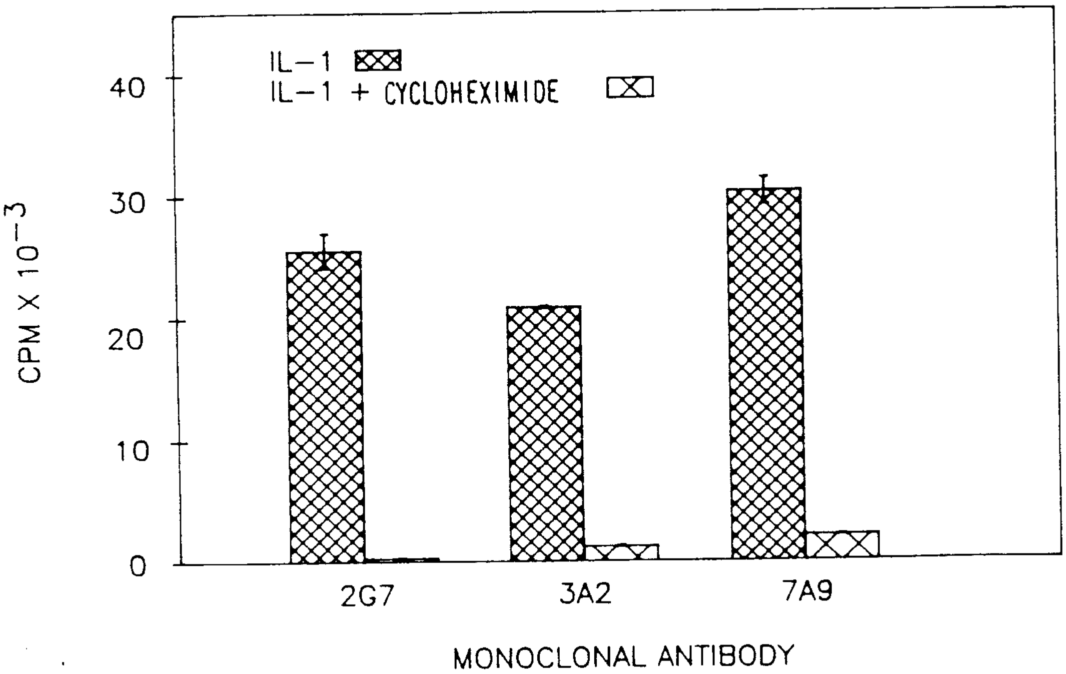


FIG.25B

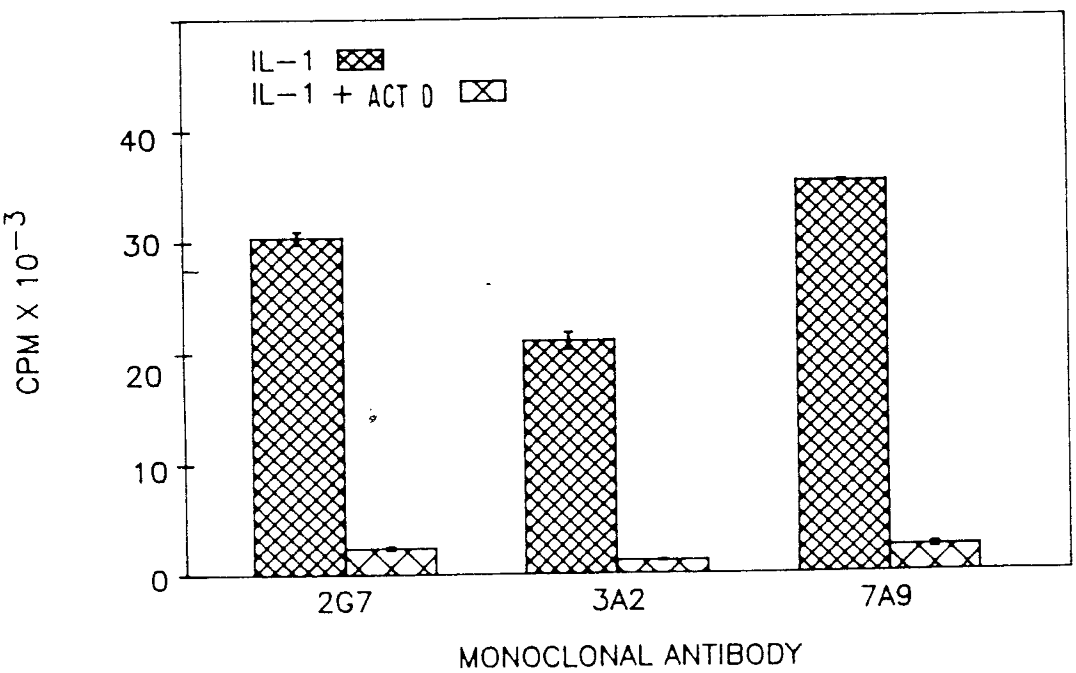

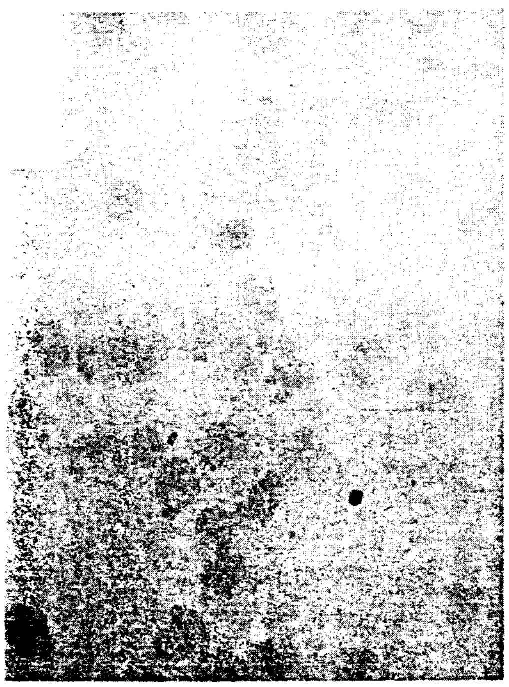

FIG.26A

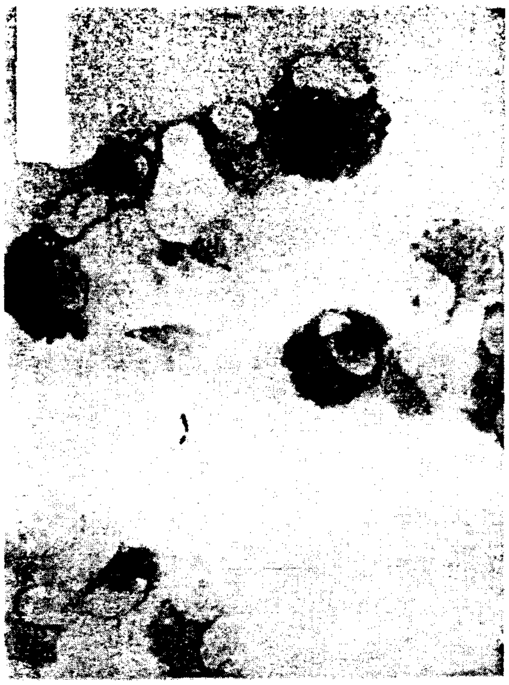

FIG.26B

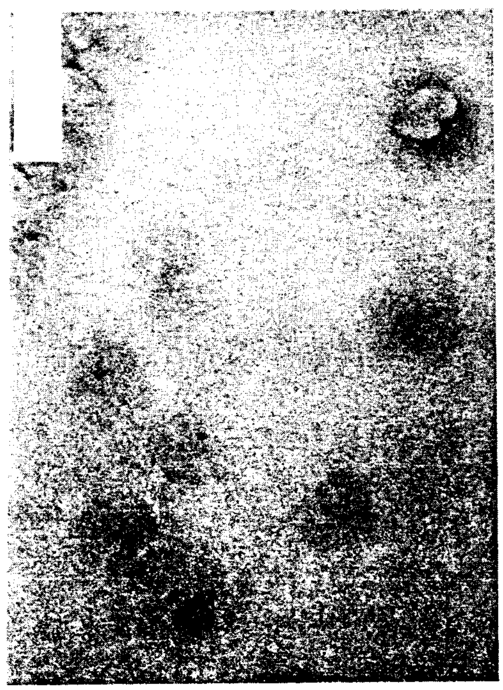

FIG.26C

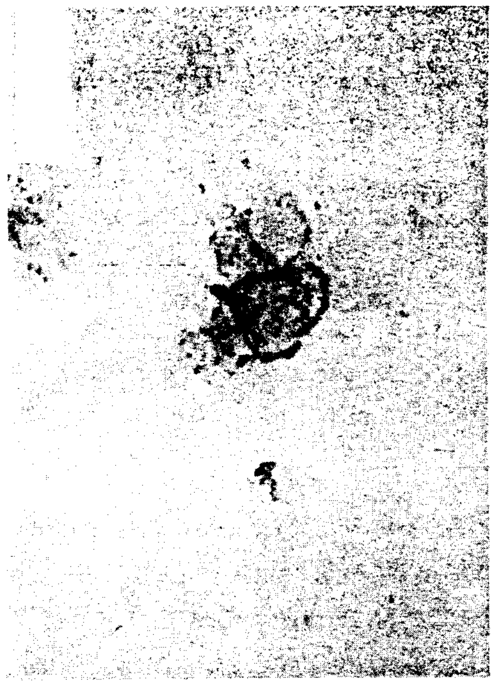

FIG.26D

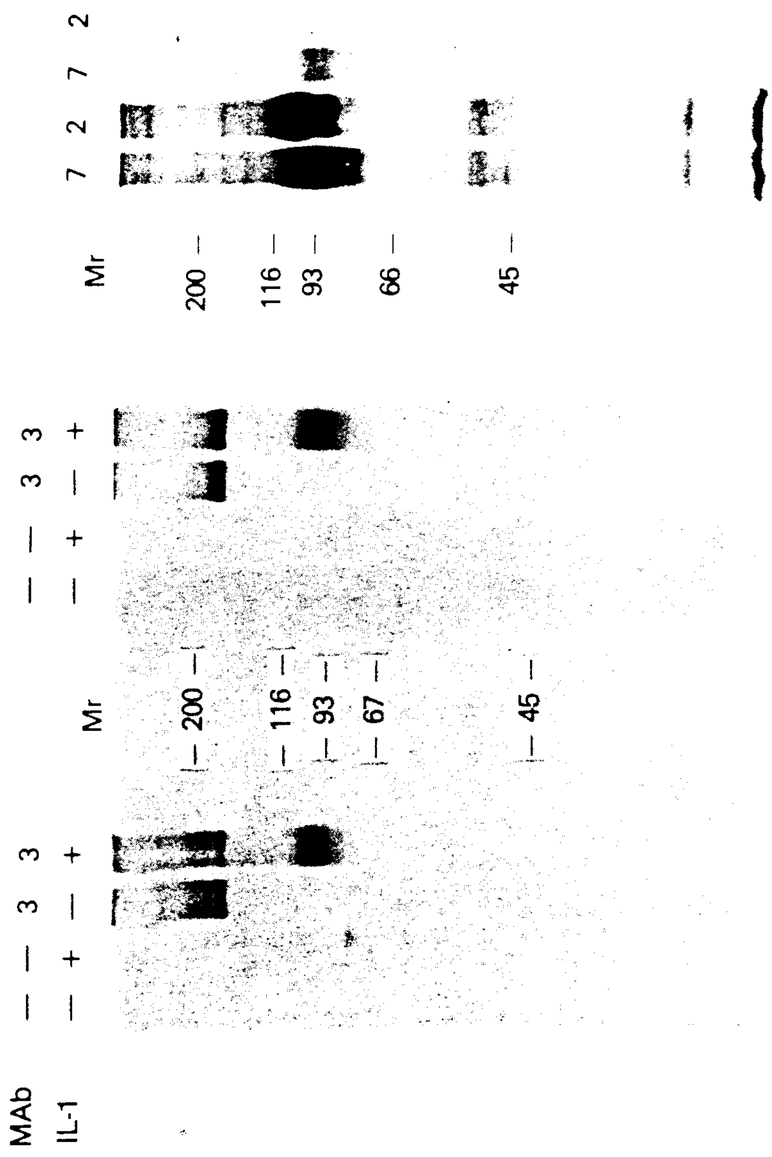


FIG.27A

FIG.27B

FIG.27C

FIG.28

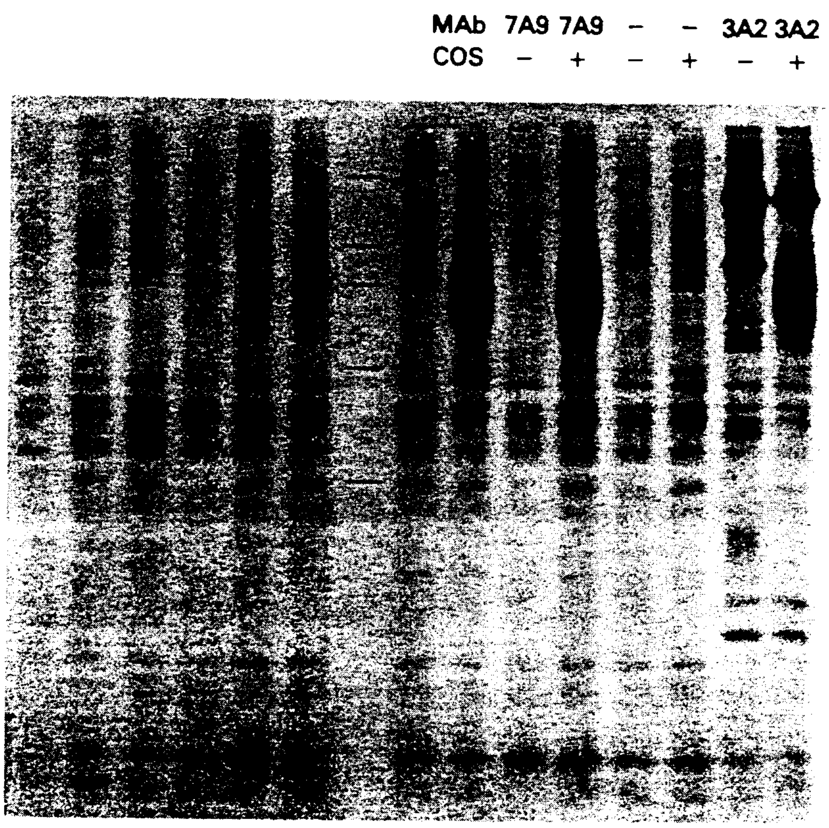

FIG.29A

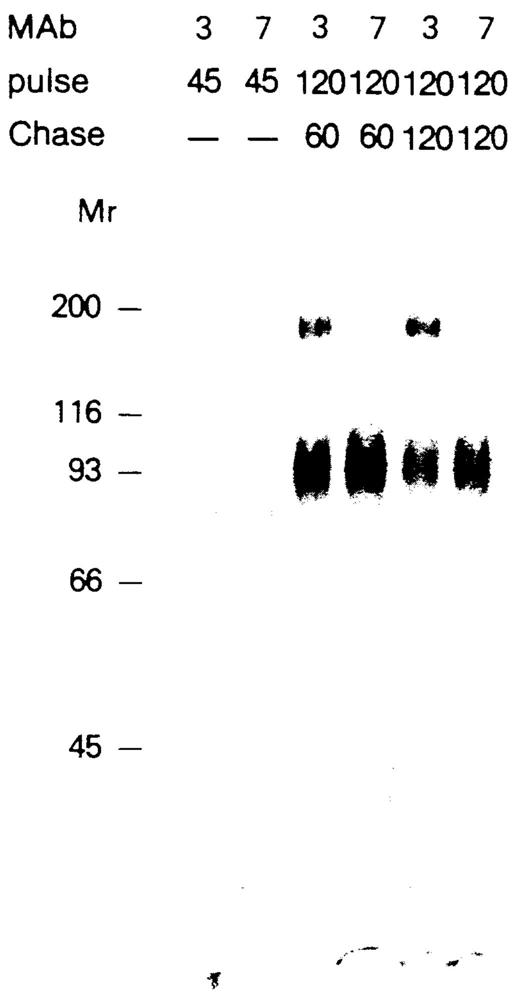


FIG.29B

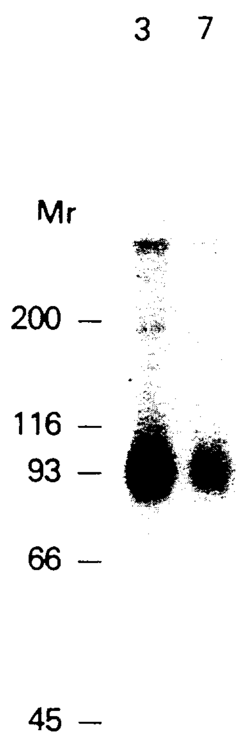

FIG.30

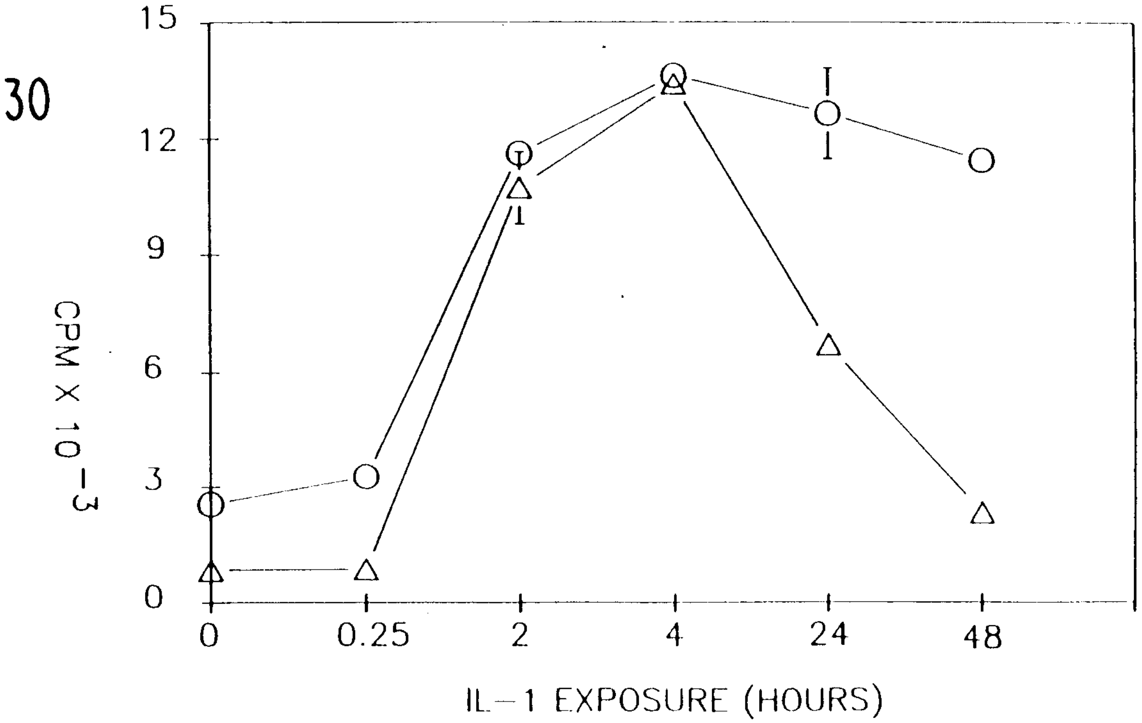


FIG.33 REPORTED SEQUENCE OF VCAM-1 ANTIGEN AND SEQUENCE FOR 1E7/2G7 ANTIGEN RECOVERED BY PCR

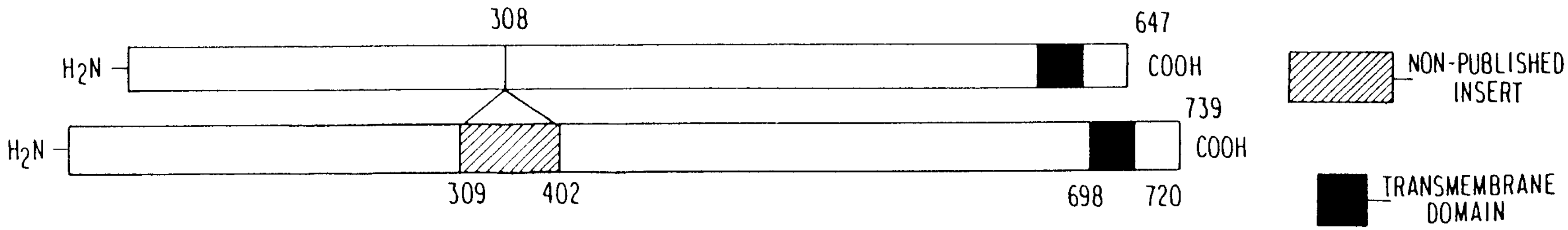

FIG. 31 ELAM-1 AND TRUNCATED VERSIONS: cDNA

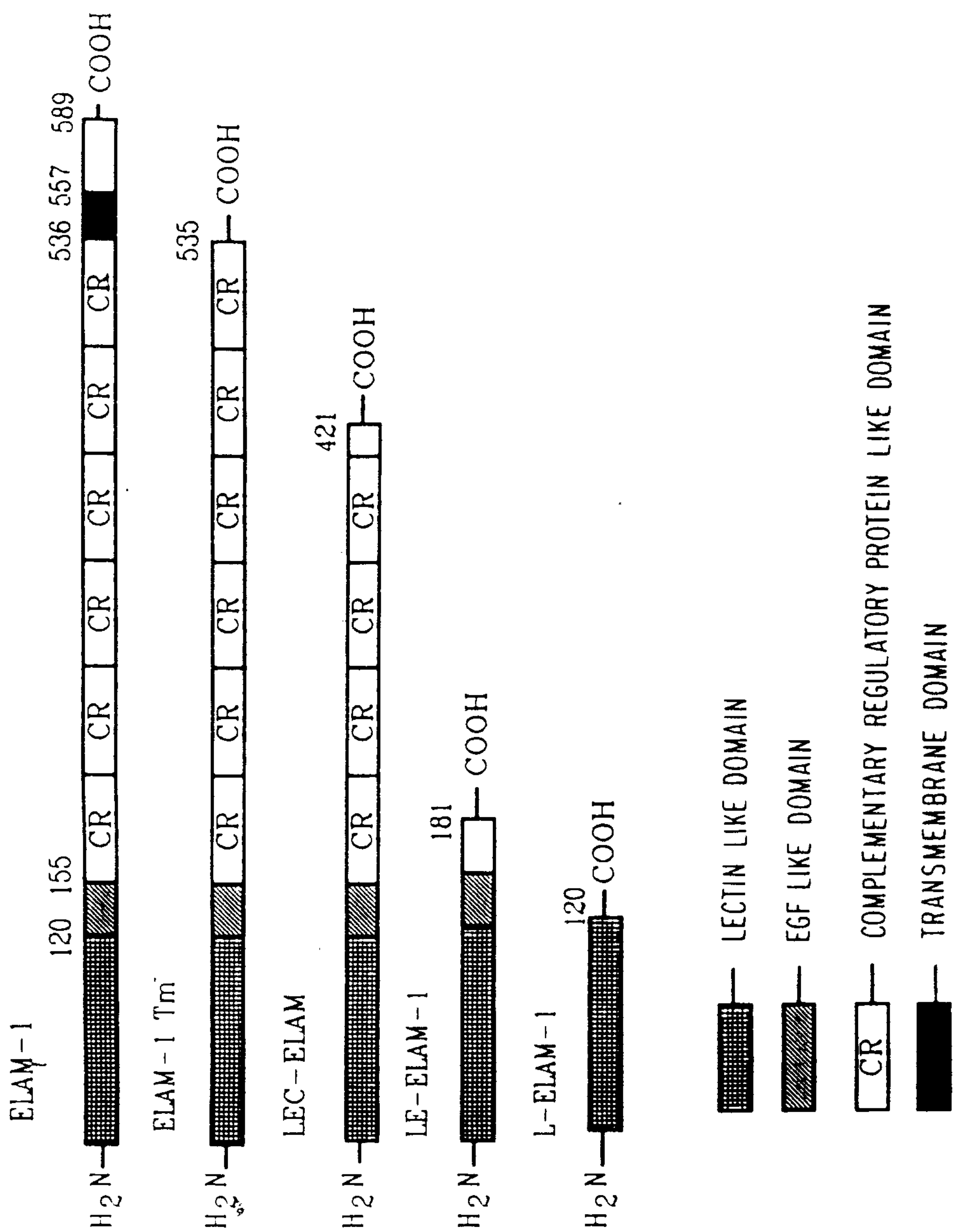

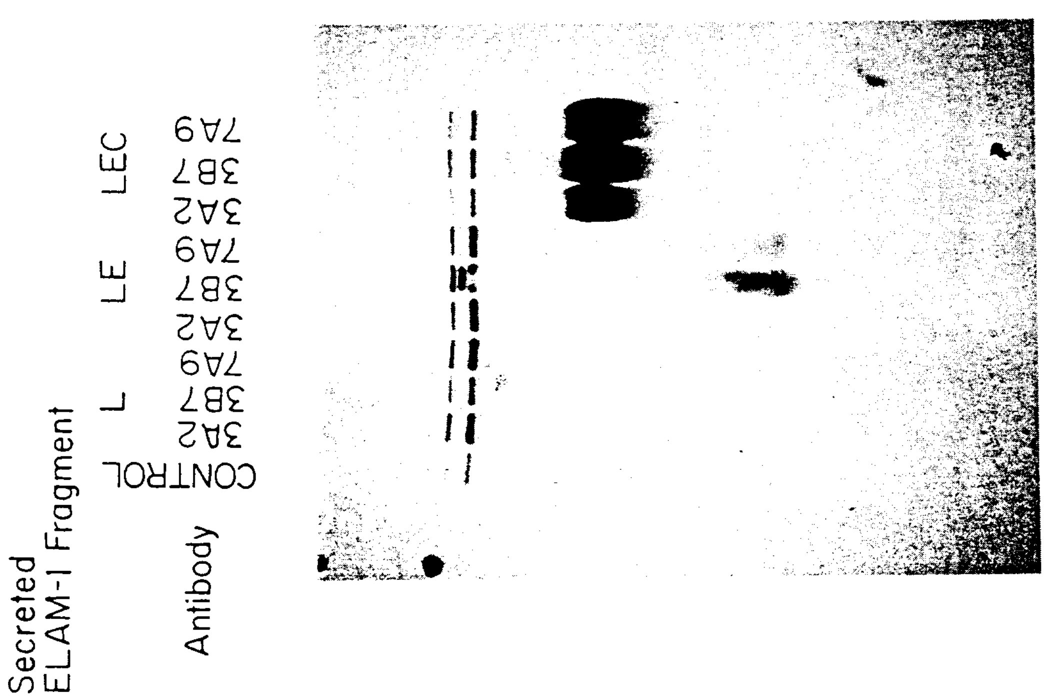


FIG. 32A

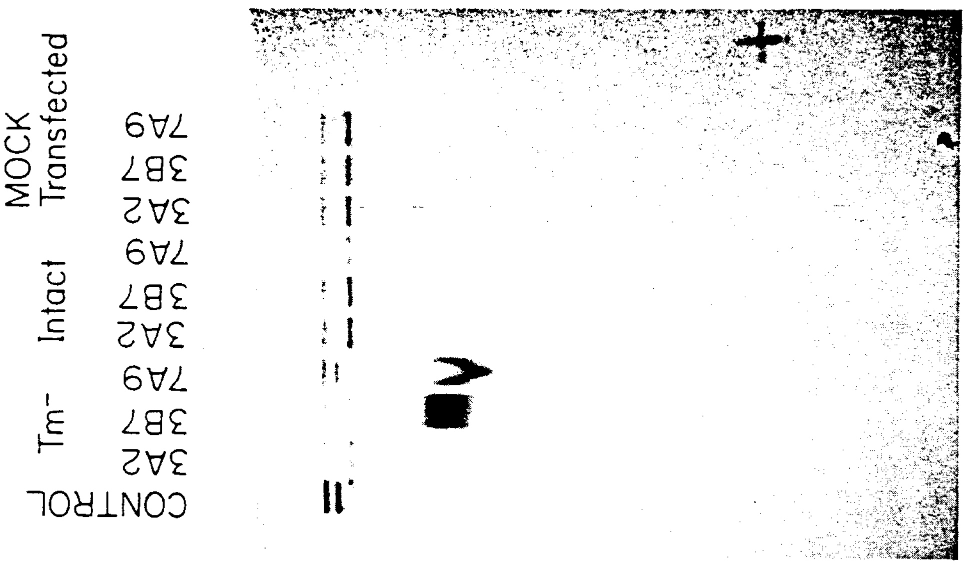


FIG. 32B

FIG.34A

```
         30         40         50         60         70         80         90
          |          |          |          |          |          |          |
ATGCCTGGGAAGATGGTCGTGATCCTTGGAGCCTCAAATATACTTTGGATAATGTTTGCAGCTTCTCAAGCT
 M  P  G  K  M  V  V  I  L  G  A  S  N  I  L  W  I  M  F  A  A  S  Q  A

       100        110        120        130        140        150        160
         |          |          |          |          |          |          |
TTTAAAATCGAGACCACCCCAGAATCTAGATATCTTGCTCAGATTGGTGACTCCGTCTCATTGACTTGCAGC
 F  K  I  E  T  T  P  E  S  R  Y  L  A  Q  I  G  D  S  V  S  L  T  C  S

       170        180        190        200        210        220        230
         |          |          |          |          |          |          |
ACCACAGGCTGTGAGTCCCCATTTTTCTCTTGGAGAACCCAGATAGATAGTCCACTGAATGGGAAGGTGACG
 T  T  G  C  E  S  P  F  F  S  W  R  T  Q  I  D  S  P  L  N  G  K  V  T

240        250        260        270        280        290        300        310
  |          |          |          |          |          |          |          |
AATGAGGGGACCACATCTACGCTGACAATGAATCCTGTTAGTTTTGGGAACGAACACTCTTACCTGTGCACA
 N  E  G  T  T  S  T  L  T  M  N  P  V  S  F  G  N  E  H  S  Y  L  C  T

         320        330        340        350        360        370        380
           |          |          |          |          |          |          |
GCAACTTGTGAATCTAGGAAATTGGAAAAAGGAATCCAGGTGGAGATCTACTCTTTCCCTAAGGATCCAGAG
 A  T  C  E  S  R  K  L  E  K  G  I  Q  V  E  I  Y  S  F  P  K  D  P  E
```

FIG.34B

```
390       400       410       420       430       440       450
 |         |         |         |         |         |         |
ATTCATTTGAGTGGCCCTCTGGAGGCTGGGAAGCCGATCACAGTCAAGTGTTCAGTTGCTGATGTATACCCA
 I  H  L  S  G  P  L  E  A  G  K  P  I  T  V  K  C  S  V  A  D  V  Y  P

460       470       480       490       500       510       520
 |         |         |         |         |         |         |
TTTGACAGGCTGGAGATAGACTTACTGAAAGGAGATCATCTCATGAAGAGTCAGGAATTTCTGGAGGATGCA
 F  D  R  L  E  I  D  L  L  K  G  D  H  L  M  K  S  Q  E  F  L  E  D  A

530       540       550       560       570       580       590
 |         |         |         |         |         |         |
GACAGGAAGTCCCTGGAAACCAAGAGTTTGGAAGTAACCTTTACTCCTGTCATTGAGGATATTGGAAAAGTT
 D  R  K  S  L  E  T  K  S  L  E  V  T  F  T  P  V  I  E  D  I  G  K  V

600       610       620       630       640       650       660       670
 |         |         |         |         |         |         |         |
CTTGTTTGCCGAGCTAAATTACACATTGATGAAATGGATTCTGTGCCCACAGTAAGGCAGGCTGTAAAAGAA
 L  V  C  R  A  K  L  H  I  D  E  M  D  S  V  P  T  V  R  Q  A  V  K  E

      680       690       700       710       720       730       740
       |         |         |         |         |         |         |
TTGCAAGTCTACATATCACCCAAGAATACAGTTATTTCTGTGAATCCATCCACAAAGCTGCAAGAAGGTGGC
 L  Q  V  Y  I  S  P  K  N  T  V  I  S  V  N  P  S  T  K  L  Q  E  G  G
```

FIG. 34C

```
     750       760       770       780       790       800       810
TCTGTGACCATGACCTGTTCCAGCGAGGGTCTACCAGCTCCAGAGATTTTCTGGAGTAAGAAATTAGATAAT
 S  V  T  M  T  C  S  S  E  G  L  P  A  P  E  I  F  W  S  K  K  L  D  N

   820       830       840       850       860       870       880
GGGAATCTACAGCACCTTTCTGGAAATGCAACTCTCACCTTAATTGCTATGAGGATGGAAGATTCTGGAATT
 G  N  L  Q  H  L  S  G  N  A  T  L  T  L  I  A  M  R  M  E  D  S  G  I

890       900       910       920       930       940       950
TATGTGTGTGAAGGAGTTAATTTGATTGGGAAAAACAGAAAAGAGGTGGAATTAATTGTTCAAGAGAAACCA
 Y  V  C  E  G  V  N  L  I  G  K  N  R  K  E  V  E  L  I  V  Q  E  K  P

960       970       980       990       1000      1010      1020      1030
TTTACTGTTGAGATCTCCCCTGGACCCCGGATTGCTGCTCAGATTGGAGACTCAGTCATGTTGACATGTAGT
 F  T  V  E  I  S  P  G  P  R  I  A  A  Q  I  G  D  S  V  M  L  T  C  S

       1040      1050      1060      1070      1080      1090      1100
GTCATGGGCTGTGAATCCCCATCTTTCTCCTGGAGAACCCAGATAGACAGCCCTCTGAGCGGGAAGGTGAGG
 V  M  G  C  E  S  P  S  F  S  W  R  T  Q  I  D  S  P  L  S  G  K  V  R
```

FIG.34D

```
     1110        1120        1130        1140        1150        1160        1170
        |           |           |           |           |           |           |
AGTGAGGGGACCAATTCCACGCTGACCCTGAGCCCTGTGAGTTTTGAGAACGAACACTCTTATCTGTGCACA
 S  E  G  T  N  S  T  L  T  L  S  P  V  S  F  E  N  E  H  S  Y  L  C  T

  1180        1190        1200        1210        1220        1230        1240
     |           |           |           |           |           |           |
GTGACTTGTGGACATAAGAAACTGGAAAAGGGAATCCAGGTGGAGCTCTACTCATTCCCTAGAGATCCAGAA
 V  T  C  G  H  K  K  L  E  K  G  I  Q  V  E  L  Y  S  F  P  R  D  P  E

1250        1260        1270        1280        1290        1300        1310
  |           |           |           |           |           |           |
ATCGAGATGAGTGGTGGCCTCGTGAATGGGAGCTCTGTCACTGTAAGCTGCAAGGTTCCTAGCGTGTACCCC
 I  E  M  S  G  G  L  V  N  G  S  S  V  T  V  S  C  K  V  P  S  V  Y  P

1320        1330        1340        1350        1360        1370        1380        1390
|           |           |           |           |           |           |           |
CTTGACCGGCTGGAGATTGAATTACTTAAGGGGGAGACTATTCTGGAGAATATAGAGTTTTTGGAGGATACG
 L  D  R  L  E  I  E  L  L  K  G  E  T  I  L  E  N  I  E  F  L  E  D  T

        1400        1410        1420        1430        1440        1450        1460
           |           |           |           |           |           |           |
GATATGAAATCTCTAGAGAACAAAAGTTTGGAAATGACCTTCATCCCTACCATTGAAGATACTGGAAAAGCT
 D  M  K  S  L  E  N  K  S  L  E  M  T  F  I  P  T  I  E  D  T  G  K  A
```

FIG. 34E

```
      1470      1480      1490      1500      1510      1520      1530
         |         |         |         |         |         |         |
CTTGTTTGTCAGGCTAAGTTACATATTGATGACATGGAATTCGAACCCAAACAAAGGCAGAGTACGCAAACA
 L  V  C  Q  A  K  L  H  I  D  D  M  E  F  E  P  K  Q  R  Q  S  T  Q  T

      1540      1550      1560      1570      1580      1590      1600
         |         |         |         |         |         |         |
CTTTATGTCAATGTTGCCCCCAGAGATACAACCGTCTTGGTCAGCCCTTCCTCCATCCTGGAGGAAGGCAGT
 L  Y  V  N  V  A  P  R  D  T  T  V  L  V  S  P  S  S  I  L  E  E  G  S

      1610      1620      1630      1640      1650      1660      1670
         |         |         |         |         |         |         |
TCTGTGAATATGACATGCTTGAGCCAGGGCTTTCCTGCTCCGAAAATCCTGTGGAGCAGGCAGCTCCCTAAC
 S  V  N  M  T  C  L  S  Q  G  F  P  A  P  K  I  L  W  S  R  Q  L  P  N

      1680      1690      1700      1710      1720      1730      1740      1750
         |         |         |         |         |         |         |         |
GGGGAGCTACAGCCTCTTTCTGAGAATGCAACTCTCACCTTAATTTCTACAAAAATGGAAGATTCTGGGGTT
 G  E  L  Q  P  L  S  E  N  A  T  L  T  L  I  S  T  K  M  E  D  S  G  V
```

FIG. 34F

```
      1760      1770      1780      1790      1800      1810      1820
TATTTATGTGAAGGAATTAACCAGGCTGGAAGAAGCAGAAAGGAAGTGGAATTAATTATCCAAGTTACTCCA
 Y  L  C  E  G  I  N  Q  A  G  R  S  R  K  E  V  E  L  I  I  Q  V  T  P

    1830      1840      1850      1860      1870      1880      1890
AAAGACATAAAACTTACAGCTTTTCCTTCTGAGAGTGTCAAAGAAGGAGACACTGTCATCATCTCTTGTACA
 K  D  I  K  L  T  A  F  P  S  E  S  V  K  E  G  D  T  V  I  I  S  C  T

  1900      1910      1920      1930      1940      1950      1960
TGTGGAAATGTTCCAGAAACATGGATAATCCTGAAGAAAAAAGCGGAGACAGGAGACACAGTACTAAAATCT
 C  G  N  V  P  E  T  W  I  I  L  K  K  K  A  E  T  G  D  T  V  L  K  S

1970      1980      1990      2000      2010      2020      2030
ATAGATGGCGCCTATACCATCCGAAAGGCCCAGTTGAAGGATGCGGGAGTATATGAATGTGAATCTAAAAAC
 I  D  G  A  Y  T  I  R  K  A  Q  L  K  D  A  G  V  Y  E  C  E  S  K  N
```

FIG.34G

```
2040      2050      2060      2070      2080      2090      2100      2110
   |         |         |         |         |         |         |         |
   AAAGTTGGCTCACAATTAAGAAGTTTAACACTTGATGTTCAAGGAAGAGAAAACAACAAAGACTATTTTTCT
    K  V  G  S  Q  L  R  S  L  T  L  D  V  Q  G  R  E  N  N  K  D  Y  F  S

         2120      2130      2140      2150      2160      2170      2180
            |         |         |         |         |         |         |
   CCTGAGCTTCTCGTGCTCTATTTTGCATCCTCCTTAATAATACCTGCCATTGGAATGATAATTTACTTTGCA
    P  E  L  L  V  L  Y  F  A  S  S  L  I  I  P  A  I  G  M  I  I  Y  F  A

       2190      2200      2210      2220      2230      2240
          |         |         |         |         |         |
   AGAAAAGCCAACATGAAGGGGTCATATAGTCTTGTAGAAGCACAGAAATCAAAAGTGTAG
    R  K  A  N  M  K  G  S  Y  S  L  V  E  A  Q  K  S  K  V  -
```